# EUROPEAN PATENT SPECIFICATION

(11) **EP 4 137 472 B1**
(45) Date of publication and mention of the grant of the patent: **07.08.2024**
(21) Application number: 21845215.9
(22) Date of filing: 22.06.2021
(51) Int. Cl.: C07C 15/27, C07C 15/24, H10K 85/40, H10K 50/11, H10K 85/60, C07C 15/28

(54) **NOVEL COMPOUND AND ORGANIC LIGHT-EMITTING DEVICE COMPRISING THE SAME**
NEUARTIGE VERBINDUNG UND ORGANISCHE LICHTEMITTIERENDE VORRICHTUNG DIESE ENTHALTEND
NOUVEAU COMPOSÉ ET DISPOSITIF ÉMETTEUR DE LUMIÈRE ORGANIQUE LE COMPRENANT

(30) Priority: 24.07.2020 KR 20200092539
(43) Date of publication of application: 22.02.2023
(73) Proprietor: LG Chem, Ltd., Seoul 07336 (KR)
(72) Inventor: LIM, Byeong Yun, Daejeon 34122 (KR); LEE, Jaechol, Daejeon 34122 (KR); KIM, Yongwook, Daejeon 34122 (KR); YU, Soyoung, Daejeon 34122 (KR); KIM, Shin Sung, Daejeon 34122 (KR); KIM, Young Kwang, Daejeon 34122 (KR); CHO, Beomshin, Daejeon 34122 (KR)
(74) Representative: Hoffmann Eitle
(86) International application number: PCT/KR2021/007804
(87) International publication number: WO 2022/019491

(56) References cited:
- KR-A- 20110 085 178
- KR-A- 20120 097 322
- KR-A- 20140 058 292
- KR-A- 20200 053 259
- KR-A- 20200 070 801
- US-A1- 2017 365 789

## Description

### [TECHNICAL FIELD]

### CROSS-REFERENCE TO RELATED APPLICATION(S)

This application claims the benefit of and priority of Korean Patent Application No. 10-2020-0092539 filed on July 24, 2020 with the Korean Intellectual Property Office.

The present disclosure relates to a novel compound and an organic light emitting device comprising the same.

### [BACKGROUND ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

For the organic materials used in the organic light emitting devices as described above, the development of new materials is continuously required.

Meanwhile, recently, in order to reduce process costs, an organic light emitting device using a solution process, particularly an inkjet process, has been developed instead of a conventional deposition process. In the initial stage of development, attempts have been made to develop organic light emitting devices by coating all organic light emitting device layers by a solution process, but current technology has limitations. Therefore, only HIL, HTL, and EML are processed by a solution process, and a hybrid process utilizing traditional deposition processes is being studied as a subsequent process.

Therefore, the present disclosure provides a novel material for an organic light emitting device that can be used for an organic light emitting device and at the same time, can be used for a solution process.

KR 2020 0053259 A describes compounds for organic light emitting devices represented by the following formula: 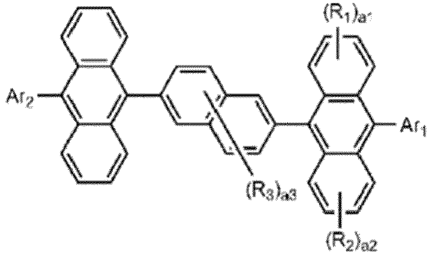 wherein Ar₁ and Ar₂ are each independently substituted or unsubstituted C₆₋₆₀ aryl; or C₂₋₆₀ heteroaryl containing at least one selected from the group consisting of substituted or unsubstituted N, O and S, R₁ and R₂ are the same or different from each other and are each independently hydrogen; substituted or unsubstituted C₁₋₆₀ alkyl; or substituted or unsubstituted C₃₋₆₀ cycloalkyl, provided that at least one of R₁ and R₂ is substituted or unsubstituted C₁₋₆₀ alkyl; or substituted or unsubstituted C₃₋₆₀ cycloalkyl, R₃ is hydrogen; heavy hydrogen; halogen; cyano; nitro; amino; substituted or unsubstituted C₁₋₆₀ alkyl; substituted or unsubstituted C₃₋₆₀ cycloalkyl; substituted or unsubstituted C₂₋₆₀ alkenyl; substituted or unsubstituted C₆₋₆₀ aryl; or C₂₋₆₀ heteroaryl containing at least one selected from the group consisting of substituted or unsubstituted N, O and S,a₁ and a2 are each independently integers from 0 to 4, and a1 +a2 is 1 or more, and a3 is an integer from 0 to 6. A specific compound disclosed in this document is the following:

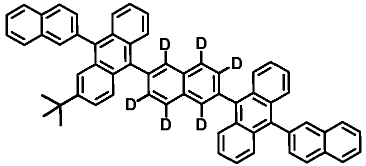

KR 2020 0070801 A describes compounds for organic light emitting devices represented by the following formula: 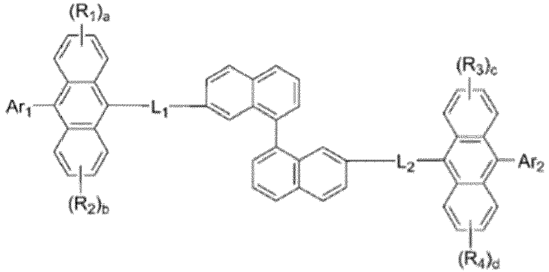 wherein Ar₁ and Ar₂ are each independently a substituted or unsubstituted C₆₋₆₀ aryl, and L₁ and L₂ are each independently a direct bond; substituted or unsubstituted C₆₋₆₀ arylene; or C₂₋₆₀ heteroarylene containing one or more of substituted or unsubstituted O, N, Si, and S, and R₁ to R₄ are each independently deuterium; halogen; hydroxy; cyano; nitrile; nitro; Amino; substituted or unsubstituted C₁₋₆₀ alkyl; substituted or unsubstituted C₁₋₆₀ haloalkyl; substituted or unsubstituted C₁₋₆₀ thioalkyl; substituted or unsubstituted C₁₋₆₀ alkoxy; substituted or unsubstituted C1-60 haloalkoxy; substituted or unsubstituted C₃₋₆₀ cycloalkyl; substituted or unsubstituted C₁₋₆₀ alkenyl; substituted or unsubstituted C₆₋₆₀ aryl; substituted or unsubstituted C₆₋₆₀ aryloxy; or C₂₋₆₀ heteroaryl containing one or more of substituted or unsubstituted O, N, Si, and S, and a, b, c, and d are each independently integers of 0 to 4.

KR 2014 0058292 A describes compounds for organic light emitting devices represented by the following formula:

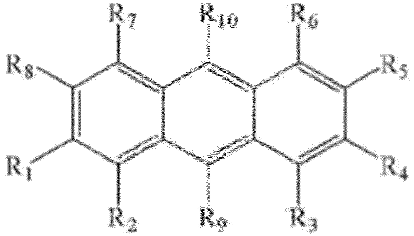

KR 2012 0097322 A describes compounds for organic light emitting devices represented by the following formula 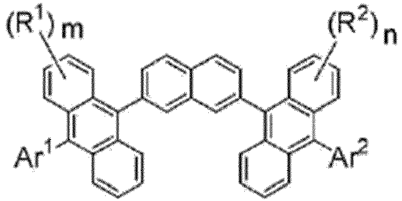 wherein Ar₁ and Ar₂ are each independently optionally substituted aryl, and R¹ and R² are each independently an alkyl with 1 to 4 carbon atoms or a cycloalkyl with 3 to 6 carbon atoms, m and n are each independently an integer of 0 to 8, and at least one hydrogen may be substituted with deuterium. A specific compound described in this document is the following:

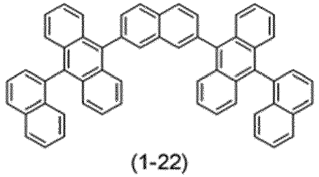

US 2017/365789 A1 describes deuterated indolocarbazole compound for use in organic light emitting devices represented by the following formula: 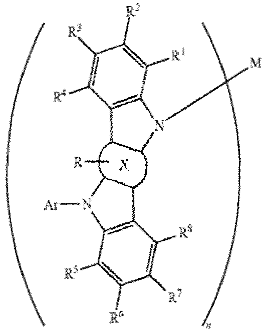 wherein X is an aromatic or heteroaromatic ring, and the others can be selected from a variety of substituents, provided at least one of R and R¹-R⁸ is a D-atom. In respect of other possible substituent meanings, reference is made to the document.

KR 2011 0085178 A describes compounds for use in organic light emitting devices having the following structure: 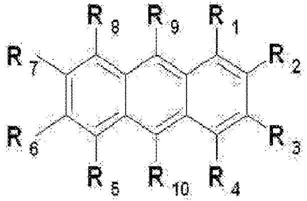 wherein R₁-R₁₀ are selected from the group consisting of: hydrogen, deuterium, nitrile group, fluorine group, nitro group, -OH group, silicon group, amide group, ester group, formyl group, substituted or unsubstituted C₁₋₃₀ alkyl group, substituted or unsubstituted C₃₋₆₀ cycloalkyl group, substituted or unsubstituted C₁₋₃₀ alkoxy group, substituted or unsubstituted C₆₋₆₀ aryloxy group, substituted or unsubstituted silyl group, substituted or unsubstituted C₁₃₋₅₀ fluorenyl group, substituted or unsubstituted carbazole group; a substituted or unsubstituted C3-60 heterocycle group including one or more of N, O, S, Si, or P atoms; and a substituted or unsubstituted C6-C60 aryl group.

### [Prior Art Literature]

### [Patent Literature]

(Patent Literature 0001) Korean Unexamined Patent Publication No. 10-2000-0051826

### [DETAILED DESCRIPTION OF THE INVENTION]

### [Technical Problem]

It is an object of the present disclosure to provide a novel compound and an organic light emitting device comprising the same.

### [Technical Solution]

According to an aspect of the present disclosure, there is provided a compound represented by the following Chemical Formula 1: 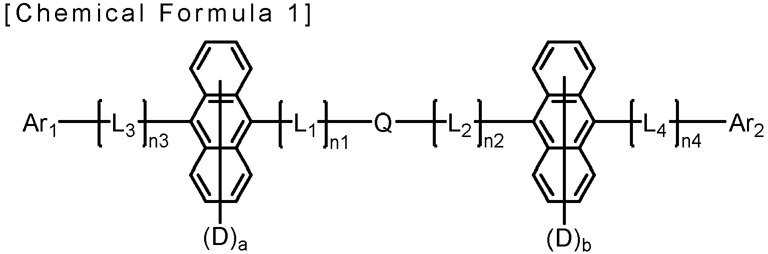 wherein, in Chemical Formula 1,
D means deuterium,
Q is naphthylene unsubstituted or substituted with deuterium,
Ar₁ and Ar₂ are each independently a substituted or unsubstituted C₆₋₆₀ aryl,
L₁ to L₄ are each independently a substituted or unsubstituted C₆₋₆₀ arylene,
n1 to n4 are each independently an integer of 0 to 2, a and b are each independently an integer of 0 to 8, provided that a+b is 1 or more, and
when each of n1 to n4 is 2, the structures in parentheses ([ ]) are the same as or different from each other.

According to another aspect of the present disclosure, there is provided an organic light emitting device comprising: a first electrode; a second electrode that is provided opposite to the first electrode; and a light emitting layer that is provided between the first electrode and the second electrode, wherein the light emitting layer comprises the compound represented by Chemical Formula 1.

### [ADVANTAGEOUS EFFECTS]

The above-mentioned compound represented by Chemical Formula 1 can be used as a material for an organic material layer of an organic light emitting device, can be used in a solution process, and can improve the efficiency and lifetime characteristics in the organic light emitting device.

### [BRIEF DESCRIPTION OF THE DRAWINGS]

FIG. 1 shows an example of an organic light emitting device comprising a substrate 1, an anode 2, a light emitting layer 3, and a cathode 4.
FIG. 2 shows an example of an organic light emitting device comprising a substrate 1, an anode 2, a hole injection layer 5, a hole transport layer 6, a light emitting layer 3, an electron injection and transport layer 7, and a cathode 4.

### [DETAILED DESCRIPTION OF THE EMBODIMENTS]

Hereinafter, embodiments of the present disclosure will be described in more detail to facilitate understanding of the invention.

### (Definition of Terms)

As used herein, the notation 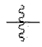 and 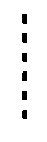 mean a bond linked to another substituent group, and D means deuterium.

As used herein, the term "substituted or unsubstituted" means being unsubstituted or substituted with one or more substituents selected from the group consisting of deuterium; a halogen group; a cyano group; a nitro group; a hydroxy group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heteroaryl containing at least one of N, O and S atoms, or being unsubstituted or substituted with a substituent to which two or more substituents of the above-exemplified substituents are linked. For example, "a substituent in which two or more substituents are linked" may be a biphenyl group. Namely, a biphenyl group may be an aryl group, or it may also be interpreted as a substituent in which two phenyl groups are linked.

In the present disclosure, the carbon number of a carbonyl group is not particularly limited, but is preferably 1 to 40. Specifically, the carbonyl group may be a substituent group having the following structural formulas, but is not limited thereto.

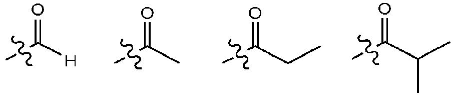

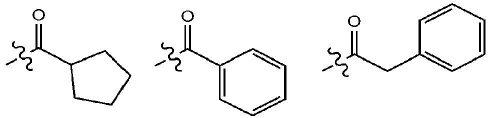

In the present disclosure, an ester group may have a structure in which oxygen of the ester group may be substituted by a straight-chain, branched-chain, or cyclic alkyl group having 1 to 25 carbon atoms, or an aryl group having 6 to 25 carbon atoms. Specifically, the ester group may be a substituent group having the following structural formulas, but is not limited thereto.

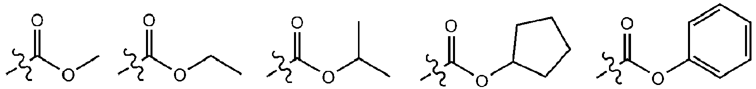

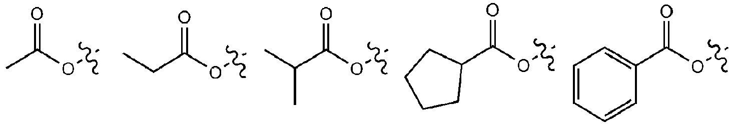

In the present disclosure, the carbon number of an imide group is not particularly limited, but is preferably 1 to 25. Specifically, the imide group may be a substituent group having the following structural formulas, but is not limited thereto.

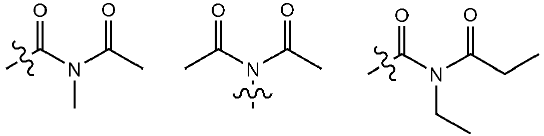

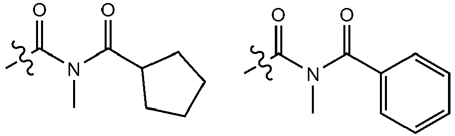

In the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, and the like, but is not limited thereto.

In the present disclosure, a boron group specifically includes a trimethylboron group, a triethyl boron group, a t-butyldimethylboron group, a triphenylboron group, and a phenylboron group, but is not limited thereto.

In the present disclosure, examples of a halogen group include fluorine, chlorine, bromine, or iodine.

In the present disclosure, the alkyl group may be straight-chain or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 1-methylhexyl, 2-methylhexyl, 3-methylhexyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to still another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to still another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The aryl group may be a phenyl group, a biphenylyl group, a terphenylyl group or the like as the monocyclic aryl group, but is not limited thereto. The polycyclic aryl group includes a naphthyl group, an anthryl group, a phenanthryl group, a pyrenyl group, a perylenyl group, a chrysenyl group, a fluorenyl group, benzofluorenyl group, or the like, but is not limited thereto.

In the present disclosure, the fluorenyl group may be substituted, and two substituents may be connected to each other to form a spiro structure. In the case where the fluorenyl group is substituted, 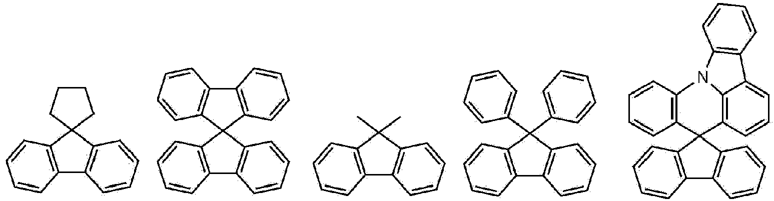 and the like can be formed. However, the structure is not limited thereto. Further, the benzofluorenyl group may be substituted similarly to the fluorenyl group, or two may be connected to each other to form a spiro structure.

In the present disclosure, a heteroaryl is a heteroaryl containing at least one of O, N, Si and S as a heteroatom, and the carbon number thereof is not particularly limited, but is preferably 2 to 60. Examples of the heteroaryl include xanthene, thioxanthene, a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, the arylamine group and the arylsily group is the same as the above-mentioned examples of the aryl group. In the present disclosure, the alkyl group in the aralkyl group, the alkylaryl group and the alkylamine group is the same as the above-mentioned examples of the alkyl group. In the present disclosure, the heteroaryl in the heteroarylamine may be applied to the above-mentioned description of the heteroaryl. In the present disclosure, the alkenyl group in the aralkenyl group is the same as the above-mentioned examples of the alkenyl group. In the present disclosure, the above-mentioned description of the aryl group may be applied except that the arylene is a divalent group. In the present disclosure, the above-mentioned description of the heteroaryl may be applied except that the heteroarylene is a divalent group.

In the present disclosure, the term "deuterated or substituted with deuterium" means that at least one available hydrogen in each Chemical Formula is substituted by deuterium. Specifically, being substituted with deuterium in the definition of each Chemical Formula or substituent means that at least one or more of the positions where hydrogen can be bonded in a molecule are substituted by deuterium.

Additionally, in the present disclosure, the term "deuterium substitution rate" means the percentage of the number of substituted deuteriums to the total number of hydrogens that may be present in each chemical formula.

### (Compound)

The present disclosure provides the compound represented by Chemical Formula 1.

The compound represented by Chemical Formula 1 is a compound having a structure in which two 9,10-anthracenylene are connected by a naphthylene linker (Q), which is charactered in that at least one of the hydrogens of the two 9,10-anthracenylene is substituted with deuterium. At this time, since the bond energy of the C-D bond is larger than that of the C-H bond, the compound has a stronger binding energy in the molecule as compared with the compound not substituted with deuterium, whereby the material stability can be increased. Therefore, the compound represented by Chemical Formula 1 may increase the efficiency and stability of the organic light emitting device, as compared with a compound having a structure that is not substituted with deuterium.

In particular, in order for the compound having a specific structure to exhibit the effect of increasing the binding energy in a molecule and improving material stability due to deuterium substitution, the deuterium substitution rate of the compound is higher, specifically, the deuterium substitution rate is preferably 80% or more. In this case, in order for a compound having a structure in which two 9,10-anthracenylene is connected by a naphthylene linker to exhibit a deuterium substitution rate of 80% or more, at least one hydrogen in the anthracenylene moiety must be substituted with deuterium. Therefore, in order to exhibit the effect of improving device characteristics due to deuterium substitution of a compound having a structure in which two 9,10-anthracenylene is connected by a naphthylene linker, it is necessary to have a structure as in Chemical Formula 1 in which at least one of the hydrogens of the two anthracenylene is substituted with deuterium.

Therefore, when the compound represented by Chemical Formula 1 in which the C-D bond exists in anthracenylene is used as a host material in the light emitting layer of the organic light emitting device, it can be significantly improved in the efficiency and lifetime of the organic light emitting device, as compared with the case where a compound having no CD bond in anthracenylene and having a CD bond only in other structures is used as a host material in the light emitting layer.

Further, the compound represented by Chemical Formula 1 has high solubility in an organic solvent used in the solution process, for example, an organic solvent such as cyclohexanone. Thus, it is suitable for use in a large area solution process such as an inkjet coating method using a solvent having a high boiling point.

Meanwhile, a, indicating the number of deuterium substitutions of anthracenylene on the left side of Q in Chemical Formula 1, is 0, 1, 2, 3, 4, 5, 6, 7, or 8. Further, b, indicating the number of deuterium substitutions of anthracenylene on the right side of Q in Chemical Formula 1, is 0, 1, 2, 3, 4, 5, 6, 7, or 8.

Further, since at least one of the hydrogens of the two anthracenylene in Chemical Formula 1 is deuterium, a+b is an integer of 1 to 16. More specifically, a+b is 2, 3, 4, 5, 6, 7, 8, 9, 10, 11, 12, 13, 14, 15, or 16. At this time, a compound in which a + b is 16, that is, a compound in which all the hydrogens bonded to the available carbons at positions 1 to 8 of 9, 10-anthracenylene are substituted with deuterium, has more C-D bonds in the molecule, whereby the material stability can be further increased. Thereby, the organic light emitting device employing the compound in which a+b is 16 in Chemical Formula 1 can further improve the efficiency and lifetime characteristics.

Further, in Chemical Formula 1, Q is naphthylene unsubstituted or substituted with 1 to 6 deuteriums.

Specifically, Q is 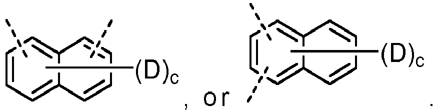 Here, D is deuterium, and c is an integer of 0 to 6.

More specifically, Q is any one of the divalent substituents represented by the following Chemical Formulas 2a to 2j: 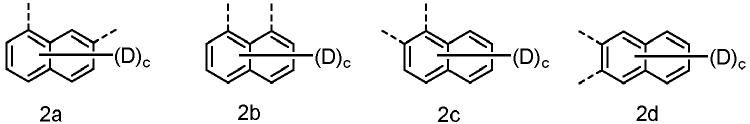 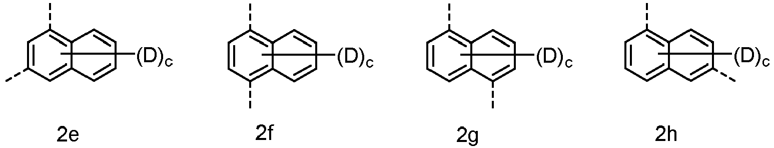 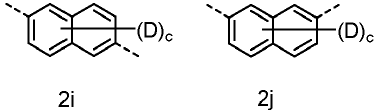 wherein, in Chemical Formulas 2a to 2j,
c is an integer of 0 to 6.

That is, in Chemical Formulas 2a to 2j, c indicating the number of deuterium substitutions is 0, 1, 2, 3, 4, 5, or 6.

Further, in Chemical Formula 1, Ar₁ and Ar₂ may each independently be a C₆₋₂₀ aryl unsubstituted or substituted with one or more deuteriums.

Specifically, Ar₁ and Ar₂ may be each independently phenyl unsubstituted or substituted with 1 to 5 deuteriums; or naphthyl unsubstituted or substituted with 1 to 7 deuteriums.

At this time, at least one of Ar₁ and Ar₂ is 1-naphthyl unsubstituted or substituted with 1 to 7 deuteriums; or
at least one of Ar₁ and Ar₂ may be 2-naphthyl unsubstituted or substituted with 1 to 7 deuteriums.

For example, Ar₁ and Ar₂ is 1-naphthyl unsubstituted or substituted with 1 to 7 deuteriums; or
Ar₁ and Ar₂ may be 2-naphthyl unsubstituted or substituted with 1 to 7 deuteriums.
one of Ar₁ and Ar₂ is 1-naphthyl unsubstituted or substituted with 1 to 7 deuteriums, and the rest is 2-naphthyl unsubstituted or substituted with 1 to 7 deuteriums; or
one of Ar₁ and Ar₂ is phenyl unsubstituted or substituted with 1 to 5 deuteriums, and the rest is 1-naphthyl unsubstituted or substituted with 1 to 7 deuteriums; or
one of Ar₁ and Ar₂ is phenyl unsubstituted or substituted with 1 to 5 deuteriums; and the rest may be 2-naphthyl unsubstituted or substituted with 1 to 7 deuteriums.

In this case, Ar₁ and Ar₂ may be the same as each other. Alternatively, Ar₁ and Ar₂ may be different.

Further, in Chemical Formula 1, L₁ to L₄ may be each independently a C₆₋₂₀ arylene unsubstituted or substituted with one or more deuteriums.

Specifically, L₁ to L₄ are each independently phenylene unsubstituted or substituted with one or more deuteriums; biphenyldiyl unsubstituted or substituted with one or more deuteriums; or naphthylene unsubstituted or substituted with one or more deuteriums.

More specifically, L₁ and L₂ are each independently phenylene unsubstituted or substituted with 1 to 4 deuteriums; or naphthylene unsubstituted or substituted with 1 to 6 deuteriums.

In this case, n1, indicating the number of L₁, is 0, 1, or 2, and when n1 is 2, two L₁s may be the same or different from each other.

In addition, n2, indicating the number of L₂, is 0, 1, or 2, and when n2 is 2, two L₂s may be the same as or different from each other.

Therefore, n1 +n2 may be 0, 1, 2, 3, or 4.

For example, L₁ and L₂ may be each independently 1,2-phenylene unsubstituted or substituted with 1 to 4 deuteriums; 1,3-phenylene unsubstituted or substituted with 1 to 4 deuteriums; 1,4-phenylene unsubstituted or substituted with 1 to 4 deuteriums; 1,2-naphthylene unsubstituted or substituted with 1 to 6 deuteriums; 1,3-naphthylene unsubstituted or substituted with 1 to 6 deuteriums; 1,4-naphthylene unsubstituted or substituted with 1 to 6 deuteriums; 1,5-naphthylene unsubstituted or substituted with 1 to 6 deuteriums; 1,6-naphthylene unsubstituted or substituted with 1 to 6 deuteriums; 1,7-naphthylene unsubstituted or substituted with 1 to 6 deuteriums; 1,8-naphthylene unsubstituted or substituted with 1 to 6 deuteriums; 2,3-naphthylene unsubstituted or substituted with 1 to 6 deuteriums; 2,6-naphthylene unsubstituted or substituted with 1 to 6 deuteriums; or 2,7-naphthylene unsubstituted or substituted with 1 to 6 deuteriums.

More specifically, L₃ and L₄ may be each independently phenylene unsubstituted or substituted with 1 to 4 deuteriums.

In this case, n3 indicating the number of L₃s is 0, 1, or 2, and when n3 is 2, two L₃s may be the same or different from each other.

Also, n4 indicating the number of L4s is 0, 1, or 2, and when n4 is 2, two L4s may be the same or different from each other.

Therefore, n3+n4 may be 0, 1, 2, 3, or 4.

For example, L₃ and L₄ may be each independently 1,2-phenylene unsubstituted or substituted with 1 to 4 deuteriums; 1,3-phenylene unsubstituted or substituted with 1 to 4 deuteriums; or 1,4-phenylene unsubstituted or substituted with 1 to 4 deuteriums.

Alternatively, n1 +n2 may be 0, 1, 2, or 3, and n3+n4 may be 0, 1, or 2.

Preferably, n1 +n2+n3+n4 is 0, 1, 2, 3, or 4.

Meanwhile, the deuterium substitution rate of the compound may be 50% to 100%. Specifically, the deuterium substitution rate of the compound may be 60% or more, 70% or more, 80% or more, 85% or more, 88% or more, 90% or more, 91% or more, 92% or more, 93% or more, or 94% or more, and 100% or less. The deuterium substitution rate of these compounds is calculated as the number of substituted deuteriums relative to the total number of hydrogens that can be present in the Chemical Formula, wherein the number of substituted deuterium may be obtained through MALDI-TOF MS (Matrix-Assisted Laser Desorption/Ionization Time-of-Flight Mass Spectrometer) analysis.

More specifically, the compound represented by Chemical Formula 1 may be a compound in which a+b is 16; or a compound having a deuterium substitution rate of 80% to 100%.

Further, the compound may be represented by the following Chemical Formula 1-1: 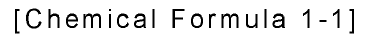 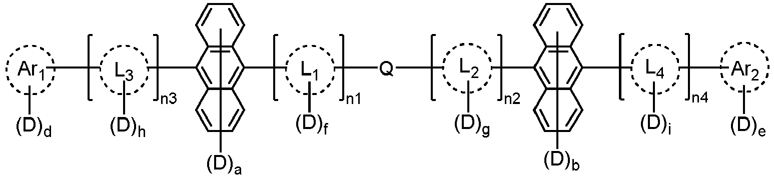 wherein, in Chemical Formula 1-1,
Q is any one of the divalent substituents represented by the following Chemical Formulas 2a to 2j, 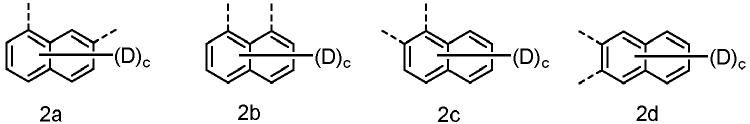 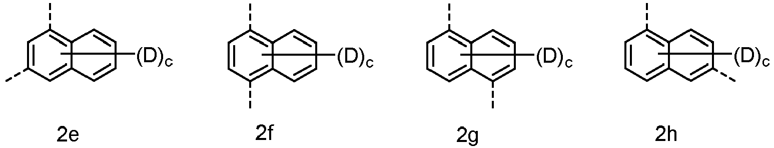 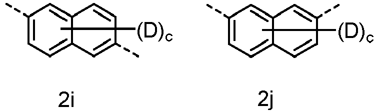 wherein, in Chemical Formulas 2a to 2j,
c is an integer of 0 to 6,
Ar₁ and Ar₂ are each independently phenyl or naphthyl,
L₁ and L₂ are each independently phenylene or naphthylene,
L₃ and L₄ are each independently phenylene,
d and e are each independently an integer of 0 to 7,
f and g are each independently an integer of 0 to 6,
h and i are each independently an integer of 0 to 4, and
a, b, and n1 to n4 are as defined in Chemical Formula 1.

In Chemical Formula 1-1, one of Ar₁ and Ar₂ may be 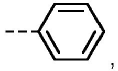 and the rest may be 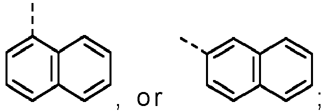 or one of Ar₁ and Ar₂ may be 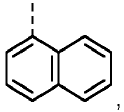 and the rest may be 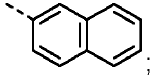 or both Ar₁ and Ar₂ may be 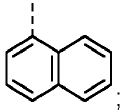 or both Ar₁ and Ar₂ may be

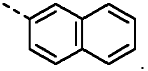

Alternatively, L₁ and L₂ may be each independently any one selected from the group consisting of:

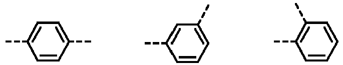

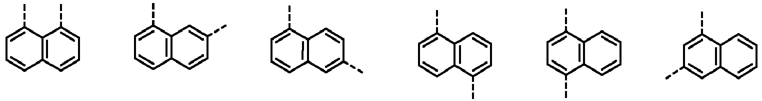

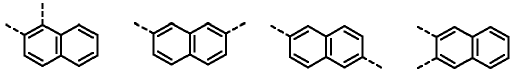

For example, L₁ and L₂ may be each independently any one selected from the group consisting of:

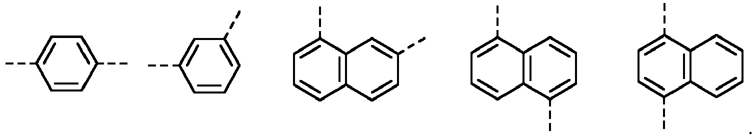

Further, L₃ and L₄ may be each independently 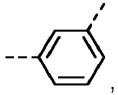 or

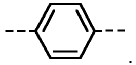

Further, in Chemical Formula 1-1, L₁ to L₄ are each independently 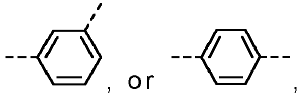
n1 to n4 are each independently 0 or 1,
n1+n2+n3+n4 is 0; or
n1+n2+n3+n4 is 1; or
n1+n2+n3+n4 is 2; or
n1+n2+n3+n4 is 3; or
n1+n2+n3+n4 may be 4.

Alternatively, in Chemical Formula 1-1, L₁ is phenylene, or naphthylene,
L₂ to L₄ are each independently 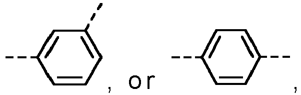
n1 is 0, 1, or 2,
n2 to n4 are each independently 0 or 1,
n1+n2+n3+n4 is 0; or
n1+n2+n3+n4 is 1; or
n1+n2+n3+n4 is 2; or
n1+n2+n3+n4 is 3; or
n1+n2+n3+n4 is 4; or
n1+n2+n3+n4 may be 5.

Further, the compound may be represented by the following Chemical Formula 1-1A or 1-1B: 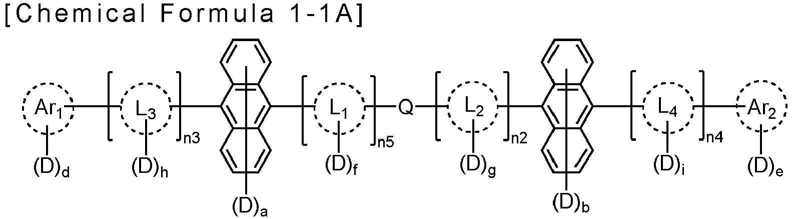 wherein, in Chemical Formula 1-1A,
Q is any one of the divalent substituents represented by the following Chemical Formulas 2a to 2j, 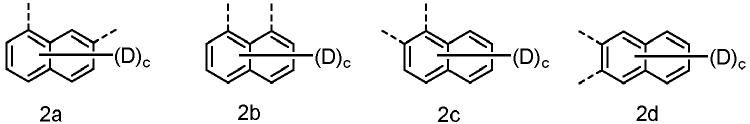 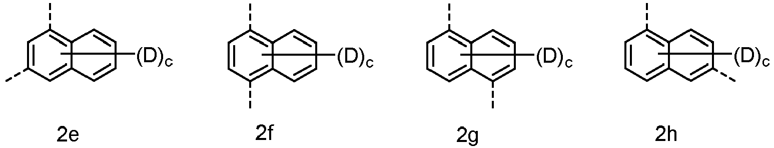 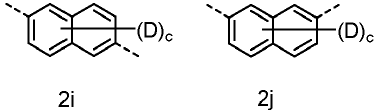 wherein, in Chemical Formulas 2a to 2j,
c is an integer of 0 to 6,
Ar₁ and Ar₂ are each independently phenyl or naphthyl,
L₁ to L₄ are each independently phenylene,
d and e are each independently an integer of 0 to 7,
f, g, h and i are each independently an integer of 0 to 4,
n2 to n5 are each independently an integer of 0 or 1,
a and b are as defined in Chemical Formula 1, 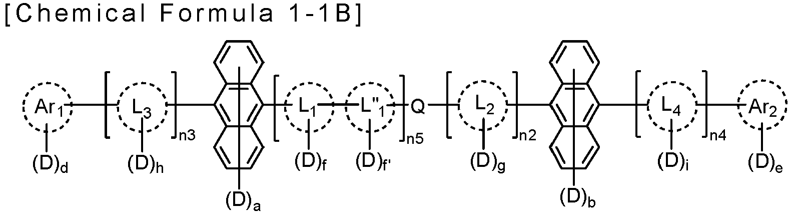 wherein, in Chemical Formula 1-1B,
   Q is any one of the divalent substituents represented by the following Chemical Formulas 2a to 2j, 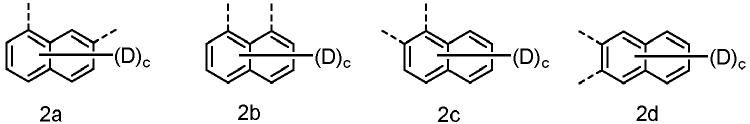 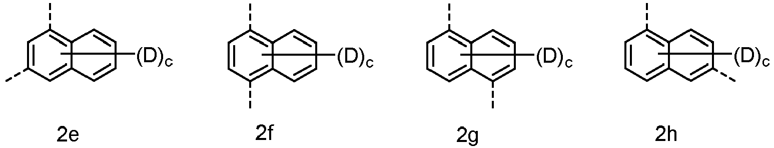 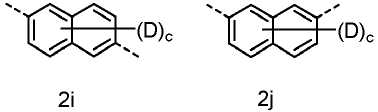 wherein, in Chemical Formulas 2a to 2j,
   c is an integer of 0 to 6,
   Ar₁ and Ar₂ are each independently phenyl or naphthyl,
   L"₁ is naphthylene,
   L₁ to L₄ are each independently phenylene,
   d and e are each independently an integer of 0 to 7,
   each f' is independently an integer of 0 to 6,
   f, g, h and i are each independently an integer of 0 to 4,
   n2 to n5 are each independently an integer of 0 or 1, and
   a and b are as defined in Chemical Formula 1.

Further, the compound may be represented by the following Chemical Formula 1-1-1: 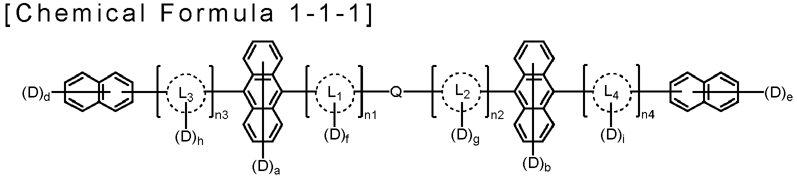 wherein, in Chemical Formula 1-1-1,
L₁ to L₄ are each independently phenylene,
n1 to n4 are each independently 0 or 1,
d and e are each independently an integer of 0 to 7,
f, g, h and i are each independently an integer of 0 to 4, Q, a and b are as defined in Chemical Formula 1-1, a+b is 16 and c+d+e+f+g+h+i is 0; or
n1+n2+n3+n4 is 0 and a+b+c+d+e is 1 to 36; or
n1+n2+n3+n4 is 1 and a+b+c+d+e+(one of f, g, h and i) is 1 to 40; or
n1+n2+n3+n4 is 2, and a+b+c+d+e+(the sum of two of f, g, h and i) is 1 to 44; or
n1+n2+n3+n4 is 3, and a+b+c+d+e+(the sum of three of f, g, h and i) is 1 to 48; or
n1+n2+n3+n4 may be 4, and a+b+c+d+e+f+g+h+i may be 1 to 52.

More specifically,
The compound represented by Chemical Formula 1-1-1 may be a compound in which a+b is 16 and c+d+e+f+g+h+i is 0; or the deuterium substitution rate of the compound is 80% to 100%.

When the deuterium substitution rate of the compound is 80% to 100%, for example,
n1+n2+n3+n4 is 0 and a+b+c+d+e is 30 to 36; or
n1+n2+n3+n4 is 1 and a+b+c+d+e+(one of f, g, h and i) is 34 to 40; or
n1+n2+n3+n4 is 2 and a+b+c+d+e+(the sum of two of f, g, h and i) is 38 to 44; or
n1+n2+n3+n4 is 3 and a+b+c+d+e+(the sum of three of f, g, h and i) is 42 to 48; or
n1+n2+n3+n4 may be 4, and a+b+c+d+e+f+g+h+i may be 46 to 52.

In addition, the compound may be represented by the following Chemical Formula 1-1-2: 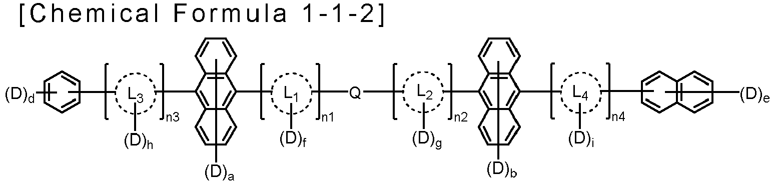 wherein, in Chemical Formula 1-1-2,
L₁ to L₄ are each independently phenylene,
n1 to n4 are each independently 0 or 1,
d is an integer of 0 to 5,
e is an integer of 0 to 7,
f, g, h and i are each independently an integer of 0 to 4,
Q, a and b are as defined in Chemical Formula 1-1,
a+b is 16 and c+d+e+f+g+h+i is 0; or
n1+n2+n3+n4 is 0 and a+b+c+d+e is 1 to 34; or
n1+n2+n3+n4 is 1 and a+b+c+d+e+(one of f, g, h and i) is 1 to 38; or
n1+n2+n3+n4 is 2 and a+b+c+d+e+(the sum of two of f, g, h and i) is 1 to 42; or
n1+n2+n3+n4 is 3, and a+b+c+d+e+(the sum of three of f, g, h and i) is 1 to 46; or
n1+n2+n3+n4 may be 4, and a+b+c+d+e+f+g+h+i may be 1 to 50.

More specifically,
The compound represented by Chemical Formula 1-1-2 may be a compound in which a+b is 16, c+d+e+f+g+h+i is 0; or the deuterium substitution rate of the compound is 80% to 100%.

When the deuterium substitution rate of the compound is 80% to 100%, for example,
n1+n2+n3+n4 is 0 and a+b+c+d+e is 28 to 34; or
n1+n2+n3+n4 is 1 and a+b+c+d+e+(one of f, g, h and i) is 32 to 38; or
n1+n2+n3+n4 is 2 and a+b+c+d+e+(the sum of two of f, g, h and i) is 36 to 42; or
n1+n2+n3+n4 is 3, and a+b+c+d+e+(the sum of three of f, g, h and i) is 40 to 46; or
n1+n2+n3+n4 may be 4, and a+b+c+d+e+f+g+h+i may be 44 to 50.

Further, the compound may be represented by the following Chemical Formula 1-1-3: 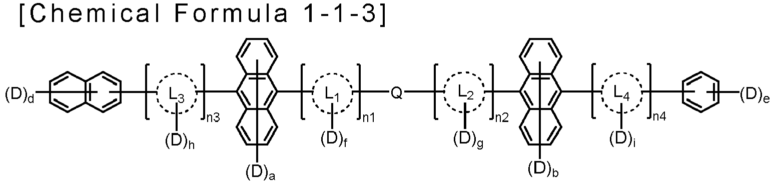 wherein, in Chemical Formula 1-1-3,
L₁ to L₄ are each independently phenylene,
n1 to n4 are each independently 0 or 1,
d is an integer of 0 to 7,
e is an integer of 0 to 5,
f, g, h and i are each independently an integer of 0 to 4,
Q, a and b are as defined in Chemical Formula 1-1,
a+b is 16 and c+d+e+f+g+h+i is 0; or
n1+n2+n3+n4 is 0 and a+b+c+d+e is 1 to 34; or
n1+n2+n3+n4 is 1 and a+b+c+d+e+(one of f, g, h and i) is 1 to 38; or
n1+n2+n3+n4 is 2 and a+b+c+d+e+(the sum of two of f, g, h and i) is 1 to 42; or
n1+n2+n3+n4 is 3, and a+b+c+d+e+(the sum of three of f, g, h and i) is 1 to 46; or
n1+n2+n3+n4 may be 4, and a+b+c+d+e+f+g+h+i may be 1 to 50.

More specifically,
The compound represented by Chemical Formula 1-1-3 may be a compound in which a+b is 16, c+d+e+f+g+h+i is 0; or the deuterium substitution rate of the compound is 80% to 100%.

When the deuterium substitution rate of the compound is 80% to 100%, for example,
n1+n2+n3+n4 is 0 and a+b+c+d+e is 28 to 34; or
n1+n2+n3+n4 is 1 and a+b+c+d+e+(one of f, g, h and i) is 32 to 38; or
n1+n2+n3+n4 is 2 and a+b+c+d+e+(the sum of two of f, g, h and i) is 36 to 42; or
n1+n2+n3+n4 is 3, and a+b+c+d+e+(the sum of three of f, g, h and i) is 40 to 46; or
n1+n2+n3+n4 may be 4, and a+b+c+d+e+f+g+h+i may be 44 to 50.

Further, the compound may be represented by the following Chemical Formula 1-1-4: 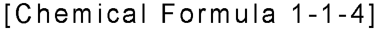 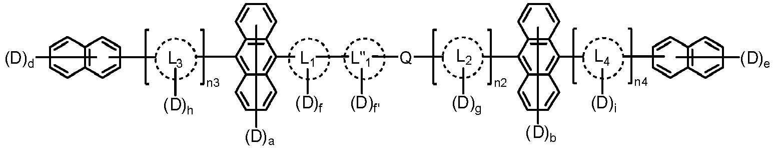 wherein, in Chemical Formula 1-1-4,
L"₁ is naphthylene,
L₁ to L₄ are each independently phenylene,
n2 to n4 are each independently 0 or 1,
d and e are each independently an integer of 0 to 7, each f' is independently an integer of 0 to 6,
f, g, h and i are each independently an integer of 0 to 4, Q, a and b are as defined in Chemical Formula 1-1, a+b is 16 and c+d+e+f+f'+g+h+i is 0; or
n2+n3+n4 is 0 and a + b + c + d + e + f+f' is 1 to 46; or
n2+n3+n4 is 1 and a+b+c+d+e+f+f'+(one of g, h and i) is 1-50; or
n2+n3+n4 is 2, and a+b+c+d+e+f+f'+(the sum of two of g, h and i) is 1 to 54; or
n2+n3+n4 may be 3, and a+b+c+d+e+f+f'+g+h+i may be 1 to 58.

More specifically,
the compound represented by Chemical Formula 1-1-3 may be a compound in which a+b is 16, c+d+e+f+f'+g+h+i is 0; or the deuterium substitution rate of the compound is 80% to 100%.

When the deuterium substitution rate of the compound is 80% to 100%, for example,
n2+n3+n4 is 0 and a + b + c + d + e + f+f' is 40 to 46; or
n2+n3+n4 is 1 and a+b+c+d+e+f+f'+(one of g, h and i) is 44-50; or
n2+n3+n4 is 2, and a+b+c+d+e+f+f'+(the sum of two of g, h and i) is 48 to 54; or
n2+n3+n4 may be 3, and a+b+c+d+e+f+f'+g+h+i may be 52 to 58.

Representative examples of the compound represented by Chemical Formula 1 are any one selected from the group consisting of compounds represented by the following Chemical Formulas: 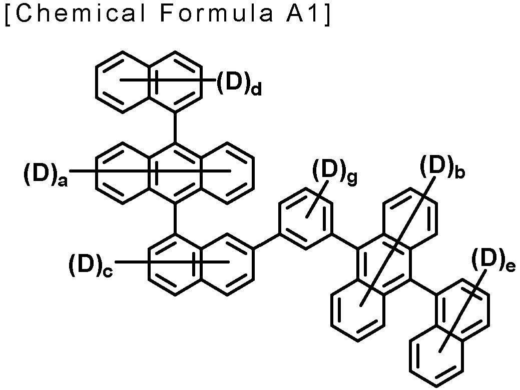 wherein, in Chemical Formula A1,
a+b+c+d+e+g is 1 to 40, 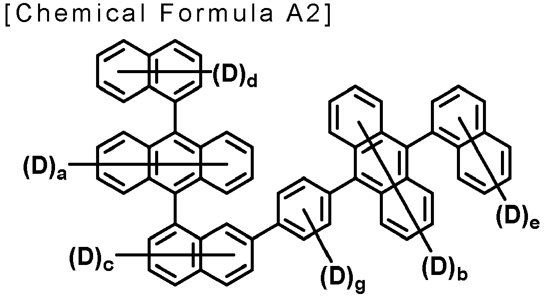
wherein, in Chemical Formula A2,
a+b+c+d+e+g is 1 to 40, 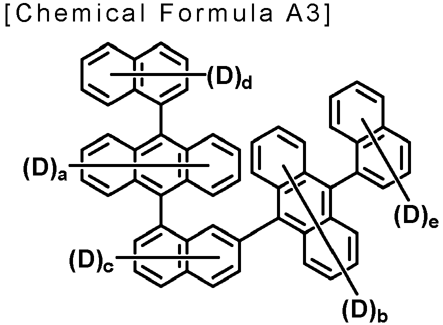
wherein, in Chemical Formula A3,
a+b+c+d+e is 1 to 36, 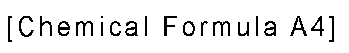 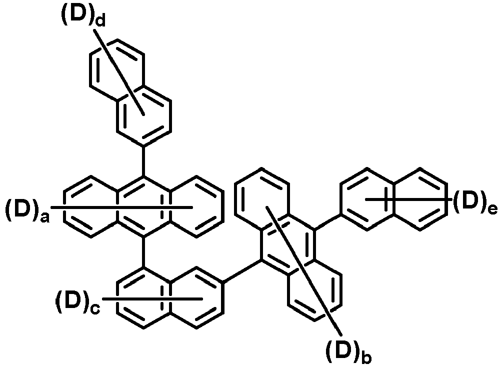
wherein, in Chemical Formula A4,
a+b+c+d+e is 1 to 36, 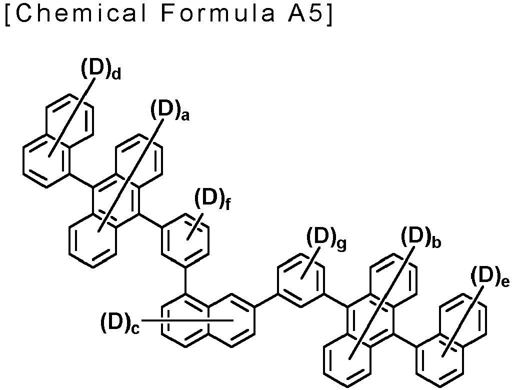
wherein, in Chemical Formula A5,
a+b+c+d+e+f+g is 1 to 44, 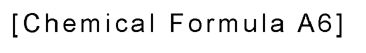 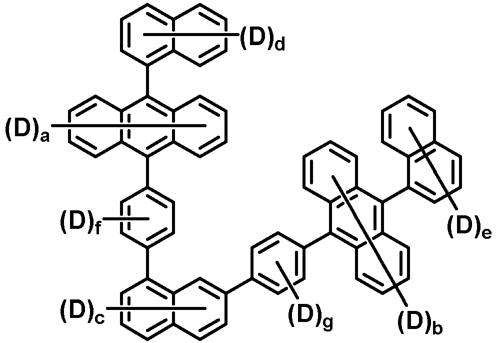
wherein, in Chemical Formula A6,
a+b+c+d+e+f+g is 1 to 44, 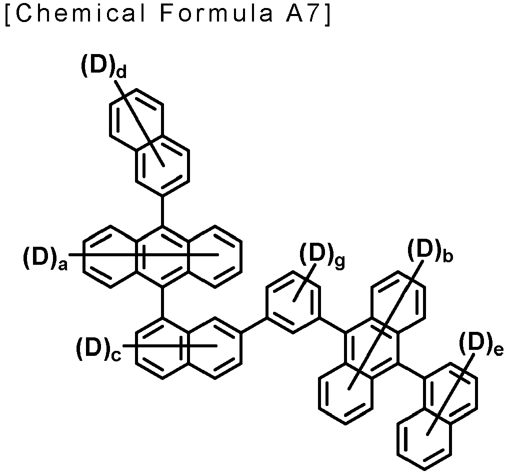
wherein, in Chemical Formula A7,
a+b+c+d+e+g is 1 to 40, 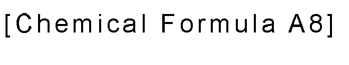 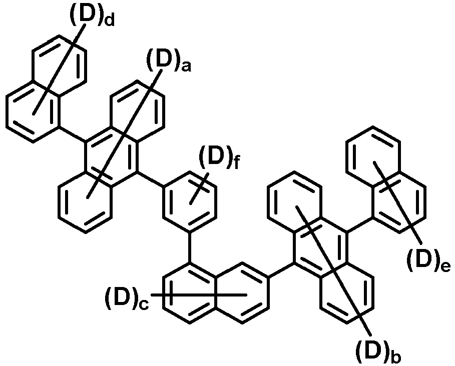
wherein, in Chemical Formula A8,
a+b+c+d+e+f is 1 to 40, 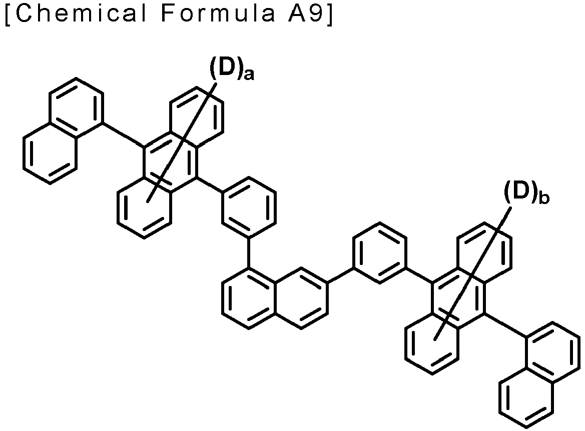
wherein, in Chemical Formula A9,
a+b is 1 to 16, 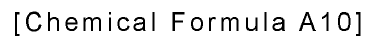 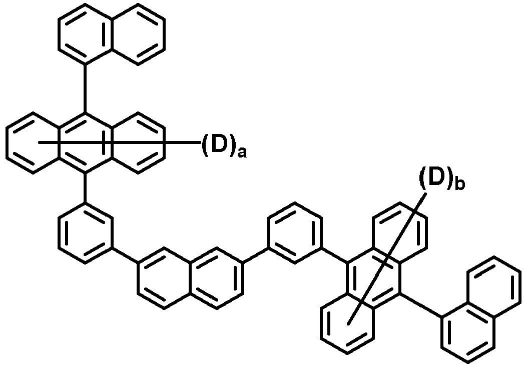
wherein, in Chemical Formula A10,
a+b is 1 to 16, 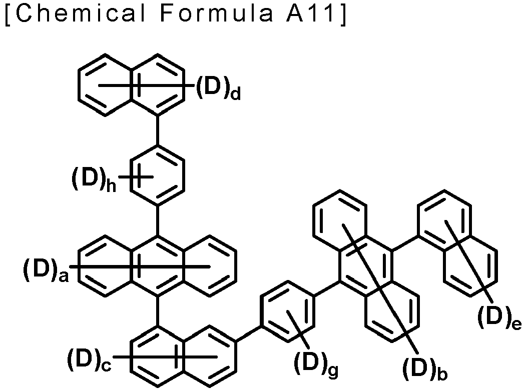
wherein, in Chemical Formula A11,
a+b+c+d+e+g+h is 1 to 44, 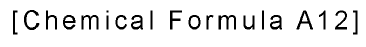 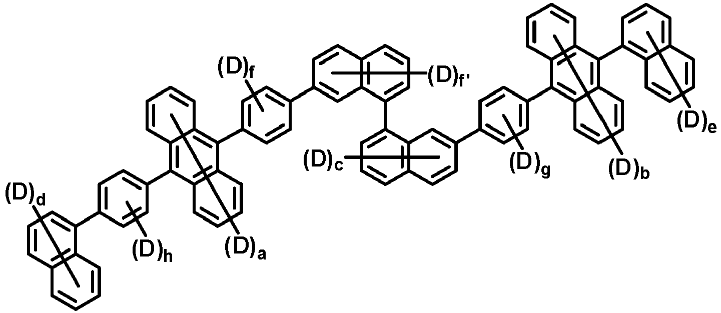
wherein, in Chemical Formula A12,
a+b+c+d+e+f+f'+g+h is 1 to 54, 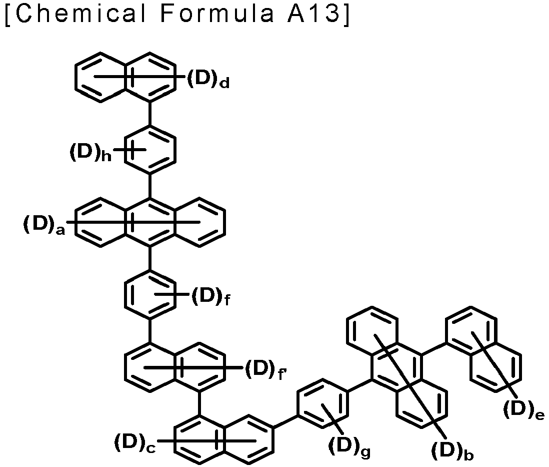
wherein, in Chemical Formula A13,
a+b+c+d+e+f+f'+g+h is 1 to 54, 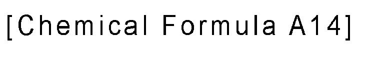 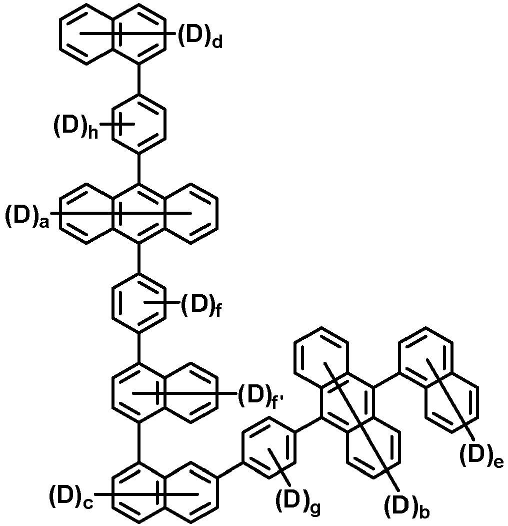
wherein, in Chemical Formula A14,
a+b+c+d+e+f+f'+g+h is 1 to 54, 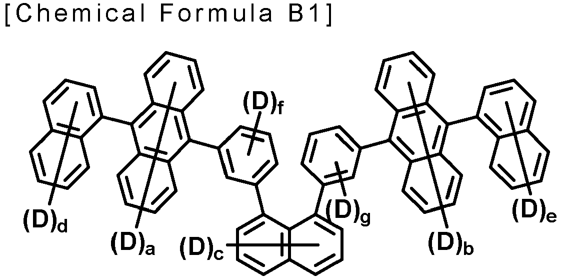
wherein, in Chemical Formula B1,
a+b+c+d+e+f+g is 1 to 44, 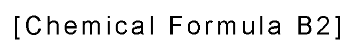 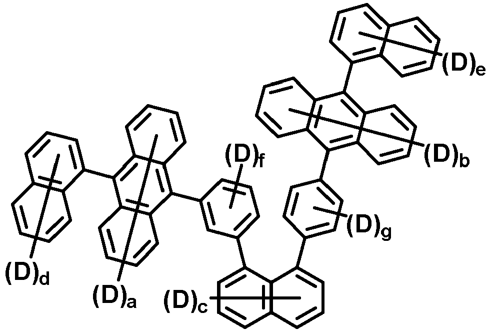
wherein, in Chemical Formula B2,
a+b+c+d+e+f+g is 1 to 44, 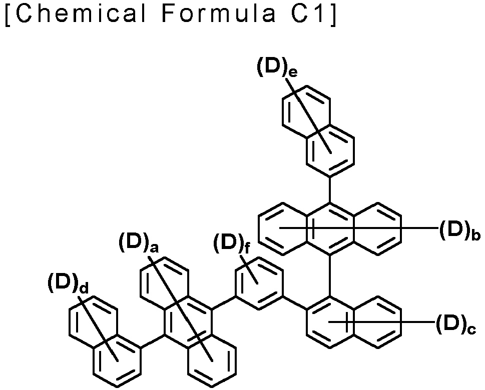
wherein, in Chemical Formula C1,
a+b+c+d+e+f is 1 to 40, 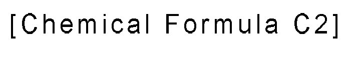 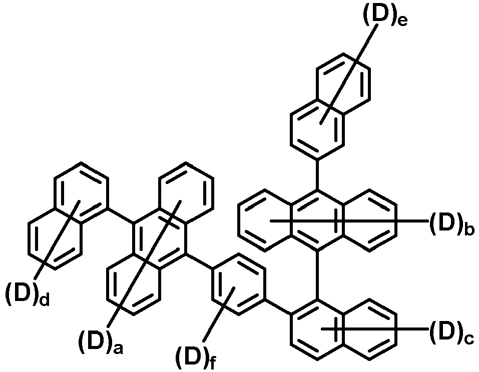
wherein, in Chemical Formula C2,
a+b+c+d+e+f is 1 to 40, 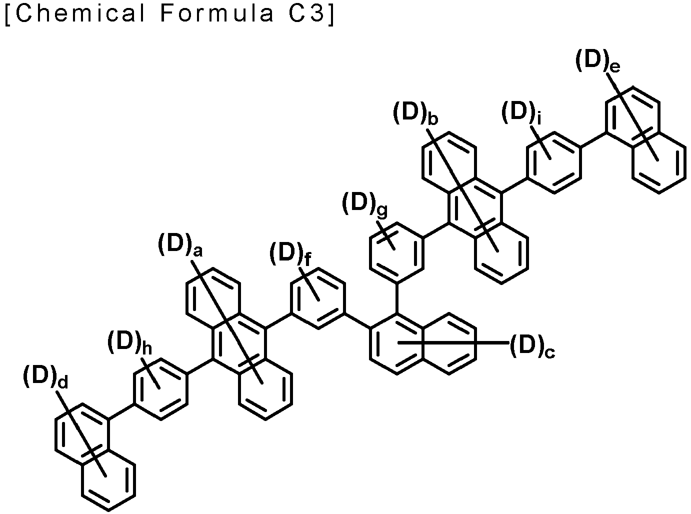
wherein, in Chemical Formula C3,
a+b+c+d+e+f+g+h+i is 1 to 52, 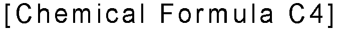 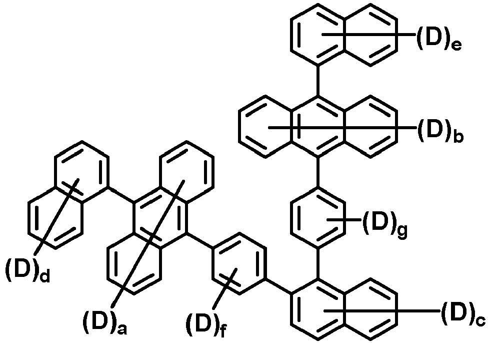
wherein, in Chemical Formula C4,
a+b+c+d+e+f+g is 1 to 44, 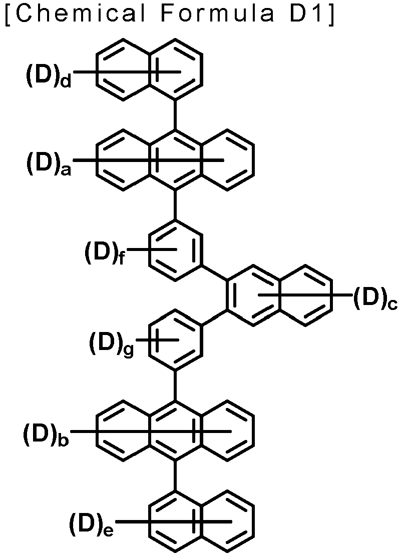
wherein, in Chemical Formula D1,
a+b+c+d+e+f+g is 1 to 44, 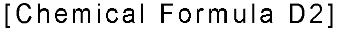 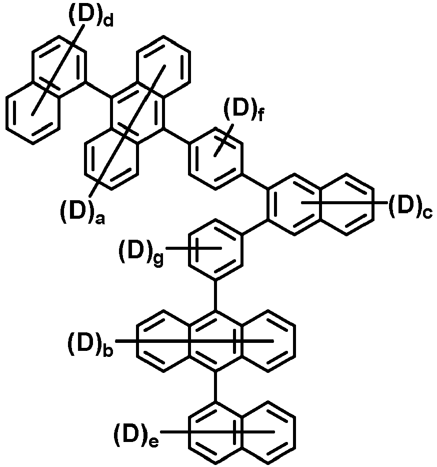
wherein, in Chemical Formula D2,
a+b+c+d+e+f+g is 1 to 44, 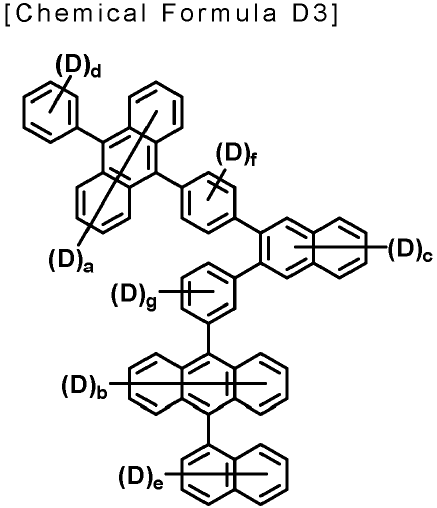
wherein, in Chemical Formula D3,
a+b+c+d+e+f+g is 1 to 42, 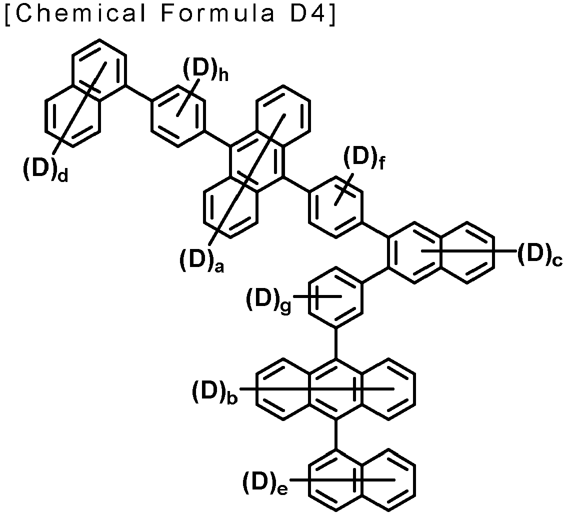
wherein, in Chemical Formula D4,
a+b+c+d+e+f+g+h is 1 to 48, 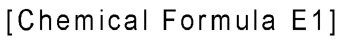 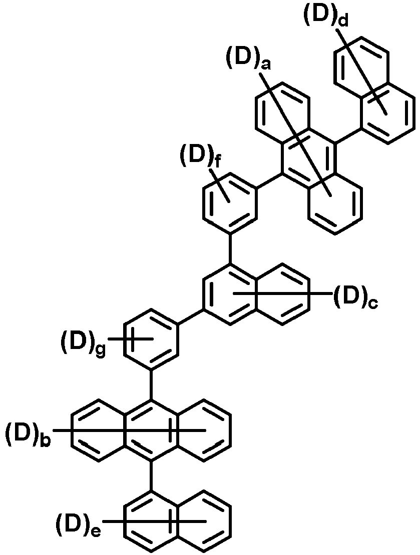
wherein, in Chemical Formula E1,
a+b+c+d+e+f+g is 1 to 44, 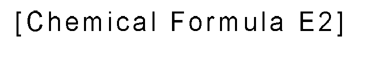 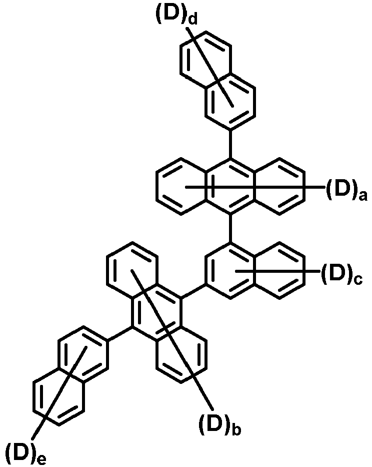
wherein, in Chemical Formula E2,
a+b+c+d+e is 1 to 36, 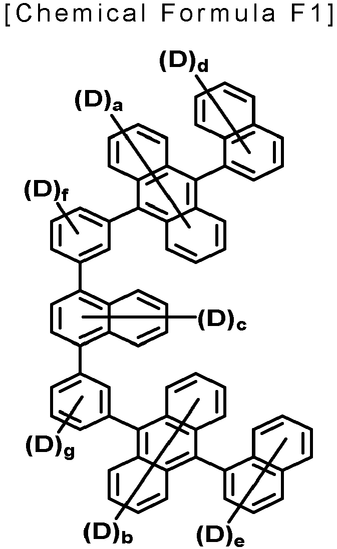
wherein, in Chemical Formula F1,
a+b+c+d+e+f+g is 1 to 44, 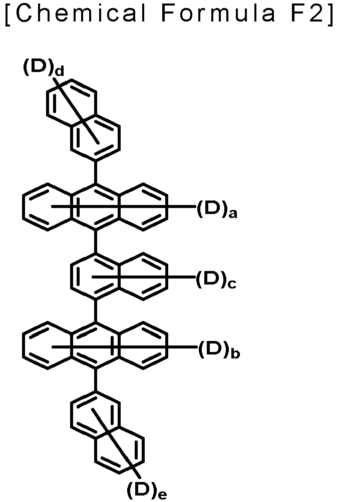
wherein, in Chemical Formula F2,
a+b+c+d+e is 1 to 36, 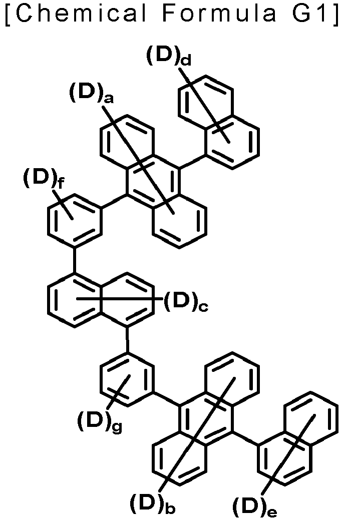
wherein, in Chemical Formula G1,
a+b+c+d+e+f+g is 1 to 44, 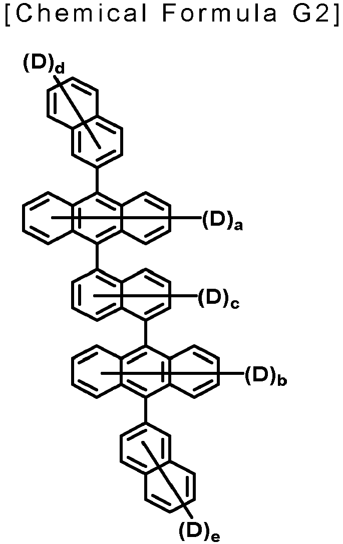
wherein, in Chemical Formula G2,
a+b+c+d+e is 1 to 36, 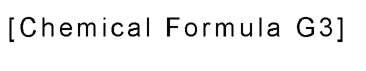 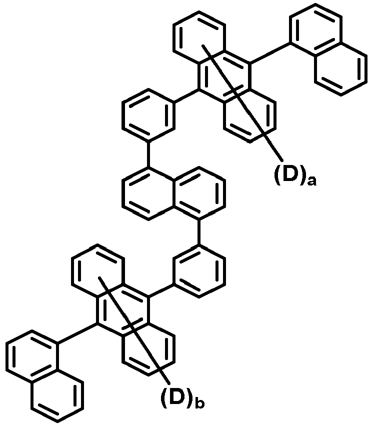
wherein, in Chemical Formula G3,
a+b is 1 to 16, 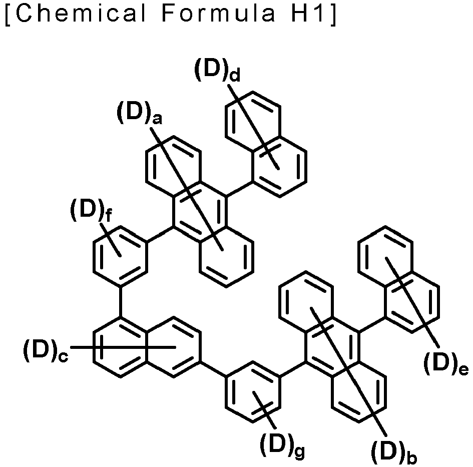
wherein, in Chemical Formula H1,
a+b+c+d+e+f+g is 1 to 44, 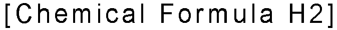 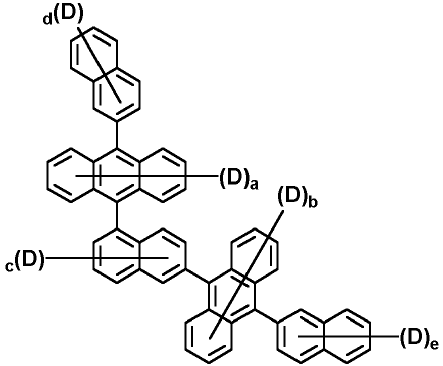
wherein, in Chemical Formula H2,
a+b+c+d+e is 1 to 36, 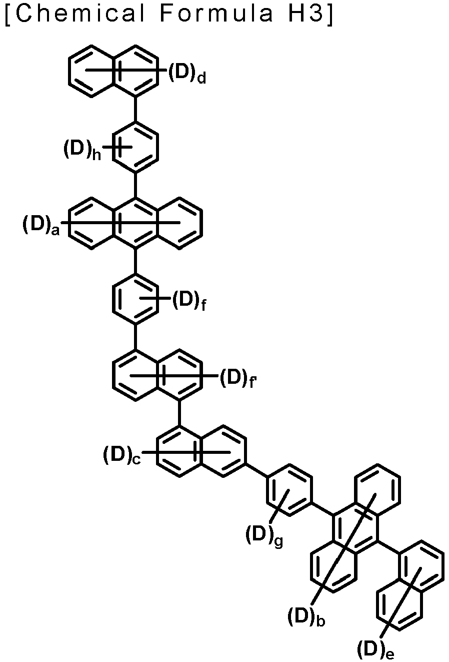
wherein, in Chemical Formula H3,
a+b+c+d+e+f+f'+g+h is 1 to 54, 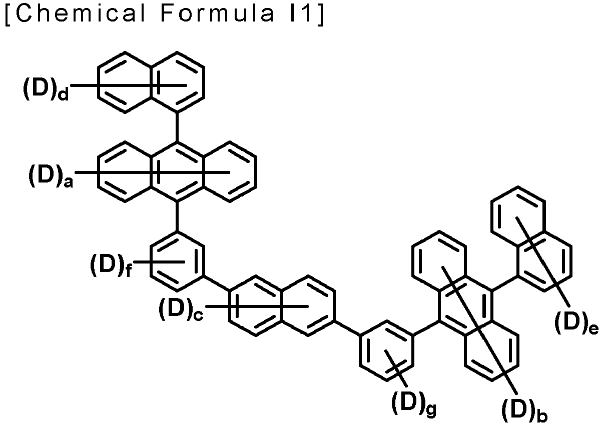
wherein, in Chemical Formula I1,
a+b+c+d+e+f+g is 1 to 44, 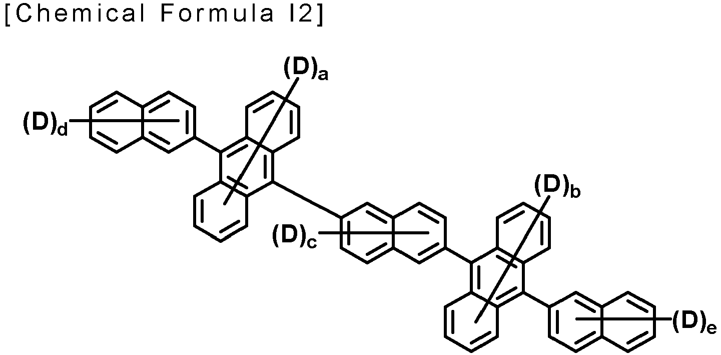
wherein, in Chemical Formula I2,
a+b+c+d+e is 1 to 36, 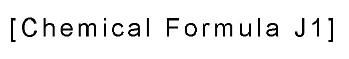 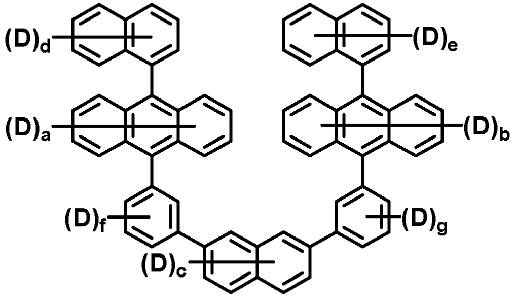
wherein, in Chemical Formula J1,
a+b+c+d+e+f+g is 1 to 36, 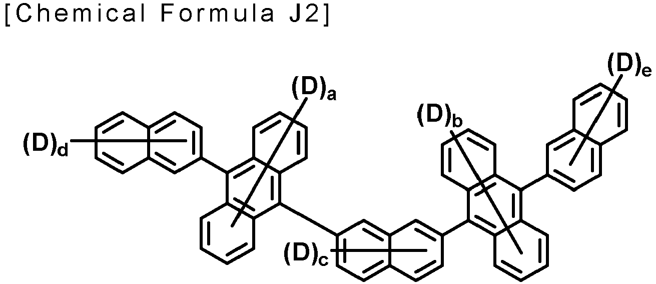
wherein, in Chemical Formula J2,
a+b+c+d+e is 1 to 36.

Meanwhile, the compound represented by Chemical Formula 1 can be prepared by the preparation method as shown in the following Reaction Scheme 1 as an example. 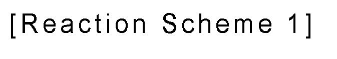 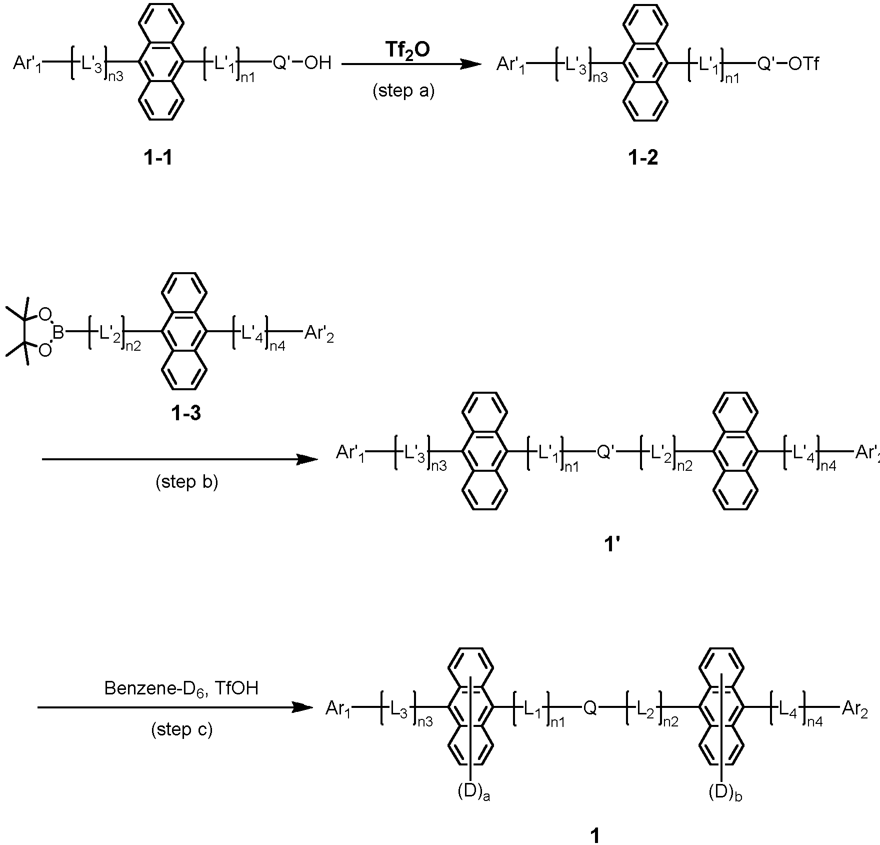 in Reaction Scheme 1, L'₁, L'₂, L'₃, L'₄, Ar'₁, Ar'₂ and Q' each represent substituents in which L₁, L₂, L₃, L₄, Ar₁, Ar₂ and Q are not deuterated, and the definitions of other substituents are the same as described above.

Specifically, the compound represented by Chemical Formula 1 can be prepared by steps a to c.

First, the step a is a step of preparing Compound 1-2 by introducing a -OTf(-O₃SCF₃) group, which is a reactive group for a Suzuki coupling reaction, to compound 1-1 using trifluoromethanesulfonic anhydride (Tf₂O).

Next, the step b is a step of preparing the compound represented by Chemical Formula 1' through the Suzuki coupling reaction of Compounds 1-2 and 1-3. The Suzuki coupling reaction is preferably carried out in the presence of a palladium catalyst and a base. Also, a reactive group for the Suzuki coupling reaction can be appropriately changed.

Next, the step c is a step of preparing a compound represented by Chemical Formula 1 by deuterating the intermediate compound 1', wherein the deuterium substitution reaction can be performed by adding the intermediate compound 1' to a deuterated solvent such as a benzene-D₆ (CeDe) solution and then reacting it with TfOH (trifluoromethanesulfonic acid).

Further, the compound represented by Chemical Formula 1 can be prepared by a preparation method as shown in the following Reaction Scheme 2 as an example. 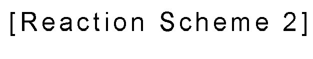 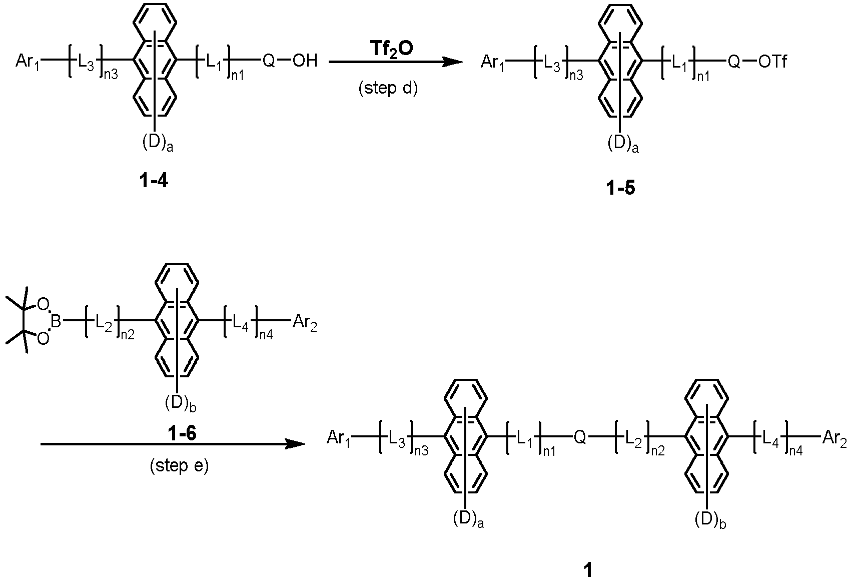 in Reaction Scheme 2, the definition of each substituent is the same as described above.

Specifically, the compound represented by Chemical Formula 1 can be prepared through steps d and e.

First, the step d is a step of preparing compound 1-5 by introducing a -OTf(-O₃SCF₃) group, which is a reactive group for Suzuki coupling reaction, to Compound 1-4 using Tf₂O.

Next, the step e is a step of preparing the compound represented by Chemical Formula 1 through the Suzuki coupling reaction of Compounds 1-5 and 1-6. The Suzuki coupling reaction is preferably performed in the presence of a palladium catalyst and a base, respectively, and the reactive group for the Suzuki coupling reaction may be appropriately changed.

Such a method for preparing the compound represented by Chemical Formula 1 can be further embodied in Preparation Examples described hereinafter.

Meanwhile, the organic material layer containing the compound according to the present disclosure can be formed by using various methods such as vacuum deposition, solution process, and the like, and the solution process will be described in detail below.

### (Coating Composition)

On the other hand, the compound according to the present disclosure can form an organic material layer, particularly a light emitting layer, of an organic light emitting device by a solution process. Specifically, the compound may be used as a host material of the light emitting layer. For this purpose, the present disclosure provides a coating composition including the above-mentioned compound according to the present disclosure and a solvent.

The solvent is not particularly limited as long as it is a solvent capable of dissolving or dispersing the compound according to the present disclosure. Examples of the solvent may include chlorine-based solvents such as chloroform, methylene chloride, 1,2-dichloroethane, 1,1,2-trichloroethane, chlorobenzene and o-dichlorobenzene; ether-based solvents such as tetrahydrofuran and dioxane; aromatic hydrocarbon-based solvents such as toluene, xylene, trimethylbenzene, mesitylene, 1-methylnaphthalene and 2-methylnaphthalene; aliphatic hydrocarbon-based solvents such as cyclohexane, methylcyclohexane, n-pentane, n-hexane, n-heptane, n-octane, n-nonane and n-decane; ketone-based solvents such as acetone, methyl ethyl ketone, and cyclohexanone; ester-based solvents such as ethyl acetate, butyl acetate and ethyl cellosolve acetate; polyalcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin and 1,2-hexanediol, and derivatives thereof; alcohol-based solvents such as methanol, ethanol, propanol, isopropanol and cyclohexanol; sulfoxide-based solvents such as dimethyl sulfoxide; amide-based solvents such as N-methyl-2-pyrrolidone and N,N-dimethylformamide; benzoate-based solvents such as butylbenzoate, methyl-2-methoxybenzoate, and ethyl benzoate; phthalate-based solvents such as dimethyl phthalate, diethyl phthalate, and diphenyl phthalate; tetraline; solvent such as 3-phenoxy-toluene, and the like. In addition, the above-mentioned solvents may be used singly or in combination of two or more solvents. Preferably, cyclohexanone may be used as the solvent.

Further, the coating composition may further include a compound used as a dopant material, and details of the compound used for the host material will be described later.

Further, the viscosity of the coating composition is preferably 1 cP or more. Also, in consideration of the easiness of coating the coating composition, the viscosity of the coating composition is preferably 10 cP or less. Further, the concentration of the compound according to the present disclosure in the coating composition is preferably 0.1 wt/v% or more. Further, in order for the coating composition to be optimally coated, the concentration of the compound according to the present disclosure in the coating composition is preferably 20 wt/v% or less.

Further, the solubility (wt%) of the compound represented by Chemical Formula 1 at room temperature/atmospheric pressure may be 0.1 wt% or more, more specifically 0.1 wt% to 5 wt%, based on the solvent cyclohexanone. Thereby, the coating composition containing the compound represented by Chemical Formula 1 and the solvent may be used in a solution process.

In another embodiment of the present disclosure, there is provided a method for forming a light emitting layer using the above-mentioned coating composition. Specifically, the method includes the steps of: coating the above-mentioned coating composition according to the present disclosure onto the anode by a solution process; and heat-treating the coated coating composition.

The solution process uses the above-mentioned coating composition according to the present disclosure, and refers to spin coating, dip coating, doctor blading, inkjet printing, screen printing, spray method, roll coating, and the like, but is not limited thereto.

The heat treatment temperature in the heat treatment step is preferably from 150 to 230°C. Further, a heat treatment time may be 1 minute to 3 hours, more preferably 10 minutes to 1 hour. Further, the heat treatment is preferably carried out in an inert gas atmosphere such as argon and nitrogen.

### (Organic Light Emitting Device)

In another embodiment of the present disclosure, there is provided an organic light emitting device including the compound represented by Chemical Formula 1. In one example, the present disclosure provides an organic light emitting device comprising: a first electrode; a second electrode that is provided opposite to the first electrode; and a light emitting layer that is provided between the first electrode and the second electrode, wherein the light emitting layer includes the compound represented by Chemical Formula 1.

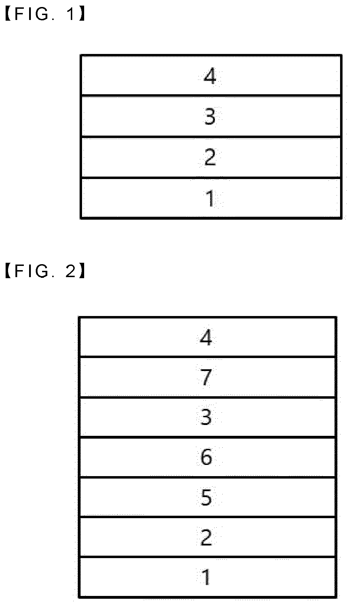

Further, the organic light emitting device according to the present disclosure may be a normal type organic light emitting device in which an anode, one or more organic material layers and a cathode are sequentially stacked on a substrate in which the first electrode is an anode, and the second electrode is a cathode. Further, the organic light emitting device according to the present disclosure may be an inverted type organic light emitting device in which a cathode, one or more organic material layers and an anode are sequentially stacked on a substrate in which the first electrode is a cathode, and the second electrode is an anode. For example, the structure of an organic light emitting device according to an embodiment of the present disclosure is illustrated in FIGS. 1 and 2.

FIG. 1 shows an example of an organic light emitting device comprising a substrate 1, an anode 2, a light emitting layer 3, and a cathode 4. In such a structure, the compound represented by Chemical Formula 1 may be included in the light emitting layer.

FIG. 2 shows an example of an organic light emitting device comprising a substrate 1, an anode 2, a hole injection layer 5, a hole transport layer 6, a light emitting layer 3, an electron injection and transport layer 7, and a cathode 4. In such a structure, the compound represented by Chemical Formula 1 may be included in the light emitting layer.

The organic light emitting device according to the present disclosure may be manufactured by materials and methods known in the art, except that the light emitting layer includes the compound according to the present disclosure and is manufactured as described above.

For example, the organic light emitting device according to the present disclosure can be manufactured by sequentially stacking an anode, an organic material layer and a cathode on a substrate. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming organic material layers including the hole injection layer, the hole transport layer, the light emitting layer and the electron transport layer thereon, and then depositing a material that can be used as the cathode thereon.

In addition to such a method, the organic light emitting device may be manufactured by sequentially depositing a cathode material, an organic material layer and an anode material on a substrate (International Publication WO2003/012890). However, the manufacturing method is not limited thereto.

In one example, the first electrode is an anode, and the second electrode is a cathode, or alternatively, the first electrode is a cathode and the second electrode is an anode.

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides, such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

The hole injection layer is a layer for injecting holes from the electrode, and the hole injection material is preferably a compound which has a capability of transporting the holes, thus has a hole injecting effect in the anode and an excellent hole injecting effect to the light emitting layer or the light emitting material, prevents excitons produced in the light emitting layer from moving to an electron injection layer or the electron injection material, and further is excellent in the ability to form a thin film. It is preferable that a HOMO (highest occupied molecular orbital) of the hole injection material is between the work function of the anode material and a HOMO of a peripheral organic material layer. Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

The hole transport layer is a layer that receives holes from a hole injection layer and transports the holes to the light emitting layer. The hole transport material is suitably a material having large mobility to the holes, which may receive holes from the anode or the hole injection layer and transfer the holes to the light emitting layer. As the hole transport material, the compound represented by Chemical Formula 1 may be used, or an arylamine-based organic material, a conductive polymer, and a block copolymer in which a conjugate portion and a non-conjugate portion are present together, and the like may be used, but the present disclosure is not limited thereto.

Meanwhile, the organic light emitting device may be provided with an electron inhibition layer between the hole transport layer and the light emitting layer. The electron inhibition layer refers to a layer which is formed on the hole transport layer, and preferably, is provided in contact with the light emitting layer, and thus serves to control hole mobility, to prevent excessive movement of electrons, and to increase the probability of hole-electron bonding, thereby improving the efficiency of the organic light emitting device. The electron inhibition layer includes an electron blocking material, and as an example of such an electron blocking material, a compound represented by the Chemical Formula 1 may be used, or an arylamine-based organic material of the like may be used, but is not limited thereto.

The light emitting layer may include a host material and a dopant material. As the host material, the above-mentioned compound represented by Chemical Formula 1 can be used. The host material may be a fused aromatic ring derivative, a heterocycle-containing compound or the like together with the compound represented by Chemical Formula 1. Specific examples of the fused aromatic ring derivatives include anthracene derivatives, pyrene derivatives, naphthalene derivatives, pentacene derivatives, phenanthrene compounds, fluoranthene compounds, and the like. Examples of the heterocyclic-containing compounds include carbazole derivatives, dibenzofuran derivatives, ladder-type furan compounds, pyrimidine derivatives, and the like, but are not limited thereto.

Further, examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

Meanwhile, the organic light emitting device may be provided with a hole blocking layer between the light emitting layer and the electron transport layer. The hole blocking layer refers to a layer which is formed on the light emitting layer, and preferably, is provided in contact with the light emitting layer, and thus severs to control electron mobility, to prevent excessive movement of holes, and to increase the probability of hole-electron bonding, thereby improving the efficiency of the organic light emitting device. The hole blocking layer includes a hole blocking material, and as an example of such hole blocking material, a compound into which an electron-withdrawing group is introduced, such as azine derivatives including triazine; triazole derivatives; oxadiazole derivatives; phenanthroline derivatives; phosphine oxide derivatives can be used, but is not limited thereto.

The electron injection and transport layer is a layer for simultaneously performing the roles of an electron transport layer and an electron injection layer that inject electrons from an electrode and transport the received electrons up to the light emitting layer, and is formed on the light emitting layer or the hole blocking layer. The electron injection and transport material is suitably a material which can receive electrons well from a cathode and transfer the electrons to a light emitting layer, and has a large mobility for electrons. Specific examples of the electron injection and transport material include: an Al complex of 8-hydroxyquinoline; a complex including Alqs; an organic radical compound; a hydroxyflavone-metal complex, a triazine derivative, and the like, but are not limited thereto. Alternatively, it may be used together with fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

The electron injection and transport layer may also be formed as a separate layer such as an electron injection layer and an electron transport layer. In such a case, the electron transport layer is formed on the light emitting layer or the hole blocking layer, and the above-mentioned electron injection and transport material may be used as the electron transport material included in the electron transport layer. In addition, the electron injection layer is formed on the electron transport layer, and examples of the electron injection material included in the electron injection layer include LiF, NaCl, CsF, Li₂O, BaO, fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

In addition to the above-mentioned materials, the light emitting layer, the hole injection layer, the hole transport layer, the electron transport layer, and the electron injection layer may further include an inorganic compound or a polymer compound such as quantum dots.

The quantum dots may be, for example, colloidal quantum dots, alloy quantum dots, core-shell quantum dots, or core quantum dots. It may be a quantum dot including elements belonging to groups 2 and 16, elements belonging to groups 13 and 15, elements belonging to groups 13 and 17, elements belonging to groups 11 and 17, or elements belonging to groups 14 and 15. Quantum dots including elements such as cadmium (Cd), selenium (Se), zinc (Zn), sulfur (S), phosphorus (P), indium (In), tellurium (Te), lead (Pb), gallium (Ga), arsenic (As) may be used.

The organic light emitting device according to the present disclosure may be a bottom emission device, a top emission device, or a double-sided light emitting device, and in particular, may be a bottom emission device that requires relatively high luminous efficiency.

In addition, the compound according to the present disclosure may be included in an organic solar cell or an organic transistor in addition to an organic light emitting device.

Hereinafter, the preparation of the compound represented by Chemical Formula 1 and the organic light emitting device including the same will be specifically described in the following Examples.

### [PREPARATION EXAMPLE]

### Preparation Example 1: Preparation of Compound A1

### Step 1-1) Preparation of Compound 1-c

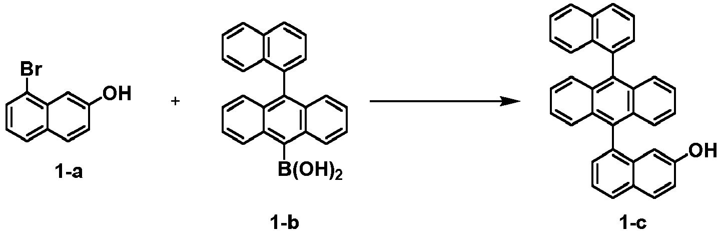

Compound 1-a (10.0 g, 1.0 eq.) and Compound 1-b (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred for 2 days. After the reaction, the reaction mixture was stirred and then cooled to room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 1-c (yield: 93%).
m/z [M+H]⁺ = 447.2

### Step 1-2) Preparation of Compound 1-d

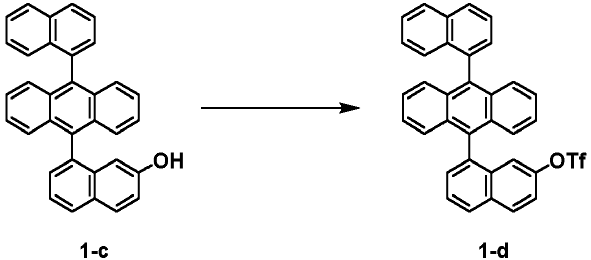

Compound 1-c (15 g, 1.0 eq.) was placed in a round bottom flask and dissolved in anhydrous CH₂Cl₂. Then, pyridine (2.0 eq.) was added dropwise to the reaction solution at room temperature, the bath temperature was lowered to 0°C, and the mixture was stirred for 10 minutes. Tf₂O (1.2 eq.) dissolved in anhydrous CH₂Cl₂ was slowly added dropwise to the mixture using a dropping funnel, the bath temperature was gradually raised from 0°C to room temperature, and the mixture was stirred overnight. After the reaction, the reaction mixture was sufficiently diluted with CH₂Cl₂, and then washed with CH₂Cl₂/brine. The organic layer was separated, water was removed with MgSO₄ and passed through a Celite-Florisil-Silica pad. The passed solution was concentrated under reduced pressure, and then purified by column chromatography to prepare Compound 1-d (yield: 98%).
m/z [M+H]⁺ = 579.1

### Step 1-3) Preparation of Compound A1'

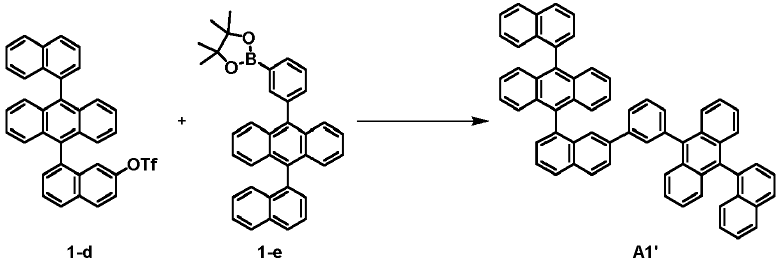

Compound 1-d (3 g, 1.0 eq.) and Compound 1-e (1.03 eq.) were placed in a round bottom flask and dissolved in PhMe. C₂CO₃ (5 eq.) dissolved in water was injected. Pd(PPh₃)₄ (10 mol%) was added dropwise thereto under a bath temperature of 90°C, and the mixture was stirred for 2 days. After the reaction, the reaction mixture was stirred and then cooled to room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound A1' (yield: 91%).
m/z [M+H]⁺ = 809.3

### Step 1-4) Preparation of Compound A1

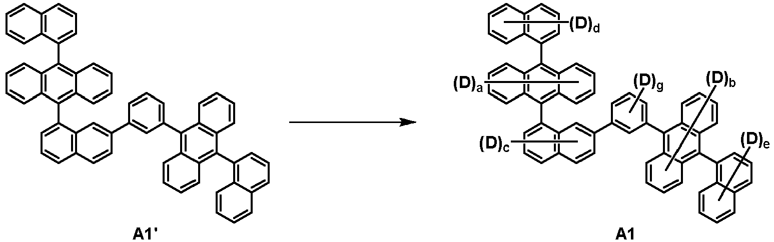

Compound A1' (2.0 g, 1.0 eq.) was placed in a round bottom flask under a nitrogen atmosphere and dissolved in benzene-D₆ (150 eq.). TfOH (2.0 eq.) was slowly added dropwise to the reaction solution, and the mixture was stirred at 25°C for 2 hours. D₂O was dropwise added to the reaction solution to terminate the reaction, and potassium phosphate tribasic (30 wt% in aqueous solution, 2.4 eq.) was added dropwise thereto and the pH of the aqueous layer was adjusted to 9-10. The reaction mixture was washed with CH₂Cl₂/DI water and the organic layer was separated. Water was removed with MgSO₄ and passed through a Celite-Florisil-Silica pad. The passed solution was concentrated under reduced pressure and purified by column chromatography to prepare Compound A1 (a+b+c+d+e+g=36-40, yield: 96%).
m/z [M+H]+ = 849.3

### Preparation Example 2: Preparation of Compound A2

### Step 2-1) Preparation of Compound A2'

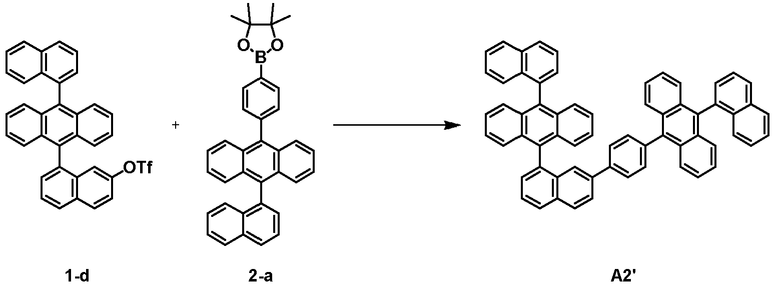

Compound A2' (yield: 92%) was prepared in the same manner as in the preparation method of Compound A1', except that Compound 2-a was used instead of Compound 1-e.
m/z [M+H]⁺ = 809.3

### Step 2-2) Preparation of Compound A2

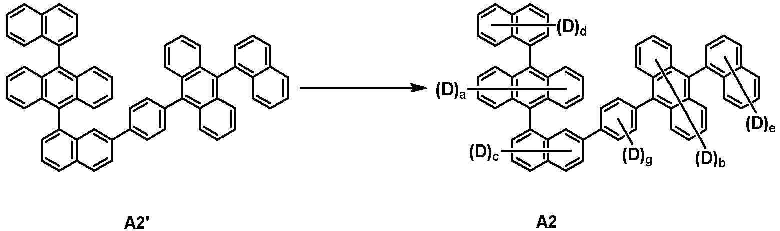

Compound A2 (a+b+c+d+e+g=36-40, yield:88%) was prepared in the same manner as in the preparation method of Compound A1, except that Compound A2' was used instead of Compound A1'.
m/z [M+H]⁺ = 849.3

### Preparation Example 3: Preparation of Compound A3

### Step 3-1) Preparation of Compound 3-a

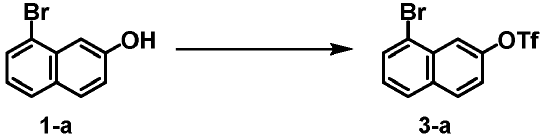

Compound 1-a (20 g, 1.0 eq.) was placed in a round bottom flask and dissolved in anhydrous CH₂Cl₂. Then, pyridine (2.0 eq.) was added dropwise to the reaction solution at room temperature, the bath temperature was lowered to 0°C, and the mixture was stirred for 10 minutes. Tf₂O (1.2 eq.) dissolved in anhydrous CH₂Cl₂ was slowly added dropwise to the mixture using a dropping funnel, the bath temperature was gradually raised from 0°C to room temperature, and the mixture was stirred overnight. After the reaction, the reaction mixture was sufficiently diluted with CH₂Cl₂, and then washed with CH₂Cl₂/brine. The organic layer was separated, water was removed with MgSO₄ and passed through a Celite-Florisil-Silica pad. The passed solution was concentrated under reduced pressure, and then purified by column chromatography to prepare Compound 3-a (yield: 99%).
m/z [M+H]⁺ = 354.9

### Step 3-2) Preparation of Compound A3'

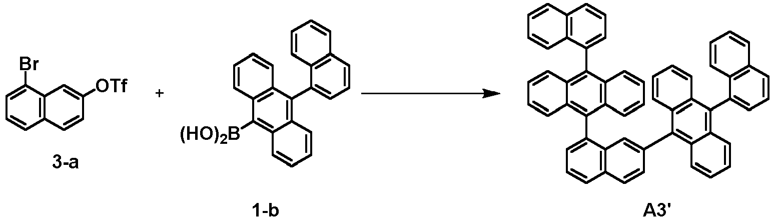

Compound 3-a (1 g, 1.0 eq.) and Compound 1-b (2.1 eq.) were placed in a round bottom flask and dissolved in PhMe(toluene). C₂CO₃ (10 eq.) dissolved in water was injected. Pd(PPh₃)₄ (20 mol%) was added dropwise thereto under a bath temperature of 90°C, and the mixture was stirred for 3 days. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound A3' (yield: 86%).
m/z [M+H]⁺ = 733.3

### Step 3-3) Preparation of Compound A3

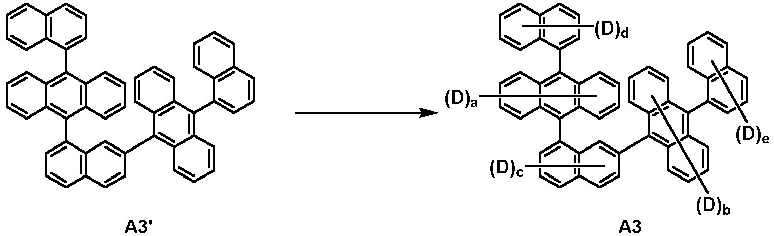

Compound A3 (a+b+c+d+e=34-36, yield:92%) was prepared in the same manner as in the preparation method of Compound A1, except that Compound A3' was used instead of Compound A1'.
m/z [M+H]⁺ = 769.3

### Preparation Example 4: Preparation of Compound A4

### Step 4-1) Preparation of Compound A4'

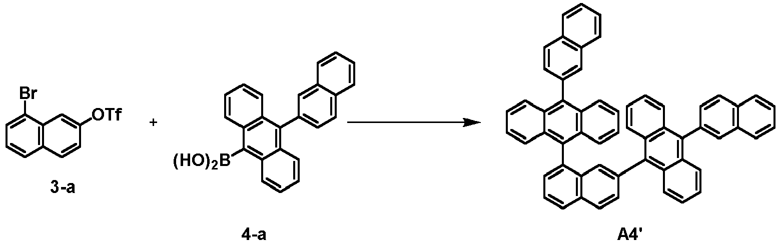

Compound A4' was prepared in the same manner as in the preparation method of Compound A3', except that Compound 4-a was used instead of Compound 1-b.
m/z [M+H]⁺ = 733.3

### Step 4-2) Preparation of Compound A4

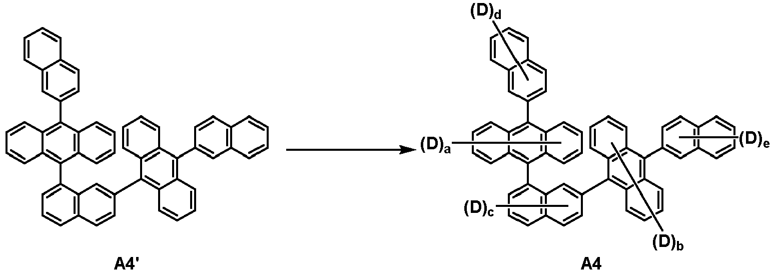

Compound A4 (a+b+c+d+e=34-36, yield:91%) was prepared in the same manner as in the preparation method of Compound A1, except that Compound A4' was used instead of Compound A1'.
m/z [M+H]⁺ = 769.3

### Preparation Example 5: Preparation of Compound A5

### Step 5-1) Preparation of Compound A5'

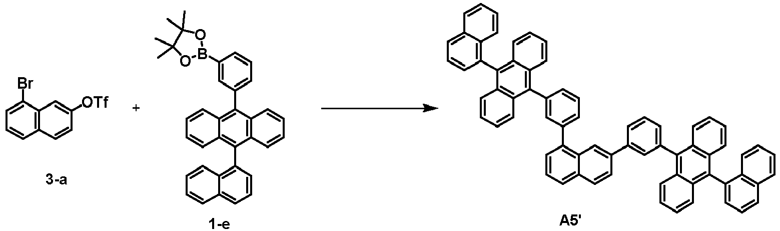

Compound A5' was prepared in the same manner as in the preparation method of Compound A3', except that Compound 1-e was used instead of Compound 1-b.
m/z [M+H]⁺ = 885.3

### Step 5-2) Preparation of Compound A5

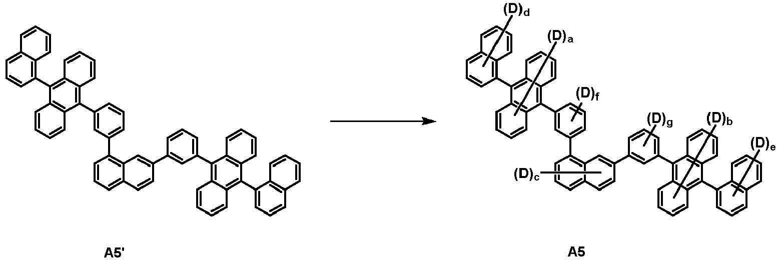

Compound A5 (a+b+c+d+e+f+g=40-44, yield:90%) was prepared in the same manner as in the preparation method of Compound A1, except that Compound A5' was used instead of Compound A1'.
m/z [M+H]⁺ = 929.3

### Preparation Example 6: Preparation of Compound A6

### Step 6-1) Preparation of Compound A6'

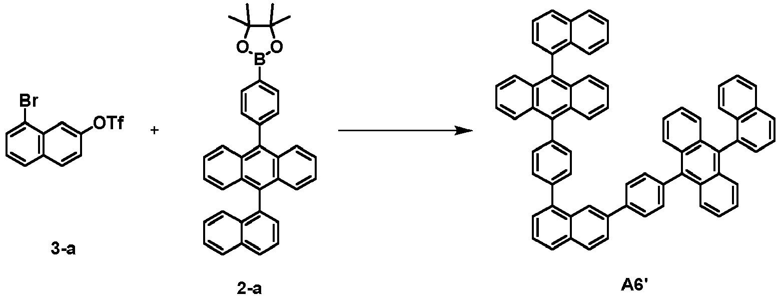

Compound A6' was prepared in the same manner as in the preparation method of Compound A3', except that Compound 2-a was used instead of Compound 1-b.
m/z [M+H]⁺ = 885.3

### Step 6-2) Preparation of Compound A6

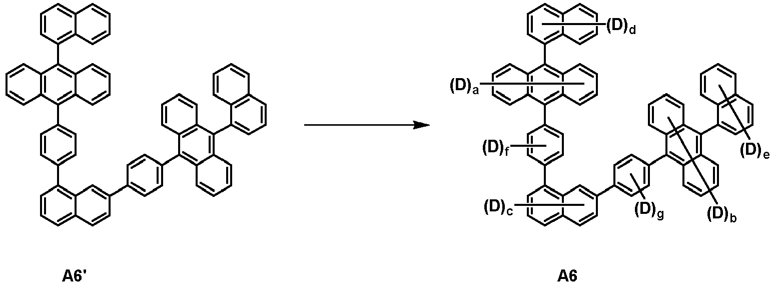

Compound A6 (a+b+c+d+e+f+g=40-44, yield:90%) was prepared in the same manner as in the preparation method of Compound A1, except that Compound A6' was used instead of Compound A1'.
m/z [M+H]⁺ = 929.3

### Preparation Example 7: Preparation of Compound A7

### Step 7-1) Preparation of Compound 7-a

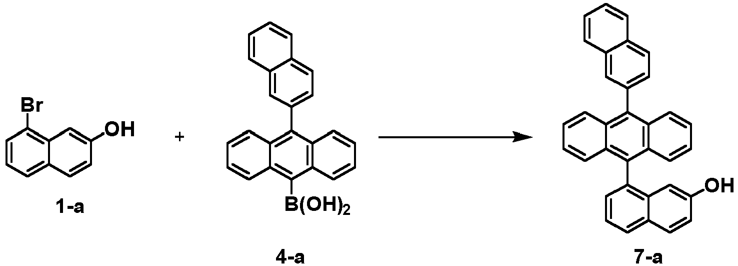

Compound 7-a (yield: 92%) was prepared in the same manner as in the preparation method of Compound 1-c, except that Compound 4-a was used instead of Compound 1-b.
m/z [M+H]⁺ = 447.2

### Step 7-2) Preparation of Compound 7-b

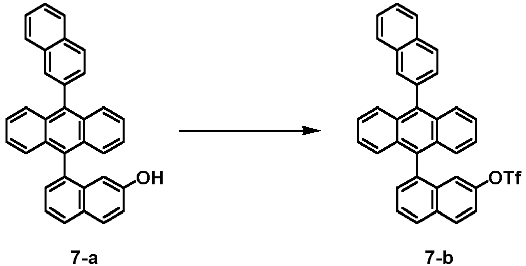

Compound 7-b (yield: 99%) was prepared in the same manner as in the preparation method of Compound 1-d, except that Compound 7-a was used instead of Compound 1-c.
m/z [M+H]⁺ = 579.1

### Step 7-3) Preparation of Compound A7'

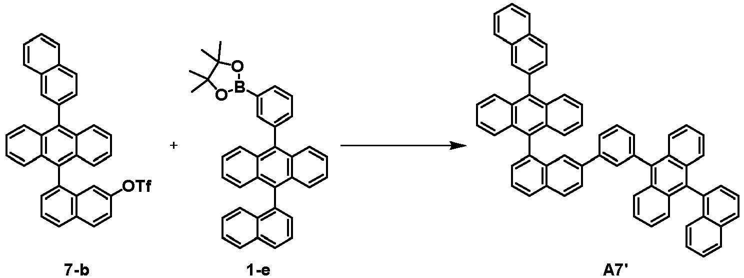

Compound A7' (yield: 84%) was prepared in the same manner as in the preparation method of Compound A1', except that Compound 7-b was used instead of Compound 1-d.
m/z [M+H]⁺ = 809.3

### Step 7-4) Preparation of Compound A7

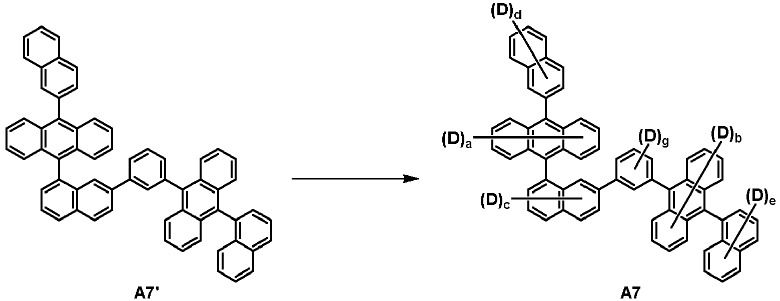

Compound A7 (a+b+c+d+e+g=36-40, (yield: 97%) was prepared in the same manner as in the preparation method of Compound A1', except that Compound A7' was used instead of Compound A1'.
m/z [M+H]⁺ = 849.3

### Preparation Example 8: Preparation of Compound A8

### Step 8-1) Preparation of Compound 8-a

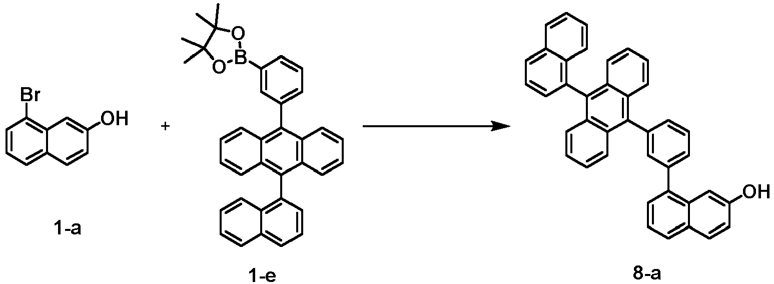

Compound 8-a (yield: 89%) was prepared in the same manner as in the preparation method of Compound 1-c, except that Compound 1-e was used instead of Compound 1-b.
m/z [M+H]⁺ = 523.2

### Step 8-2) Preparation of Compound 8-b

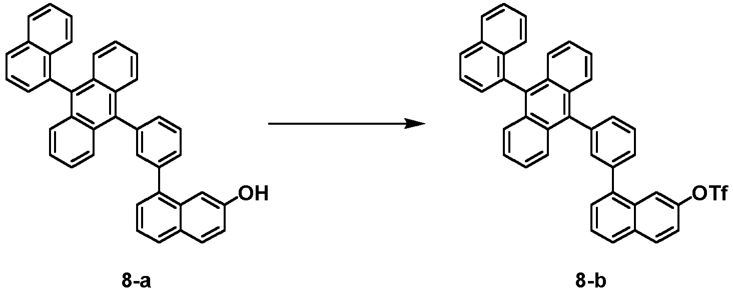

Compound 8-b (yield: 99%) was prepared in the same manner as in the preparation method of Compound 1-d, except that Compound 8-a was used instead of Compound 1-c.
m/z [M+H]⁺ = 655.2

### Step 8-3) Preparation of Compound A8'

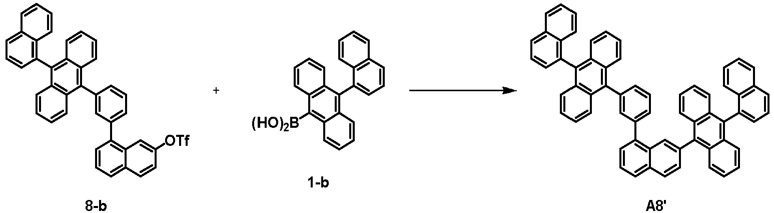

Compound 8-b (3 g, 1.0 eq.) and Compound 1-b (1.03 eq.) were placed in a round bottom flask and dissolved in PhMe. C₂CO₃ (5 eq.) dissolved in water was injected. Pd(PPh₃)₄ (10 mol%) was added dropwise thereto under a bath temperature of 90°C, and the mixture was stirred for 2 days. After the reaction, the reaction mixture was stirred and then cooled to room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound A8' (yield: 92%).
m/z [M+H]⁺ = 809.3

### Step 8-4) Preparation of Compound A8

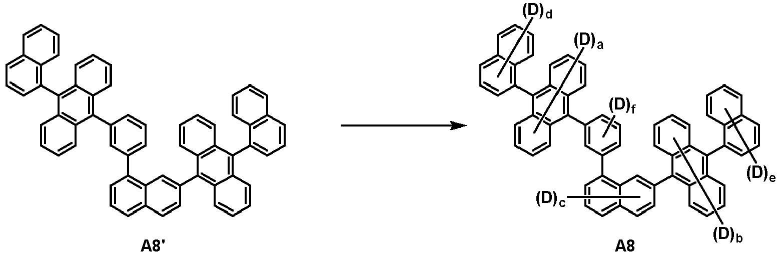

Compound A8 (a+b+c+d+e+f=36-40, yield: 93%) was prepared in the same manner as in the preparation method of Compound A1', except that Compound A8' was used instead of Compound A1'.
m/z [M+H]⁺ = 849.3

### Preparation Example 9: Preparation of Compound B1

### Step 9-1) Preparation of Compound B1'

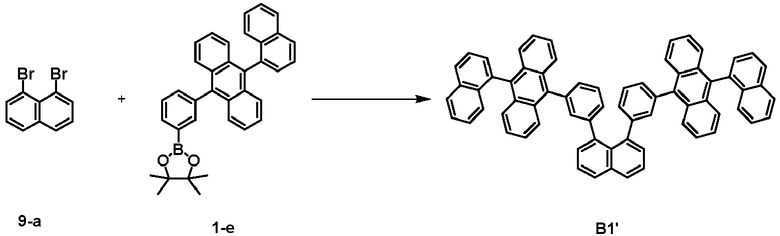

Compound 9-a (1 g, 1.0 eq.) and Compound 1-e (2.1 eq.) were placed in a round bottom flask and dissolved in PhMe. C₂CO₃ (10 eq.) dissolved in water was injected. Pd(PPh₃)₄ (20 mol%) was added dropwise thereto under a bath temperature of 90°C, and the mixture was stirred for 3 days. After the reaction, the reaction mixture was stirred and then cooled to room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound B1' (yield: 86%).
m/z [M+H]⁺ = 885.3

### Step 9-2) Preparation of Compound B1

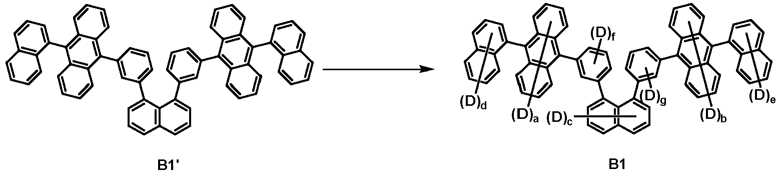

Compound B1 (a+b+c+d+e+f+g=40-44, yield: 90%) was prepared in the same manner as in the preparation method of Compound A1, except that Compound B1' was used instead of Compound A1'.
m/z [M+H]⁺ = 929.4

### Preparation Example 10: Preparation of Compound B2

### Step 10-1) Preparation of Compound 10-a

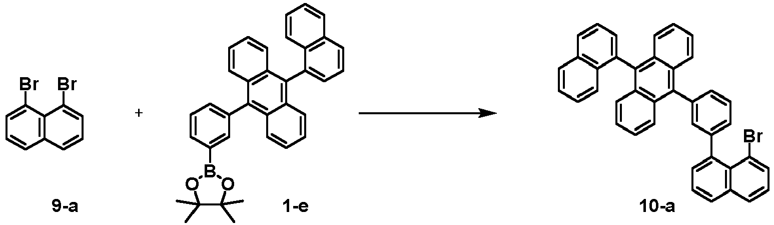

Compound 9-a (1 g, 1.0 eq.) and Compound 1-e (1.03 eq.) were placed in a round bottom flask and dissolved in PhMe. C₂CO₃ (5 eq.) dissolved in water was injected. Pd(PPh₃)₄ (10 mol%) was added dropwise thereto under a bath temperature of 90°C, and the mixture was stirred overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 10-a (yield: 83%).
m/z [M+H]⁺ = 585.1

### Step 10-2) Preparation of Compound B2'

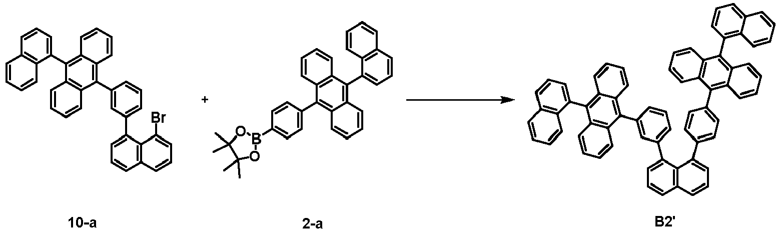

Compound 10-a (1 g, 1.0 eq.) and Compound 2-a (1.03 eq.) were placed in a round bottom flask and dissolved in PhMe. C₂CO₃ (5 eq.) dissolved in water was injected. Pd(PPh₃)₄ (10 mol%) was added dropwise thereto under a bath temperature of 90°C, and the mixture was stirred overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound B2' (yield: 85%).
m/z [M+H]⁺ = 585.1

### Step 10-2) Preparation of Compound B2

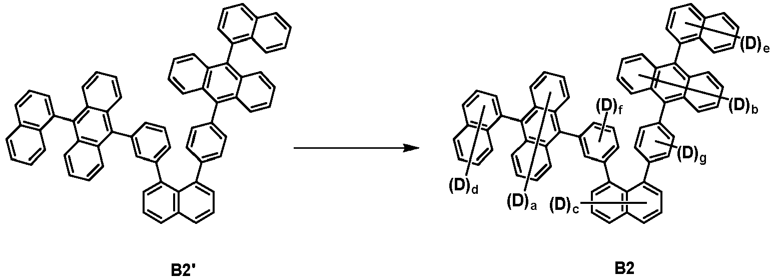

Compound B2 (a+b+c+d+e+f+g=40-44, yield: 87%) was prepared in the same manner as in the preparation method of Compound A1, except that Compound B2' was used instead of Compound A1'.
m/z [M+H]⁺ = 929.4

### Preparation Example 11: Preparation of Compound C1

### Step 11-1) Preparation of Compound 11-b

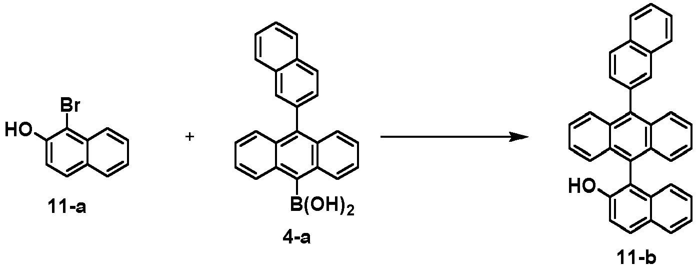

Compound 11-a (10.0 g, 1.0 eq.) and Compound 4-a (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred for 2 days. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 11-b (yield: 97%).
m/z [M+H]⁺ = 447.2

### Step 11-2) Preparation of Compound 11-c

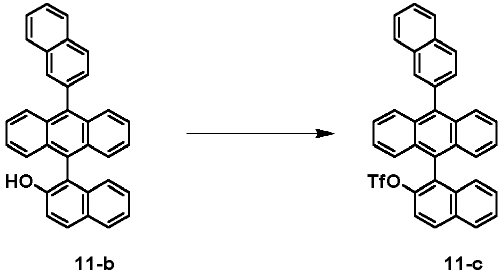

Compound 11-c (yield: 99%) was prepared in the same manner as in the preparation method of Compound 1-d, except that Compound 11-b was used instead of Compound 1-c.
m/z [M+H]⁺ = 579.1

### Step 11-3) Preparation of Compound C1'

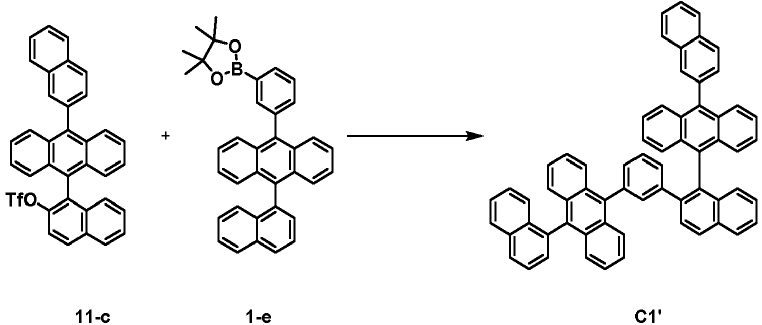

Compound C1' (yield: 92%) was prepared in the same manner as in the preparation method of Compound A1', except that Compound 11-c was used instead of Compound 1-d.
m/z [M+H]⁺ = 809.3

### Step 11-4) Preparation of Compound C1

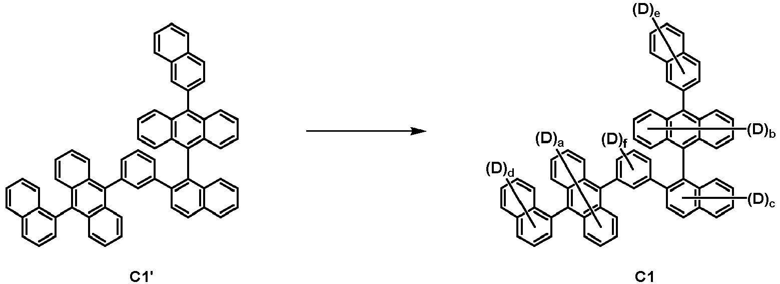

Compound C1 (a+b+c+d+e+f=36-40, yield: 92%) was prepared in the same manner as in the preparation method of Compound A1, except that Compound C1' was used instead of Compound A1'.
m/z [M+H]⁺ = 849.3

### Preparation Example 12: Preparation of Compound C2

### Step 12-1) Preparation of Compound C2'

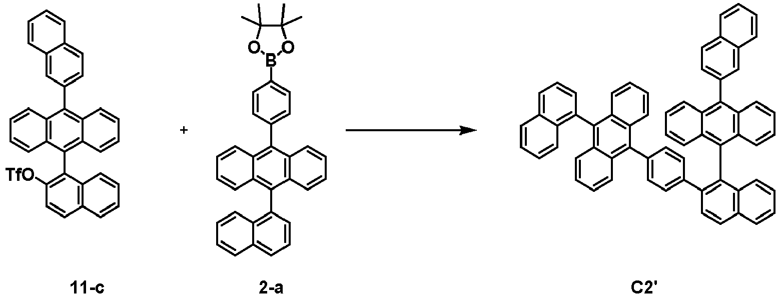

Compound C2' (yield: 96%) was prepared in the same manner as in the preparation method of Compound C1', except that Compound 2-a was used instead of Compound 1-e.
m/z [M+H]⁺ = 809.3

### Step 12-2) Preparation of Compound C2

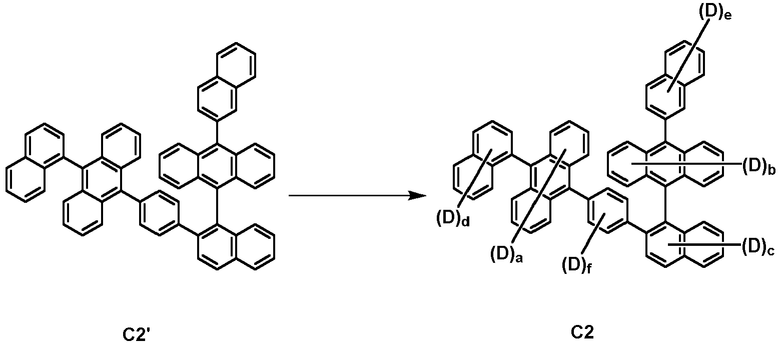

Compound C2 (a+b+c+d+e+f=36-40, yield: 88%) was prepared in the same manner as in the preparation method of Compound A1, except that Compound C2' was used instead of Compound A1'.
m/z [M+H]⁺ = 849.3

### Preparation Example 13: Preparation of Compound C3

### Step 13-1) Preparation of Compound C3'

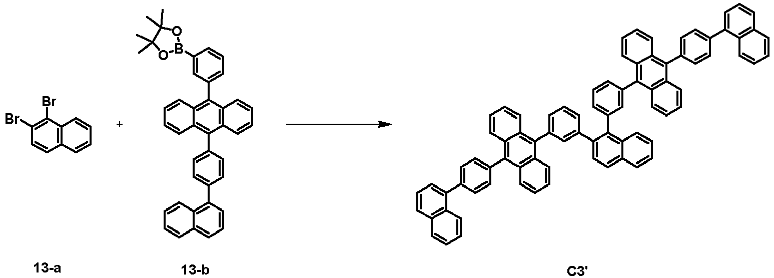

Compound 13-a (10.0 g, 1.0 eq.) and Compound 13-b (2.4 eq.) were placed in a round bottom flask and dissolved in anhydrous PhMe. C₂CO₃ (10 eq.) dissolved in water was injected. Pd(PPh₃)₄ (20 mol%) was added dropwise thereto under a bath temperature of 90°C, and the mixture was stirred for 2 days. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound C3' (yield: 83%).
m/z [M+H]⁺ = 1037.4

### Step 13-2) Preparation of Compound C3

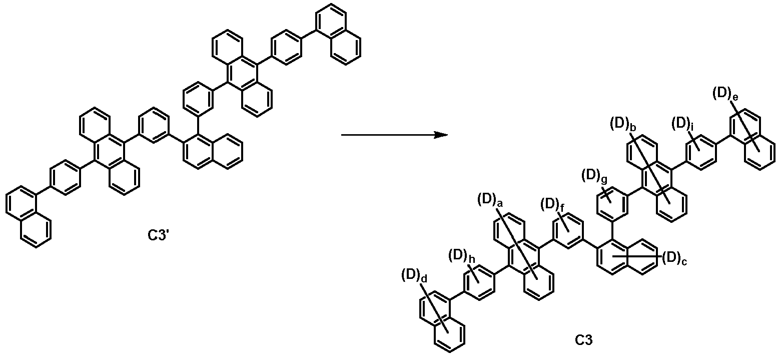

Compound C3 (a+b+c+d+e+f+g+h+i=48-52, yield: 93%) was prepared in the same manner as in the preparation method of Compound A1, except that Compound C3' was used instead of Compound A1'.
m/z [M+H]⁺ = 1089.2

### Preparation Example 14: Preparation of Compound C4

### Step 14-1) Preparation of Compound C4'

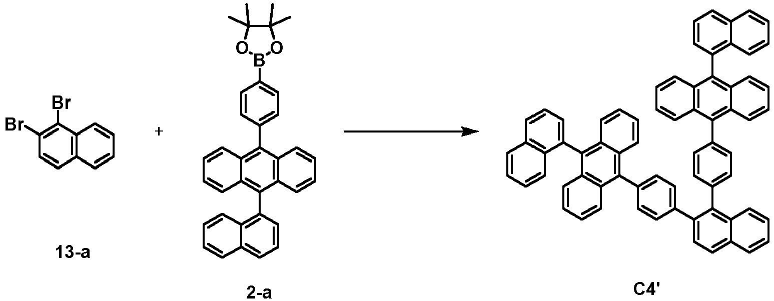

Compound C4' was prepared in the same manner as in the preparation method of Compound C3', except that Compound 2-a was used instead of Compound 13-b.
m/z [M+H]⁺ = 885.3

### Step 14-2) Preparation of Compound C4

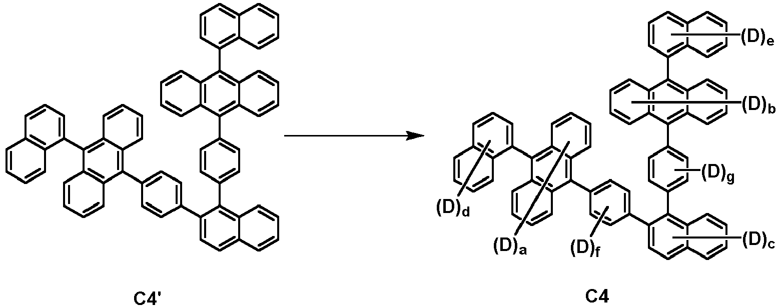

Compound C4 (a+b+c+d+e+f+g=40-44, yield: 92%) was prepared in the same manner as in the preparation method of Compound A1, except that Compound C4' was used instead of Compound A1'.
m/z [M+H]⁺ = 929.3

### Preparation Example 15: Preparation of Compound D1

### Step 15-1) Preparation of Compound D1'

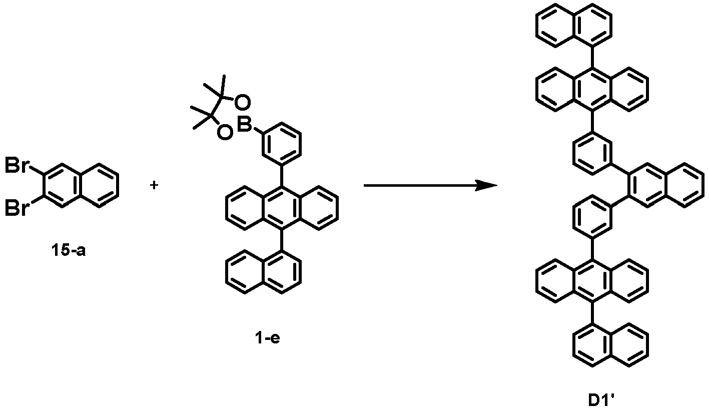

Compound 15-a (10.0 g, 1.0 eq.) and Compound 1-e (2.4 eq.) were placed in a round bottom flask and dissolved in anhydrous PhMe. C₂CO₃ (10 eq.) dissolved in water was injected. Pd(PPh₃)₄ (20 mol%) was added dropwise thereto under a bath temperature of 90°C, and the mixture was stirred for 2 days. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound D1' (yield: 83%).
m/z [M+H]⁺ = 885.3

### Step 15-2) Preparation of Compound D1

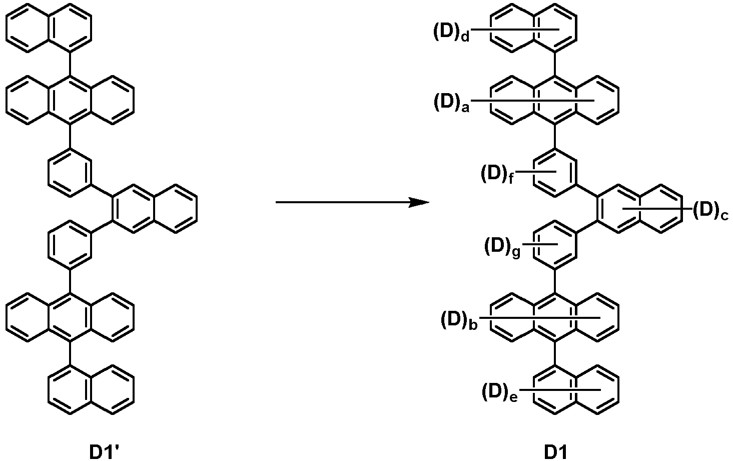

Compound D1 (a+b+c+d+e+f+g=40-44, yield: 93%) was prepared in the same manner as in the preparation method of Compound A1, except that Compound D1' was used instead of Compound A1'.
m/z [M+H]⁺ = 929.3

### Preparation Example 16: Preparation of Compound D2

### Step 16-1) Preparation of Compound 16-a

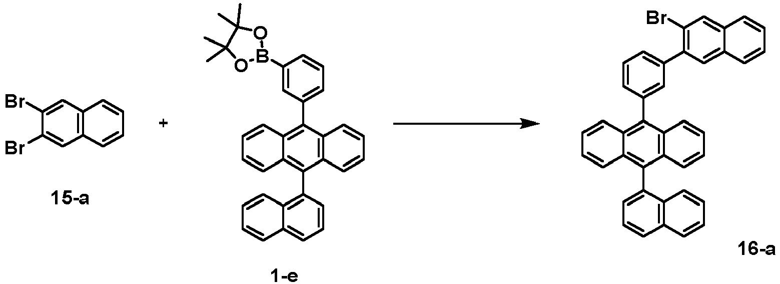

Compound 15-a (10.0 g, 1.0 eq.) and Compound 1-e (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 16-a (yield: 91%).
m/z [M+H]⁺ = 585.1

### Step 16-2) Preparation of Compound D2'

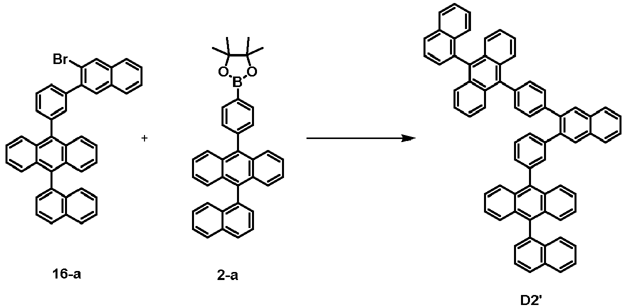

Compound 16-a (10.0 g, 1.0 eq.) and Compound 2-a (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound D2' (yield: 87%).
m/z [M+H]⁺ = 885.3

### Step 16-3) Preparation of Compound D2

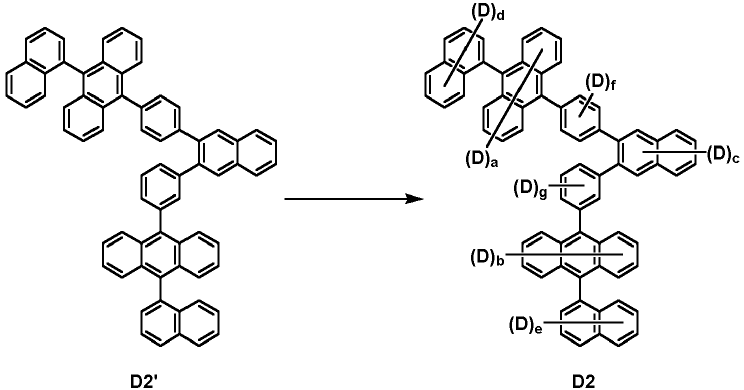

Compound D2 (a+b+c+d+e+f+g=40-44, yield: 95%) was prepared in the same manner as in the preparation method of Compound A1, except that Compound D2' was used instead of Compound A1'.
m/z [M+H]⁺ = 929.3

### Preparation Example 17: Preparation of Compound D3

### Step17-1) Preparation of Compound D3'

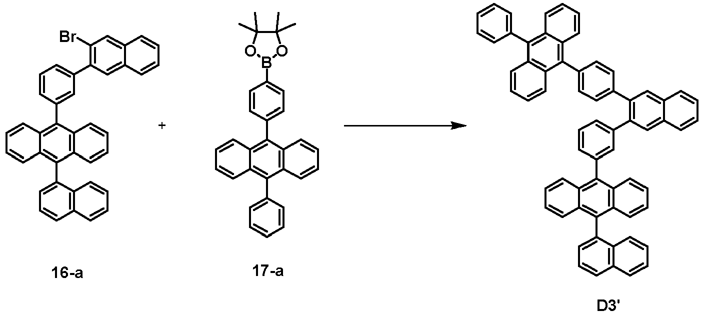

Compound 16-a (10.0 g, 1.0 eq.) and Compound 17-a (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred for overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound D3' (yield: 91%).
m/z [M+H]⁺ = 835.3

### Step 17-2) Preparation of Compound D3

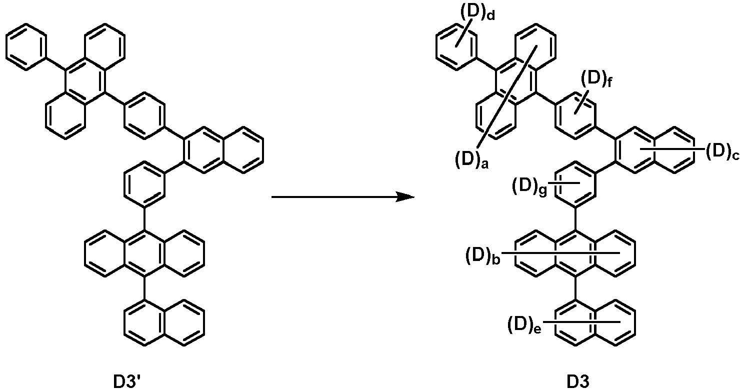

Compound D3 (a+b+c+d+e+f+g=38-42, yield: 93%) was prepared in the same manner as in the preparation method of Compound A1, except that Compound D3' was used instead of Compound A1'.
m/z [M+H]⁺ = 877.3

### Preparation Example 18: Preparation of Compound D4

### Step 18-1) Preparation of Compound D4'

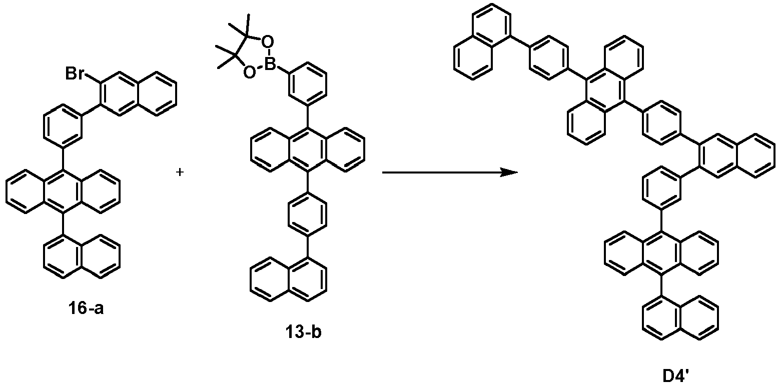

Compound 16-a (10.0 g, 1.0 eq.) and Compound 13-b (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred for overnight. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound D4' (yield: 85%).
m/z [M+H]⁺ = 961.4

### Step 18-2) Preparation of Compound D4

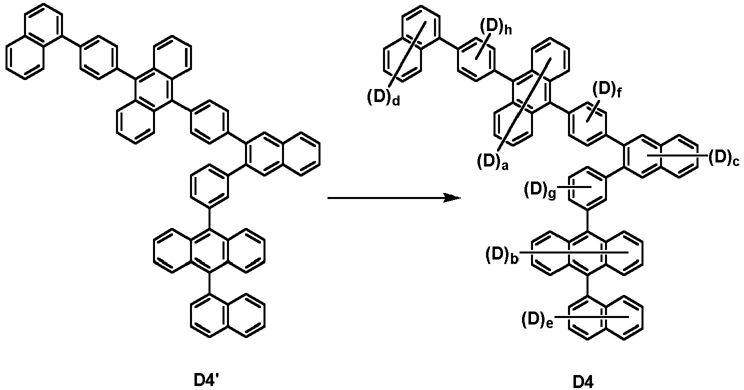

Compound D4 (a+b+c+d+e+f+g+h=44-48, yield: 94%) was prepared in the same manner as in the preparation method of Compound A1, except that Compound D4' was used instead of Compound A1'.
m/z [M+H]⁺ = 1009.4

### Preparation Example 19: Preparation of Compound E1

### Step 19-1) Preparation of Compound E1'

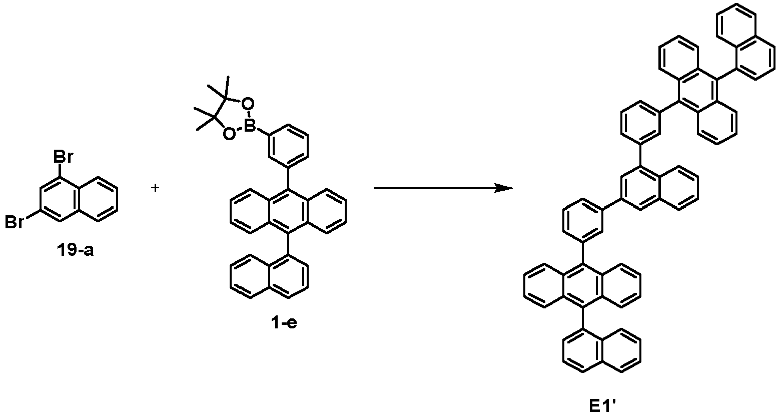

Compound 19-a (10.0 g, 1.0 eq.) and Compound 1-e (2.4 eq.) were placed in a round bottom flask and dissolved in anhydrous PhMe. C₂CO₃ (10 eq.) dissolved in water was injected. Pd(PPh₃)₄ (20 mol%) was added dropwise thereto under a bath temperature of 90°C, and the mixture was stirred for 2 days. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound E1' (yield: 88%).
m/z [M+H]⁺ = 885.3

### Step 19-2) Preparation of Compound E1

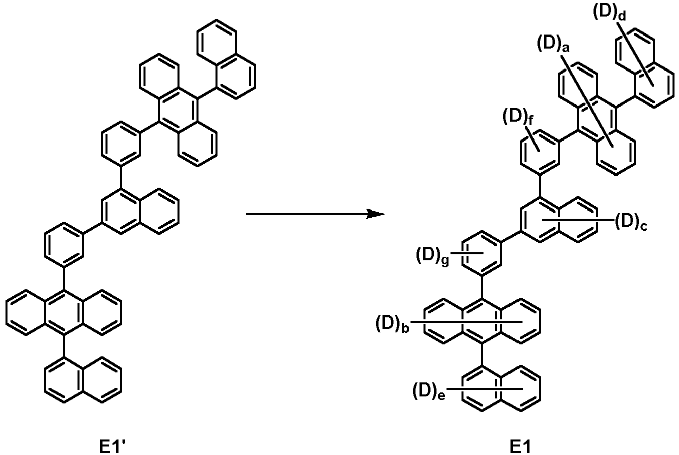

Compound E1 (a+b+c+d+e+f+g=40-44, yield: 93%) was prepared in the same manner as in the preparation method of Compound A1, except that Compound E1' was used instead of Compound A1'.
m/z [M+H]⁺ = 929.3

### Preparation Example 20: Preparation of Compound E2

### Step 20-1) Preparation of Compound E2'

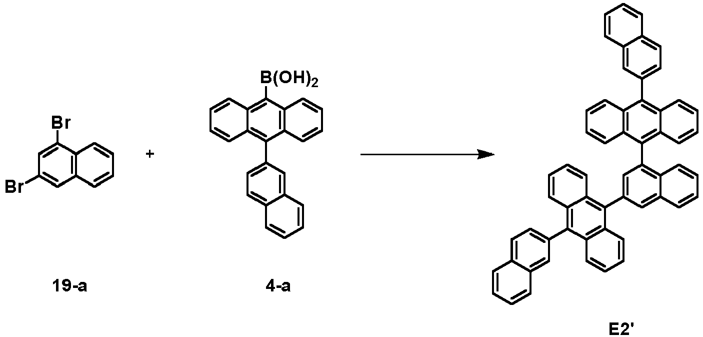

Compound E2' (yield: 83%) was prepared in the same manner as in the preparation method of Compound E1', except that Compound 4-a was used instead of Compound 1-e.
m/z [M+H]⁺ = 733.3

### Step 20-2) Preparation of Compound E2

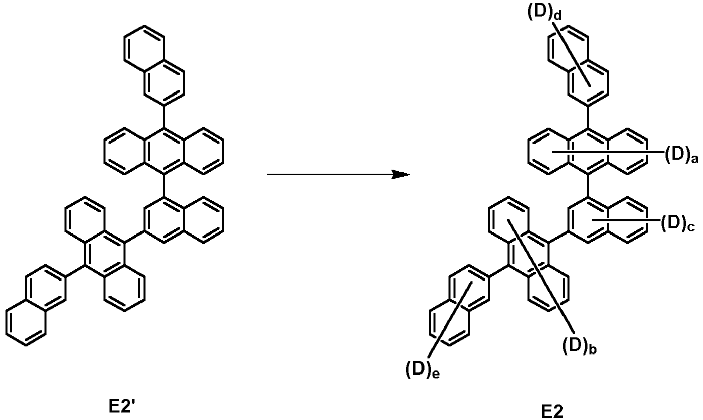

Compound E2 (a+b+c+d+e=34-36, yield: 93%) was prepared in the same manner as in the preparation method of Compound A1, except that Compound E2' was used instead of Compound A1'.
m/z [M+H]⁺ = 769.3

### Preparation Example 21: Preparation of Compound F1

### Step 21-1) Preparation of Compound F1'

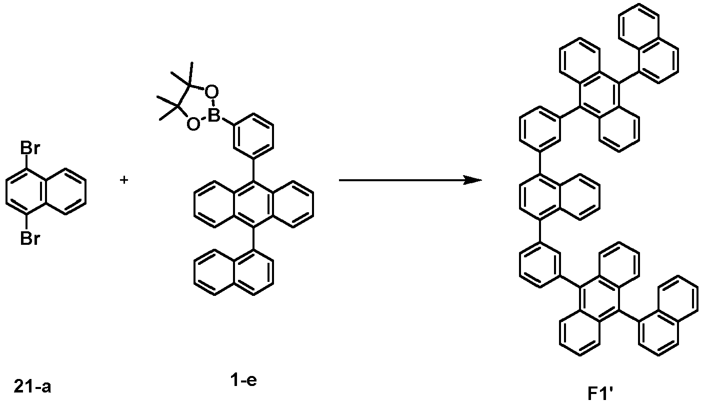

Compound F1' (yield: 90%) was prepared in the same manner as in the preparation method of Compound E1', except that Compound 21-a was used instead of Compound 19-a.
m/z [M+H]⁺ = 885.3

### Step 21-2) Preparation of Compound F1

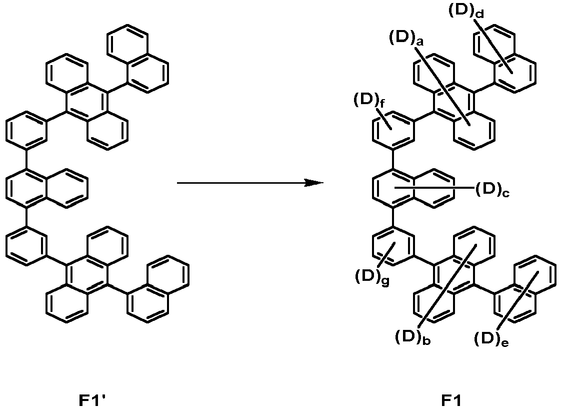

Compound F1 (a+b+c+d+e+f+g=40-44, yield: 93%) was prepared in the same manner as in the preparation method of Compound A1, except that Compound F1' was used instead of Compound A1'.
m/z [M+H]⁺ = 929.3

### Preparation Example 22: Preparation of Compound F2

### Step 22-1) Preparation of Compound F2'

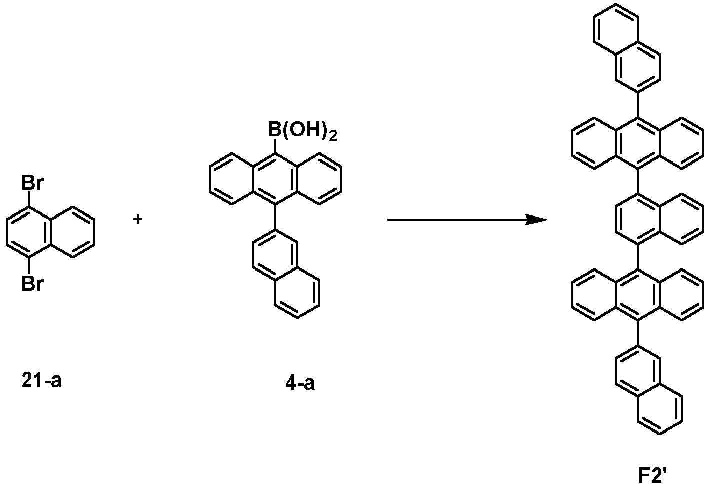

Compound F2' (yield: 87%) was prepared in the same manner as in the preparation method of Compound F1', except that Compound 4-a was used instead of Compound 1-e.
m/z [M+H]⁺ = 733.3

### Step 22-2) Preparation of Compound F2

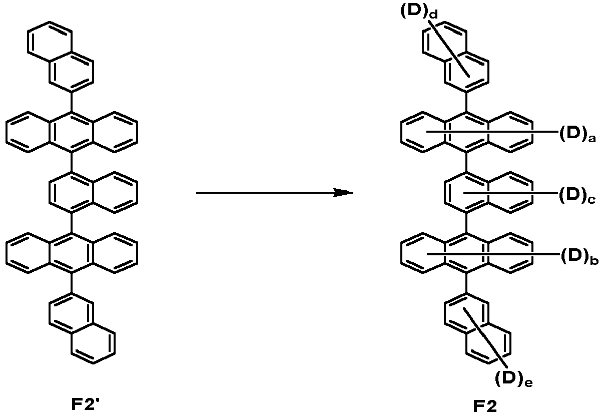

Compound F2 (a+b+c+d+e=34-36, yield: 93%) was prepared in the same manner as in the preparation method of Compound A1, except that Compound F2' was used instead of Compound A1'.
m/z [M+H]⁺ = 769.3

### Preparation Example 23: Preparation of Compound G1

### Step 23-1) Preparation of Compound G1'

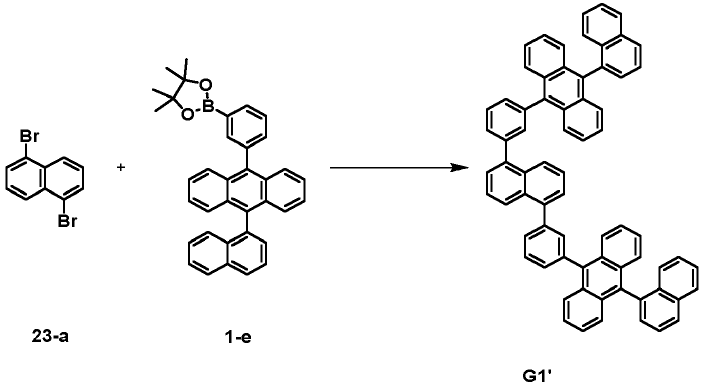

Compound G1' (yield: 87%) was prepared in the same manner as in the preparation method of Compound E1', except that Compound 23-a was used instead of Compound 19-a.
m/z [M+H]⁺ = 885.3

### Step 23-2) Preparation of Compound G1

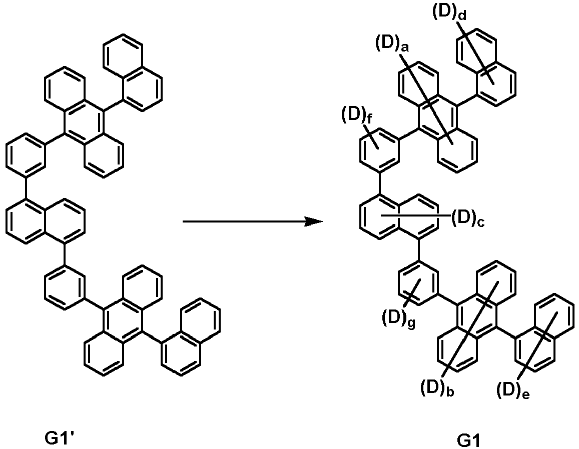

Compound G1 (a+b+c+d+e+f+g=40-44, yield: 95%) was prepared in the same manner as in the preparation method of Compound A1, except that Compound G1' was used instead of Compound A1'.
m/z [M+H]⁺ = 929.3

### Preparation Example 24: Preparation of Compound G2

### Step 24-1) Preparation of Compound G2'

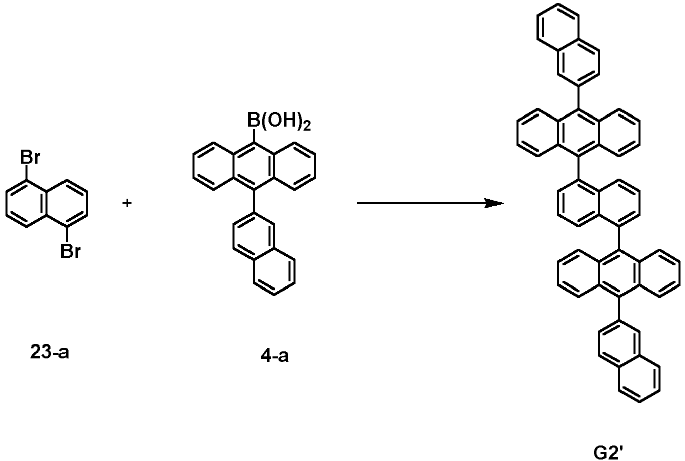

Compound G2' (yield: 91%) was prepared in the same manner as in the preparation method of Compound G1', except that Compound 4-a was used instead of Compound 1-e.
m/z [M+H]⁺ = 733.3

### Step 24-2) Preparation of Compound G2

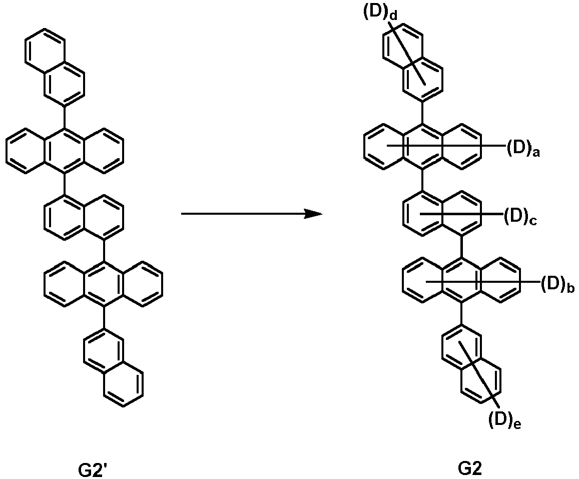

Compound G2 (a+b+c+d+e=34-36, yield: 93%) was prepared in the same manner as in the preparation method of Compound A1, except that Compound G2' was used instead of Compound A1'.
m/z [M+H]⁺ = 769.3

### Preparation Example 25: Preparation of Compound H1

### Step 25-1) Preparation of Compound H1'

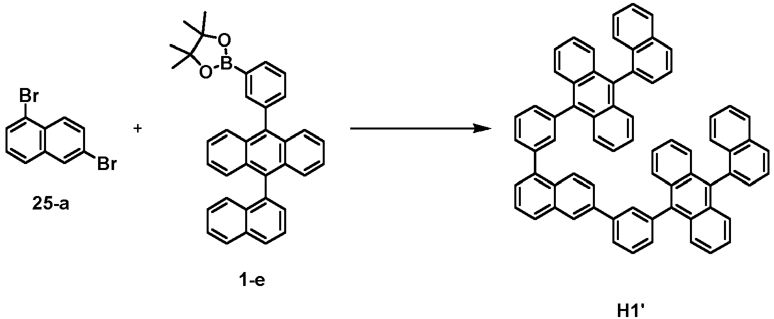

Compound H1' (yield: 86%) was prepared in the same manner as in the preparation method of Compound E1', except that Compound 25-a was used instead of Compound 19-a.
m/z [M+H]⁺ = 885.3

### Step 25-2) Preparation of Compound H1

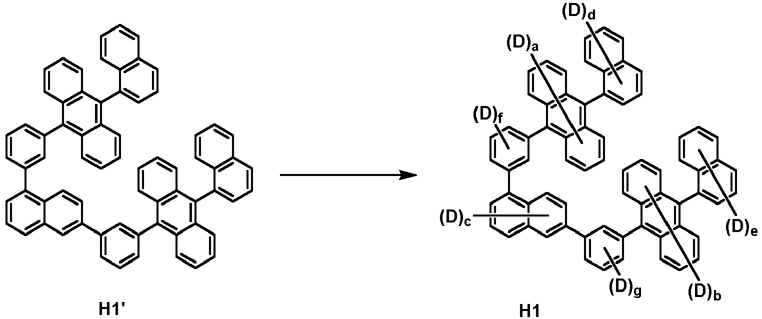

Compound H1 (a+b+c+d+e+f+g=40-44, yield: 90%) was prepared in the same manner as in the preparation method of Compound A1, except that Compound H1' was used instead of Compound A1'.
m/z [M+H]⁺ = 929.3

### Preparation Example 26: Preparation of Compound H2

### Step 26-1) Preparation of Compound H2'

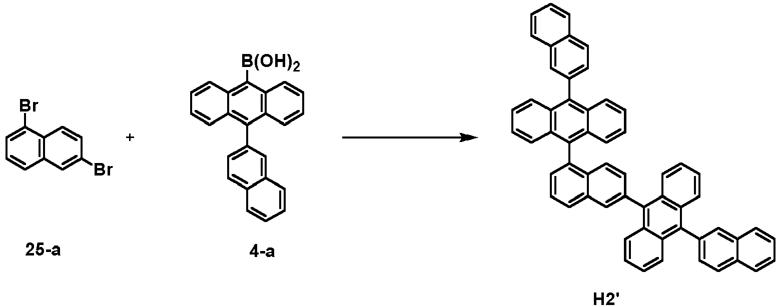

Compound H2' (yield: 86%) was prepared in the same manner as in the preparation method of Compound H1', except that Compound 4-a was used instead of Compound 1-e.
m/z [M+H]⁺ = 733.3

### Step 26-2) Preparation of Compound H2

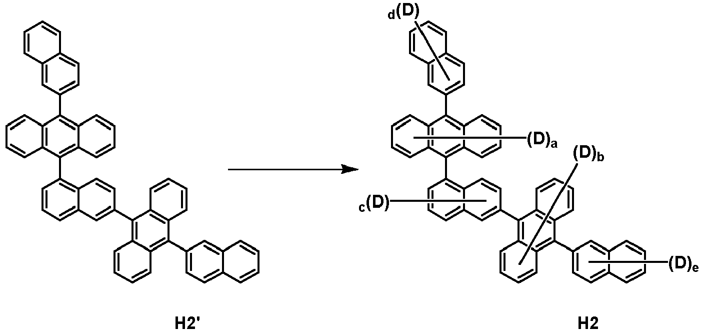

Compound H2 (a+b+c+d+e=34-36, yield: 93%) was prepared in the same manner as in the preparation method of Compound A1, except that Compound H2' was used instead of Compound A1'.
m/z [M+H]⁺ = 769.3

### Preparation Example 27: Preparation of Compound I1

### Step 27-1) Preparation of Compound 11'

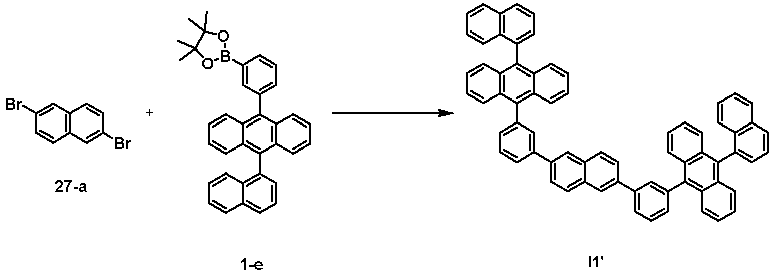

Compound 11' (yield: 89%) was prepared in the same manner as in the preparation method of Compound E1', except that Compound 27-a was used instead of Compound 19-a.
m/z [M+H]⁺ = 885.3

### Step 27-2) Preparation of Compound 11

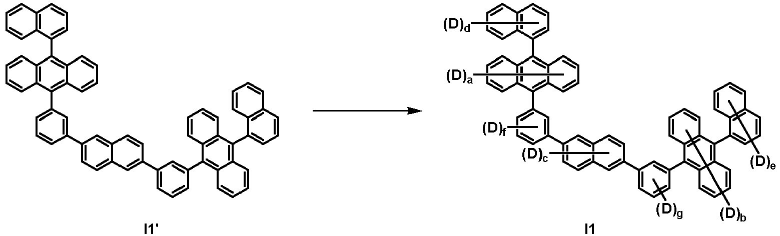

Compound 11 (a+b+c+d+e+f+g=40-44, yield: 92%) was prepared in the same manner as in the preparation method of Compound A1, except that Compound 11' was used instead of Compound A1'.
m/z [M+H]⁺ = 929.3

### Preparation Example 28: Preparation of Compound I2

### Step 28-1) Preparation of Compound I2'

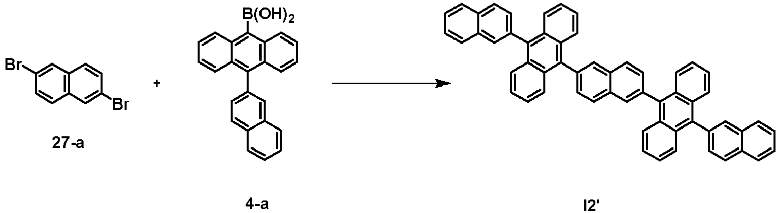

Compound I2' (yield: 91%) was prepared in the same manner as in the preparation method of Compound 11', except that Compound 4-a was used instead of Compound 1-e.
m/z [M+H]⁺ = 733.3

### Step28-2) Preparation of Compound I2

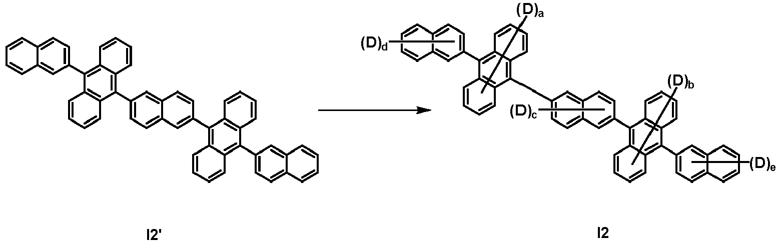

Compound 12 (a+b+c+d+e=34-36, yield:91%) was prepared in the same manner as in the preparation method of Compound A1, except that Compound I2' was used instead of Compound A1'.
m/z [M+H]⁺ = 769.3

### Preparation Example 29: Preparation of Compound J1

### Step 29-1) Preparation of Compound J1'

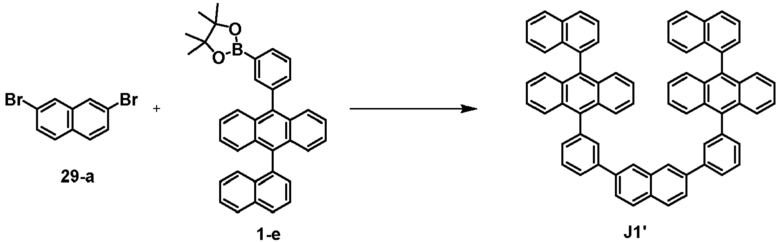

Compound J1' (yield: 91%) was prepared in the same manner as in the preparation method of Compound E1', except that Compound 29-a was used instead of Compound 19-a.
m/z [M+H]⁺ = 885.3

### Step 29-2) Preparation of Compound J1

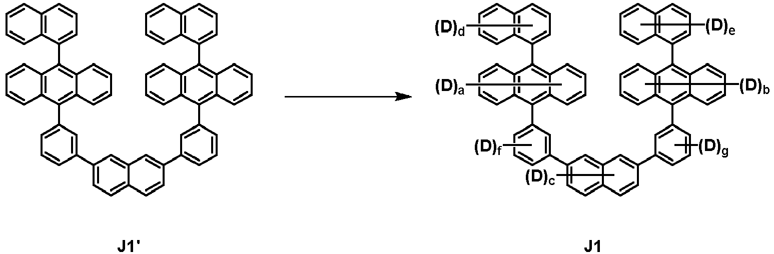

Compound J1 (a+b+c+d+e+f+g=40-44, yield: 96%) was prepared in the same manner as in the preparation method of Compound A1, except that Compound J1' was used instead of Compound A1'.
m/z [M+H]⁺ = 929.3

### Preparation Example 30: Preparation of Compound J2

### Step 30-1) Preparation of Compound J2'

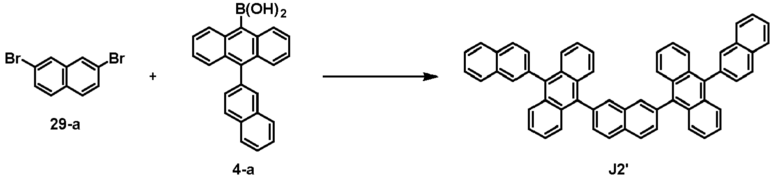

Compound J2' (yield: 91%) was prepared in the same manner as in the preparation method of Compound J1', except that Compound 4-a was used instead of Compound 1-e.
m/z [M+H]⁺ = 733.3

### Step 30-2) Preparation of Compound J2

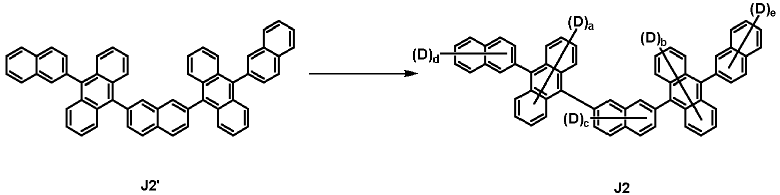

Compound J2 (a+b+c+d+e=34-36, yield: 93%) was prepared in the same manner as in the preparation method of Compound A1, except that Compound J2' was used instead of Compound A1'.
m/z [M+H]⁺ = 769.3

### Preparation Example 31: Preparation of Compound A9

### Step31-1) Preparation of Compound 31-b

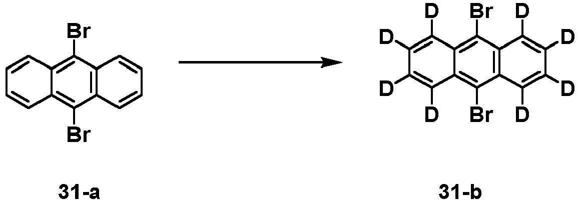

Compound 31-a (20.0 g, 1.0 eq.) was placed in a round bottom flask under a nitrogen atmosphere and dissolved in benzene-De (150 eq.). TfOH (2.0 eq.) was slowly added dropwise to the reaction solution, and the mixture was stirred at 25°C for 2 hours. D₂O was dropwise added to the reaction solution to terminate the reaction, and potassium phosphate tribasic (30 wt% in aqueous solution, 2.4 eq.) was added dropwise thereto and the pH of the aqueous layer was adjusted to 9-10. The reaction mixture was washed with CH₂Cl₂/DI water and the organic layer was separated. Water was removed with MgSO₄ and passed through a Celite-Florisil-Silica pad. The passed solution was concentrated under reduced pressure and purified by column chromatography to prepare Compound 31-b (yield: 91%).
m/z [M+H]⁺ = 342.9

### Step 31-2) Preparation of Compound 31-d

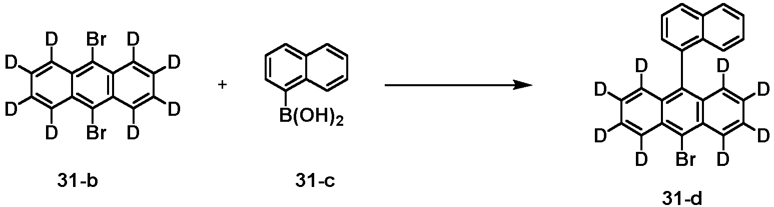

Compound 31-b (20.0 g, 1.0 eq.) and Compound 31-c (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred for 4 hours. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 31-d (yield: 91%).
m/z [M+H]⁺ = 391.1

### Step 31-3) Preparation of Compound 31-f

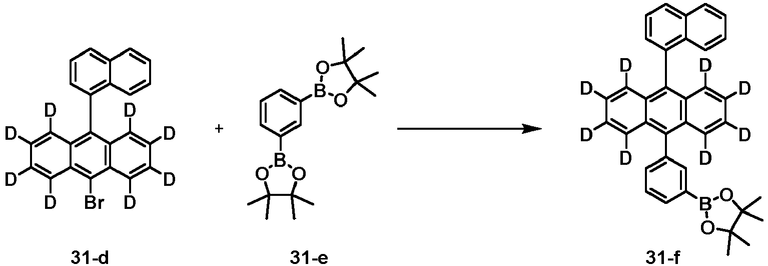

Compound 31-d (15.0 g, 1.0 eq.) and Compound 31-e (1.03 eq.) were placed in a round bottom flask and dissolved in anhydrous PhMe. C₂CO₃ (5 eq.) dissolved in water was injected. Pd(PPh₃)₄ (10 mol%) was added dropwise thereto under a bath temperature of 90°C, and the mixture was stirred for 4 hours. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound 31-f (yield: 85%).
m/z [M+H]⁺ = 515.3

### Step 31-4) Preparation of Compound A9

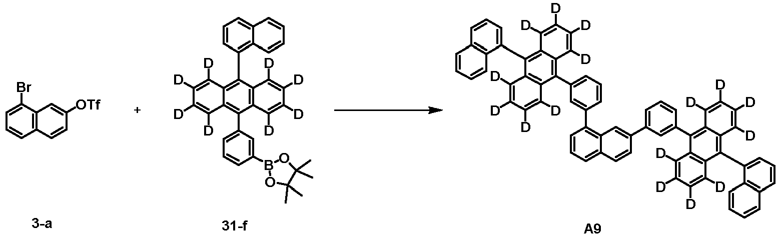

Compound 3-a (2.0 g, 1.0 eq.) and Compound 31-f (2.4 eq.) were placed in a round bottom flask and dissolved in anhydrous PhMe. C₂CO₃ (10 eq.) dissolved in water was injected. Pd(PPh₃)₄ (20 mol%) was added dropwise thereto under a bath temperature of 90°C, and the mixture was stirred for 2 days. After the reaction, the reaction mixture was cooled at room temperature, sufficiently diluted with EtOAc, and then washed with EtOAc/brine. The organic layer was separated, water was removed with MgSO₄ and passed through a Celite-Florisil-Silica pad. The passed solution was concentrated under reduced pressure, and then purified by column chromatography to prepare Compound A9 (yield: 84%).
m/z [M+H]⁺ = 901.4

### Preparation Example 32: Preparation of Compound A10

### Step 32-1) Preparation of Compound A10

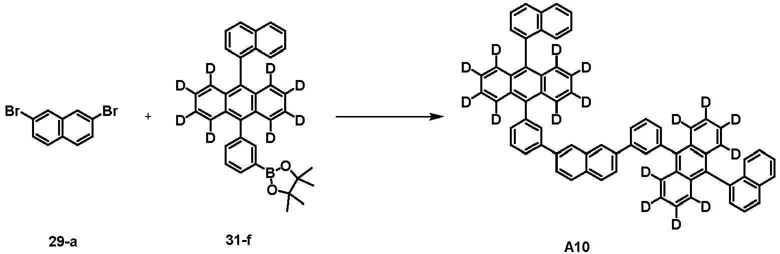

Compound A10 (yield: 91%) was prepared in the same manner as in the preparation method of Compound A9, except that Compound 29-a was used instead of Compound 3-a.
m/z [M+H]⁺ = 901.4

### Preparation Example 33: Preparation of Compound G3

### Step 33-1) Preparation of Compound G3

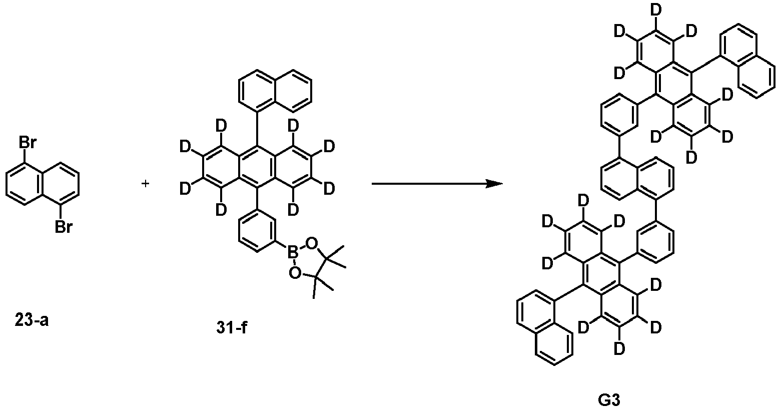

Compound G3 (yield: 87%) was prepared in the same manner as in the preparation method of Compound A9, except that Compound 23-a was used instead of Compound 3-a.

### Experimental Example 1: Confirmation of deuterium substitution rate

With respect to the compounds prepared in Preparation Examples 1 to 30, the number of substituted deuteriums in the compound was obtained through MALDI-TOF MS (Matrix-Assisted Laser Desorption/Ionization Time-of-Flight Mass Spectrometer) analysis, and then the deuterium substitution rate was calculated as a percentage of the number of substituted deuteriums to the total number of hydrogens that can be present in the Chemical Formula. The results are shown in Table 1 below.

**[Table 1]**

| Category | Compou nd | Deuterium substituti on rate (%) | Category | Compou nd | Deuterium substituti on rate (%) |
|---|---|---|---|---|---|
| Preparation Example 1 | Compound A1 | 90-100 | Preparation Example 16 | Compound D2 | 90.9-100 |
| Preparation Example 2 | Compound A2 | 90-100 | Preparation Example 17 | Compound D3 | 90.5-100 |
| Preparation Example 3 | Compound A3 | 88.9-100 | Preparation Example 18 | Compound D4 | 91.7-100 |
| Preparation Example 4 | Compound A4 | 94.4-100 | Preparation Example 19 | Compound E1 | 90.9-100 |
| Preparation Example 5 | Compound A5 | 90.9-100 | Preparation Example 20 | Compound E2 | 94.4-100 |
| Preparation Example 6 | Compound A6 | 90.9-100 | Preparation Example 21 | Compound F1 | 90.9-100 |
| Preparation Example 7 | Compound A7 | 90-100 | Preparation Example 22 | Compound F2 | 94.4-100 |
| Preparation Example 8 | Compound A8 | 90-100 | Preparation Example 23 | Compound G1 | 90.9-100 |
| Preparation Example 9 | Compound B1 | 90.9-100 | Preparation Example 24 | Compound G2 | 94.4-100 |
| Preparation Example 10 | Compound B2 | 90.9-100 | Preparation Example 25 | Compound H1 | 90.9-100 |
| Preparation Example 11 | Compound C1 | 90-100 | Preparation Example 26 | Compound H2 | 94.4-100 |
| Preparation Example 12 | Compound C2 | 90-100 | Preparation Example 27 | Compound I1 | 90.9-100 |
| Preparation Example 13 | Compound C3 | 92.3-100 | Preparation Example 28 | Compound I2 | 94.4-100 |
| Preparation Example 14 | Compound C4 | 90.9-100 | Preparation Example 29 | Compound J1 | 90.9-100 |
| Preparati Example 15 | Compound D1 | 90.9-100 | Preparation Example 30 | Compound J2 | 94.4-100 |

### Example 1

A glass substrate on which ITO (indium tin oxide) was coated as a thin film to a thickness of 500 Å was put into distilled water in which a detergent was dissolved, and ultrasonically cleaned. At this time, a product manufactured by Fischer Co. was used as the detergent, and as the distilled water, distilled water filtered twice using a filter manufactured by Millipore Co. was used. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then the substrate was cleaned for 5 minutes and then transferred to a glove box.

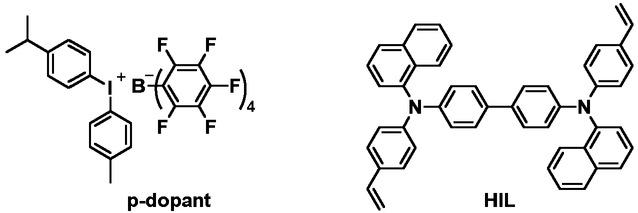

A coating composition, in which the compound p-dopant and the compound HIL (weight ratio of 2:8) were dissolved in cyclohexanone at 20 wt/v%, was spin-coated (4000 rpm) onto the ITO transparent electrode, and heat treated (cured) at 200°C for 30 minutes to form a hole injection layer with a thickness of 400Å.

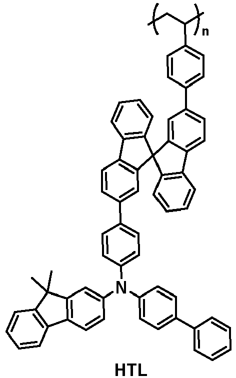

A coating composition, in which the compound HTL (Mn: 27,900; Mw: 35,600; measured by GPC (Agilent 1200 series) using PC Standard) was dissolved in toluene at 6 wt/v%, was spin-coated (4000 rpm) onto the hole injection layer, and heat-treated at 200°C for 30 minutes to form a hole transport layer with a thickness of 200Å.

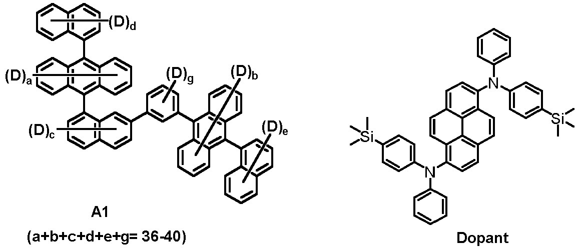

A coating composition, in which the light emitting layer host compound A1 prepared above and the light emitting layer dopant compound Dopant (weight ratio of 98:2) were dissolved in cyclohexanone at 2 wt/v%, was spin-coated (4000 rpm) on the hole transport layer and heat-treated at 180°C for 30 minutes to form a light emitting layer with a thickness of 400Å.

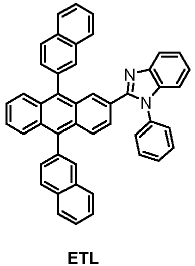

After transferring to a vacuum evaporator, the compound ETL was vacuum-deposited to a thickness of 350 Å on the light emitting layer to form an electron injection and transport layer. LiF (10 Å), and aluminum (1000 Å) were sequentially deposited on the electron injection and transport layer to form a cathode.

In the above-mentioned process, the vapor deposition rate of the organic material was maintained at 0.4 to 0.7 Å/sec, the deposition rate of LiF was maintained at 0.3 Å/sec, the deposition rate of aluminum was maintained at 2 Å/sec, and the degree of vacuum during the deposition was maintained at 2.66*10⁻⁵ to 6.66*10⁻⁶ Pa (2*10⁻⁷ to 5*10⁻⁸ torr).

### Examples 2 to Example 33

The organic light emitting devices were manufactured in the same manner as in Example 1, except that as a host of the light emitting layer, the compounds shown in Table 1 below were used instead of Compound A1. At this time, the compounds used in the Examples are summarized as follows.

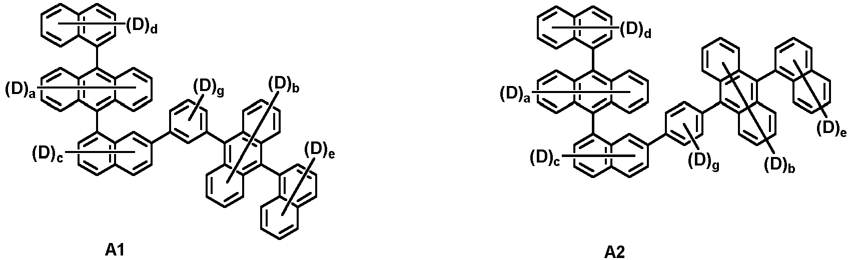

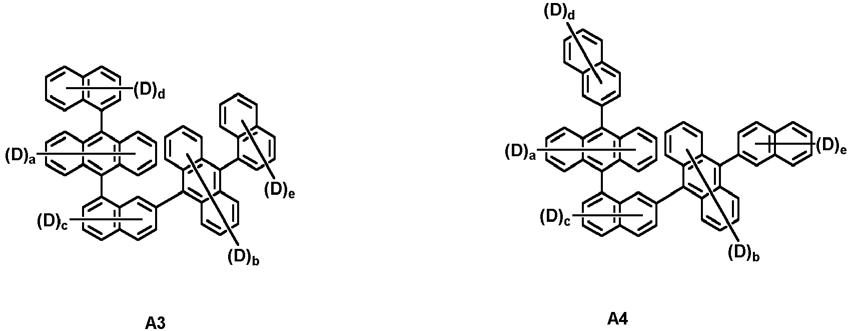

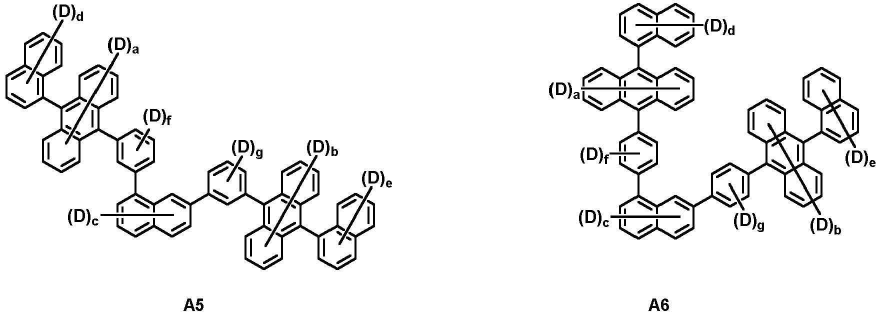

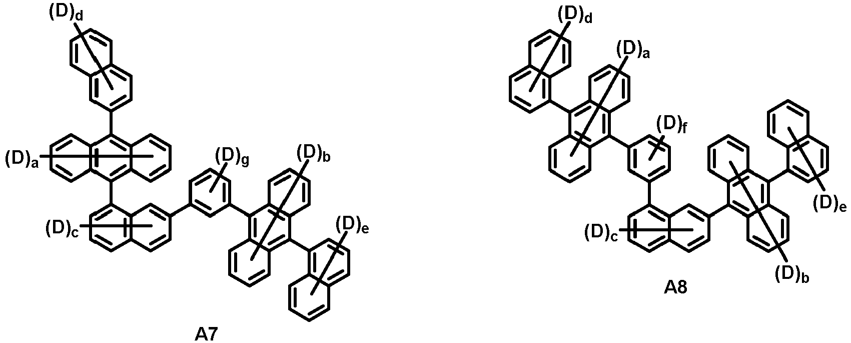

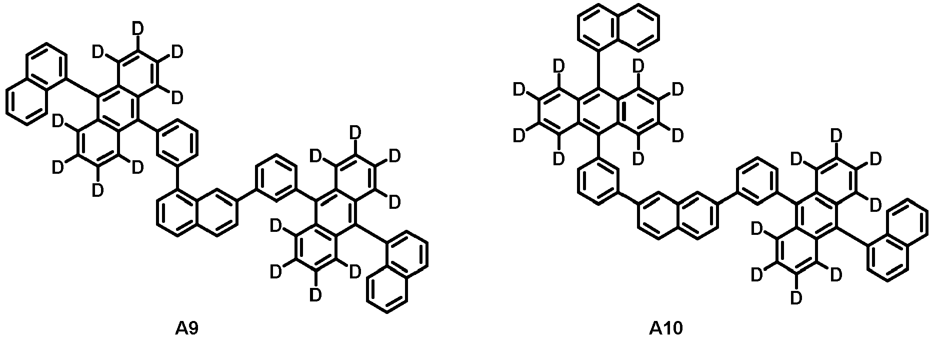

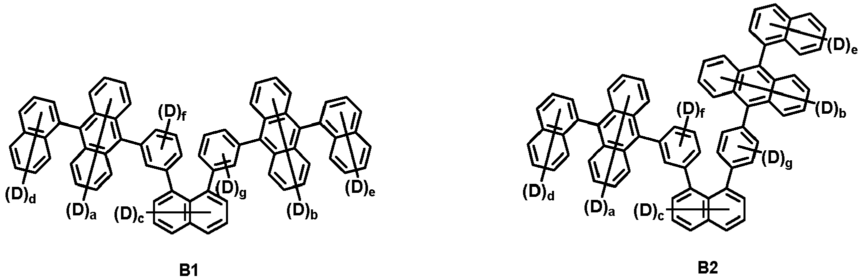

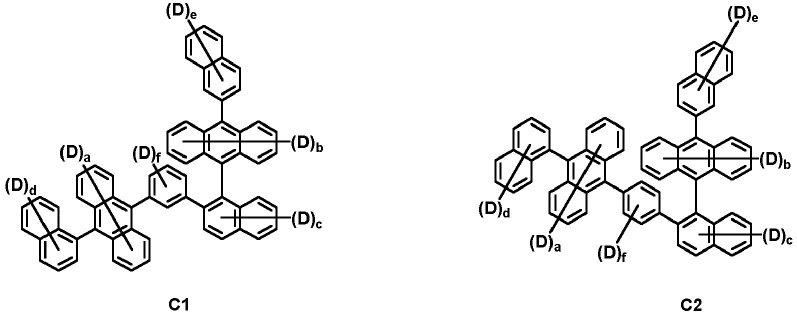

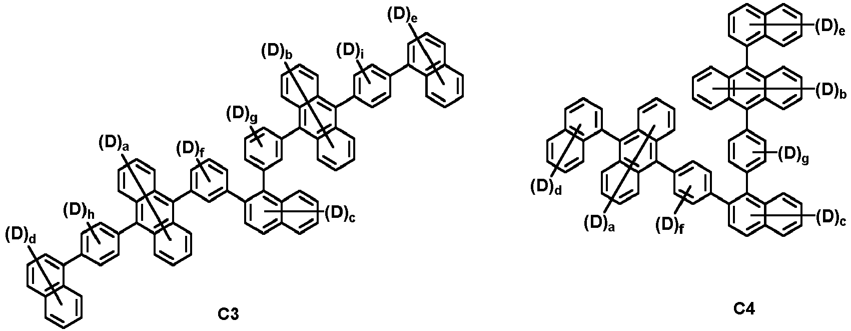

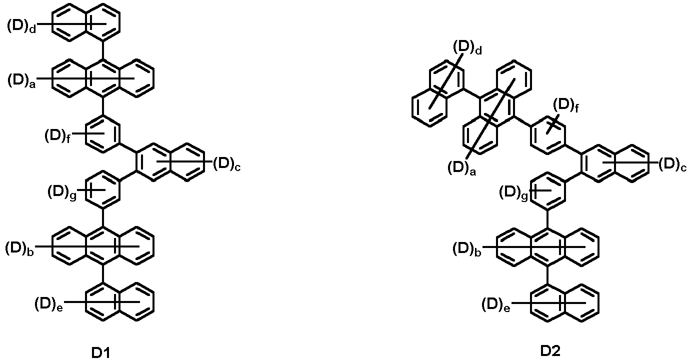

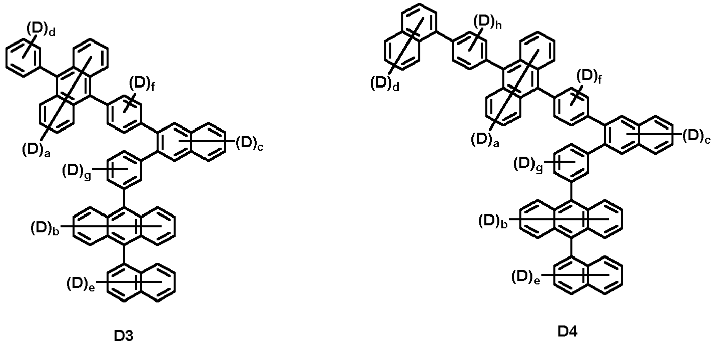

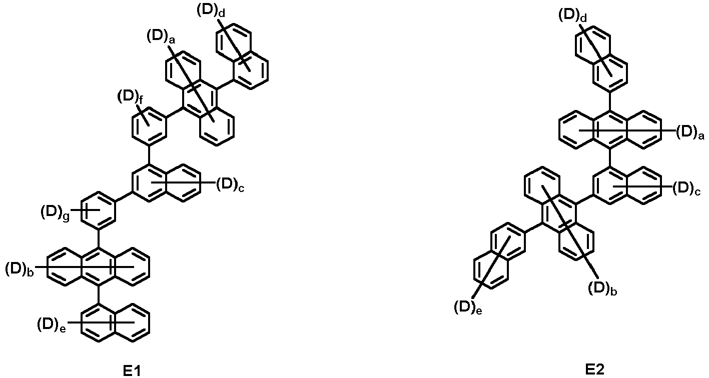

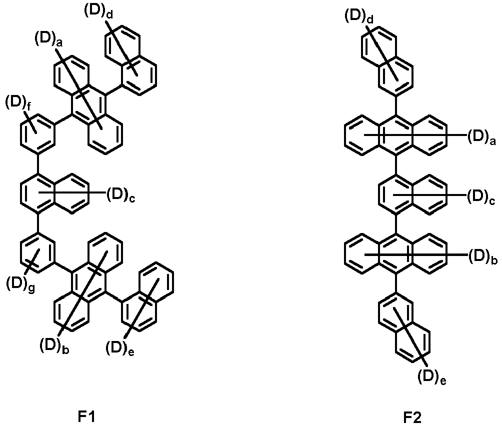

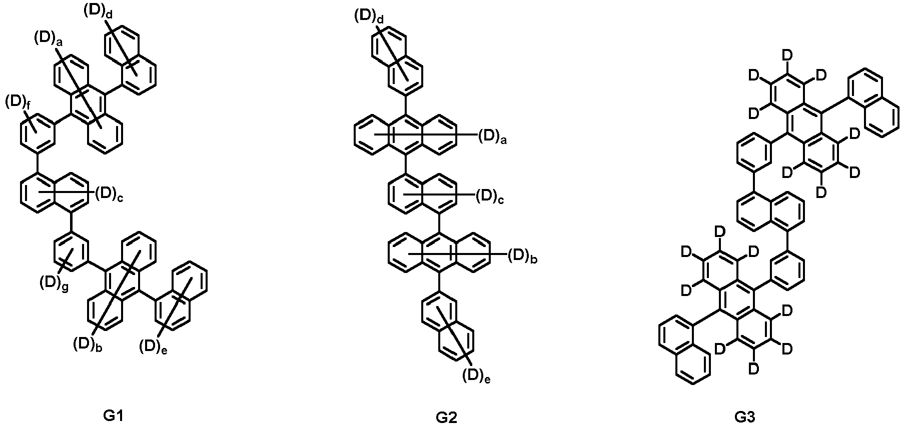

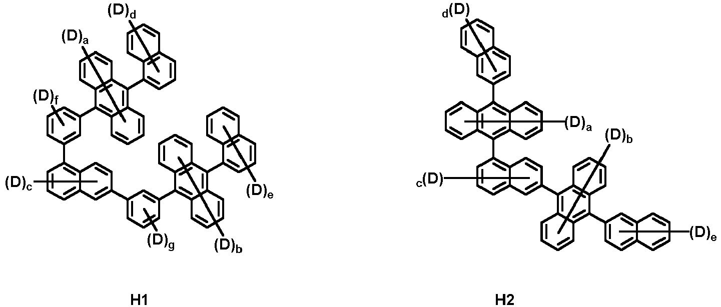

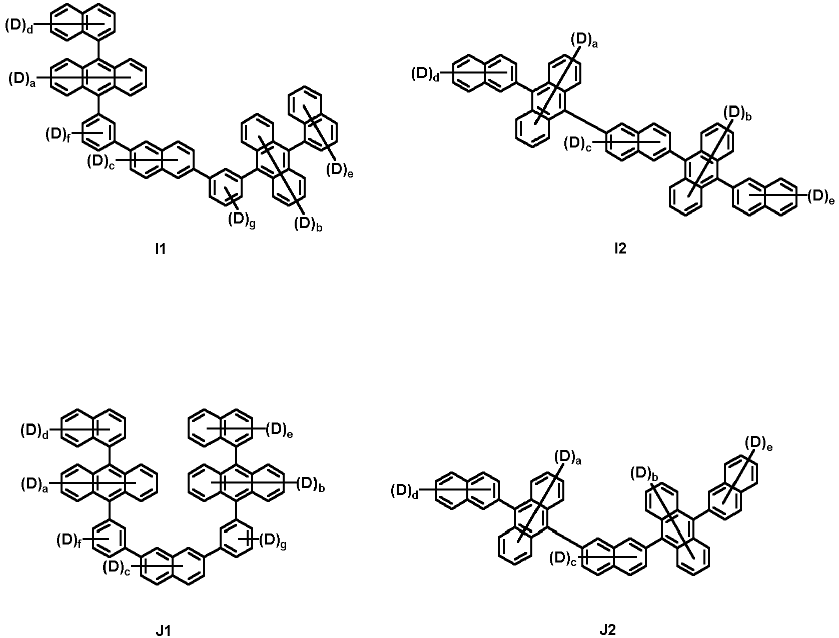

In the above compounds, a, b, c, d, e, f, g, h and i, indicating the number of substitutions of deuterium, are the same as described above.

### Comparative Examples 1 to Comparative Example 8

The organic light emitting devices were manufactured in the same manner as in Example 1, except that as a host of the light emitting layer, the compounds shown in Table 1 below were used instead of Compound A1. At this time, the compounds used in Comparative Examples are as follows.

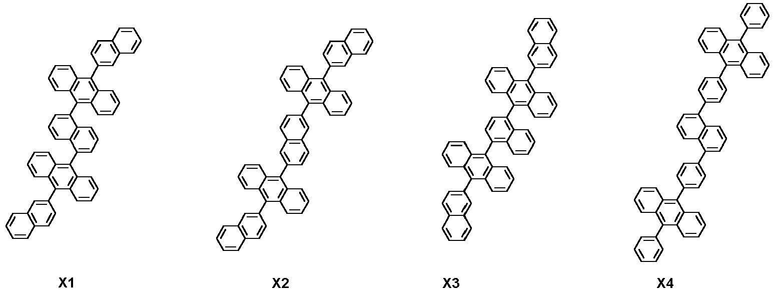

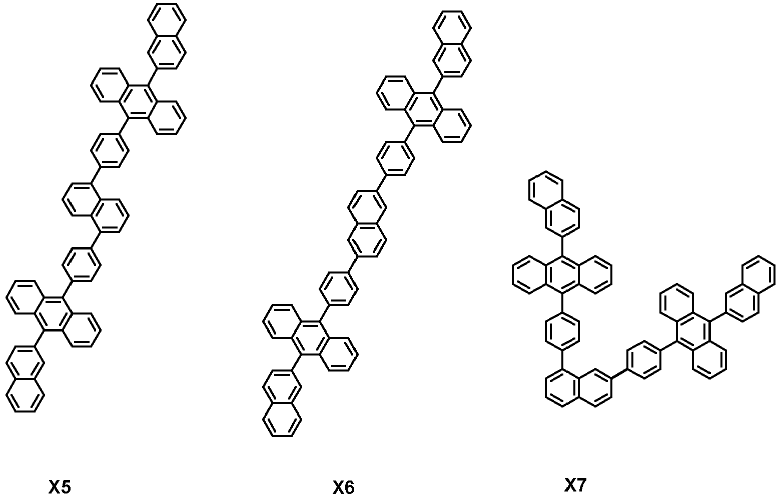

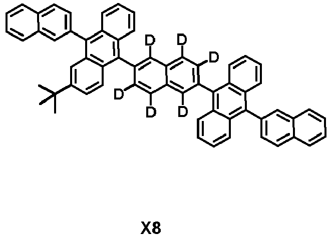

### Experimental Example 2: Characteristic Evaluation of Organic Light Emitting Device

For the organic light emitting devices manufactured in Examples and Comparative Examples, the driving voltage, external quantum efficiency (EQE) and lifetime were measured at a current density of 10 mA/cm², and the results are shown in Table 2 below. At this time, the external quantum efficiency (EQE) was determined by (number of photons emitted)/(number of charge carriers injected), and T90 means the time required for the luminance to be reduced to 90% of the initial luminance (500 nit)

**[Table 2]**

| Category | Host of light emitting layer | Driving voltage (V @10mA/cm²) | EQE (% @10mA/cm²) | Lifetime (hr) (T90 @500 nit) |
|---|---|---|---|---|
| Example 1 | Compound A1 | 4.58 | 9.71 | 388 |
| Example 2 | Compound A2 | 4.60 | 9.72 | 393 |
| Example 3 | Compound A3 | 4.61 | 9.71 | 372 |
| Example 4 | Compound A4 | 4.60 | 9.72 | 381 |
| Example 5 | Compound A5 | 4.62 | 9.79 | 383 |
| Example 6 | Compound A6 | 4.61 | 9.77 | 379 |
| Example 7 | Compound A7 | 4.62 | 9.73 | 381 |
| Example 8 | Compound A8 | 4.58 | 9.73 | 381 |
| Example 9 | Compound B1 | 4.63 | 9.74 | 379 |
| Example 10 | Compound B2 | 4.64 | 9.75 | 378 |
| Example 11 | Compound C1 | 4.64 | 9.69 | 375 |
| Example 12 | Compound C2 | 4.60 | 9.74 | 374 |
| Example 13 | Compound C3 | 4.61 | 9.69 | 379 |
| Example 14 | Compound C4 | 4.59 | 9.72 | 378 |
| Example 15 | Compound D1 | 4.62 | 9.73 | 373 |
| Example 16 | Compound D2 | 4.64 | 9.69 | 380 |
| Example 17 | Compound D3 | 4.61 | 9.73 | 376 |
| Example 18 | Compound D4 | 4.61 | 9.74 | 373 |
| Example 19 | Compound E1 | 4.60 | 9.78 | 379 |
| Example 20 | Compound E2 | 4.59 | 9.62 | 380 |
| Example 21 | Compound F1 | 4.60 | 9.60 | 379 |
| Example 22 | Compound F2 | 4.60 | 9.74 | 380 |
| Example 23 | Compound G1 | 4.62 | 9.75 | 381 |
| Example 24 | Compound G2 | 4.62 | 9.69 | 385 |
| Example 25 | Compound H1 | 4.61 | 9.73 | 382 |
| Example 26 | Compound H2 | 4.63 | 9.75 | 380 |
| Example 27 | Compound I1 | 4.62 | 9.70 | 379 |
| Example 28 | Compound I2 | 4.62 | 9.69 | 380 |
| Example 29 | Compound J1 | 4.61 | 9.71 | 378 |
| Example 30 | Compound J2 | 4.64 | 9.72 | 376 |
| Example 31 | Compound A9 | 4.60 | 9.70 | 383 |
| Example 32 | Compound A10 | 4.59 | 9.65 | 379 |
| Example 33 | Compound G3 | 4.57 | 9.64 | 373 |
| Comparative Example 1 | Compound X1 | 4.61 | 6.05 | 182 |
| Comparative Example 2 | Compound X2 | 4.64 | 6.03 | 186 |
| Comparative Example 3 | Compound X3 | 4.65 | 6.08 | 183 |
| Comparative Example 4 | Compound X4 | 4.62 | 6.02 | 186 |
| Comparative Example 5 | Compound X5 | 4.63 | 6.01 | 181 |
| Comparative Example 6 | Compound X6 | 4.63 | 5.96 | 183 |
| Comparative Example 7 | Compound X7 | 4.63 | 6.02 | 191 |
| Comparative Example 8 | Compound X8 | 4.59 | 3.59 | 87 |

As shown in Table 2 above, the organic light emitting device of Examples in which the compound represented by Chemical Formula 1 was used as a host material of the light emitting layer exhibited high efficiency and remarkably long lifetime as compared with the organic light emitting devices of Comparative Examples.

Specifically, the organic light emitting devices of Examples in which the compound represented by Chemical Formula 1 was used as a host of the light emitting layer has improved efficiency and lifetime as compared with the organic light emitting devices of Comparative Examples 1 to 7 in which Comparative Compounds X1 to X7 were used as hosts of the light emitting layer, respectively. This is considered to be because material stability is improved by substituting deuterium for two anthracene structures in the compound represented by Chemical Formula 1. Additionally, it can be confirmed that the organic light emitting device of Comparative Example 8 using Comparative Compound X8 as a host of the light emitting layer is remarkably reduced in efficiency and lifetime due to the alkyl group introduced into Comparative Compound X8 to improve solubility in organic solvents.

Therefore, it can be seen that when the compound represented by Chemical Formula 1 is employed as the host material of the organic light emitting device, it can be improved in the luminous efficiency and/or lifetime characteristics of the organic light emitting diode.

**[Description of symbols]**

| | | | |
|---|---|---|---|
| 1: | substrate | 2: | anode |
| 3: | light emitting layer | 4: | cathode |
| 5: | hole injection layer | 6: | hole transport layer |
| 7: | electron injection and transport layer | | |

## Claims

1. A compound represented by the following Chemical Formula 1: 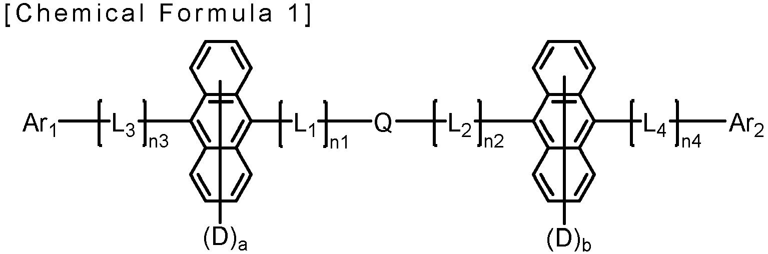 wherein, in Chemical Formula 1,
D represents deuterium,
Q is naphthylene unsubstituted or substituted with deuterium,
Ar₁ and Ar₂ are each independently a substituted or unsubstituted C₆₋₆₀ aryl,
L₁ to L₄ are each independently a substituted or unsubstituted C₆₋₆₀ arylene,
n1 to n4 are each independently an integer of 0 to 2, and
a and b are each independently an integer of 0 to 8,
provided that a+b is 1 or more.

2. The compound of claim 1, wherein:
Q is any one of the divalent substituents represented by the following Chemical Formulas 2a to 2j: 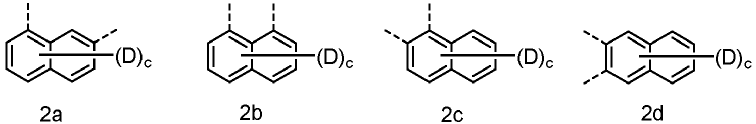 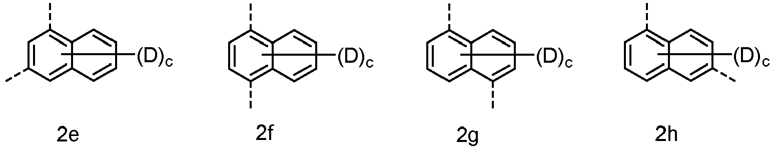 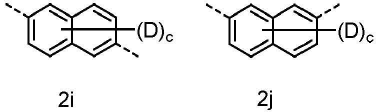 wherein, in Chemical Formulas 2a to 2j,
c is an integer of 0 to 6.

3. The compound of claim 1, wherein:
Ar₁ and Ar₂ are each independently phenyl unsubstituted or substituted with 1 to 5 deuteriums; or naphthyl unsubstituted or substituted with 1 to 7 deuteriums.

4. The compound of claim 1, wherein:
at least one of Ar₁ and Ar₂ is 1-naphthyl unsubstituted or substituted with 1 to 7 deuteriums; or
at least one of Ar₁ and Ar₂ is 2-naphthyl unsubstituted or substituted with 1 to 7 deuteriums.

5. The compound of claim 1, wherein:
L₁ and L₂ are each independently phenylene unsubstituted or substituted with 1 to 4 deuteriums; or naphthylene unsubstituted or substituted with 1 to 6 deuteriums.

6. The compound of claim 1, wherein:
L₃ and L₄ are each independently phenylene unsubstituted or substituted with 1 to 4 deuteriums.

7. The compound of claim 1, wherein:
n1+n2 is 0, 1, 2, or 3, and
n3+n4 is 0, 1, or 2.

8. The compound of claim 1, wherein:
a+b is 16.

9. The compound of claim 1, wherein:
a deuterium substitution rate of the compound is 80% to 100%.

10. The compound of claim 1, wherein:
the compound is represented by the following Chemical Formula 1-1: 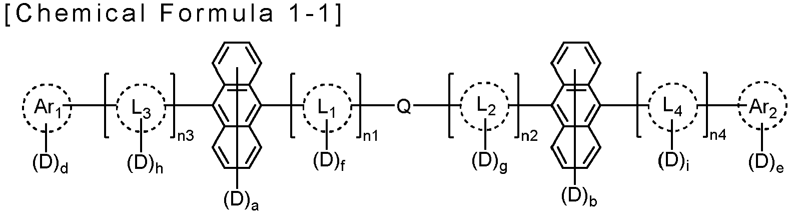 wherein, in Chemical Formula 1-1,
Q is any one of the divalent substituents represented by the following Chemical Formulas 2a to 2j, 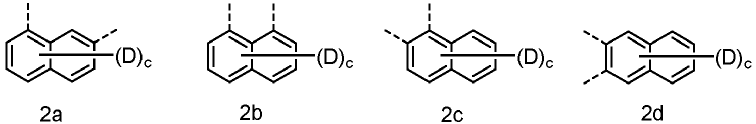 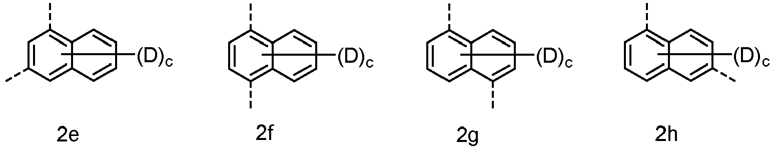 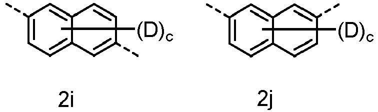 wherein, in Chemical Formulas 2a to 2j,
c is an integer of 0 to 6,
Ar₁ and Ar₂ are each independently phenyl or naphthyl,
L₁ and L₂ are each independently phenylene or naphthylene,
L₃ and L₄ are each independently phenylene,
d and e are each independently an integer of 0 to 7,
f and g are each independently an integer of 0 to 6,
h and i are each independently an integer of 0 to 4, and
a, b, and n1 to n4 are as defined in claim 1.

11. The compound of claim 10, wherein:
one of Ar₁ and Ar₂ is 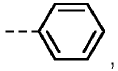 and the rest is 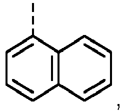 or 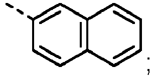 or
one of Ar₁ and Ar₂ is 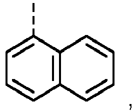 and the rest is 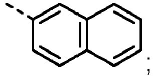 or
both Ar₁ and Ar₂ are 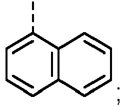 or
both Ar₁ and Ar₂ are

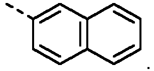

12. The compound of claim 10, wherein:
L₁ and L₂ are each independently any one selected from the group consisting of:

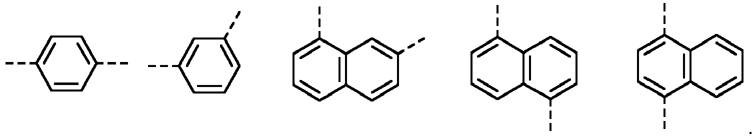

13. The compound of claim 10, wherein:
L₃ and L₄ are each independently

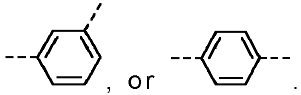

14. The compound of claim 1, wherein:
the compound is any one selected from the group consisting of compounds represented by the following Chemical Formulas: 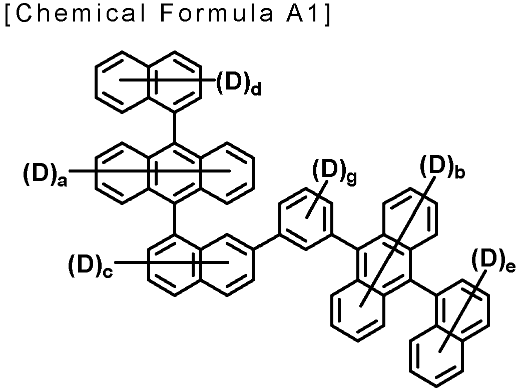
wherein, in Chemical Formula A1,
a+b+c+d+e+g is 1 to 40, 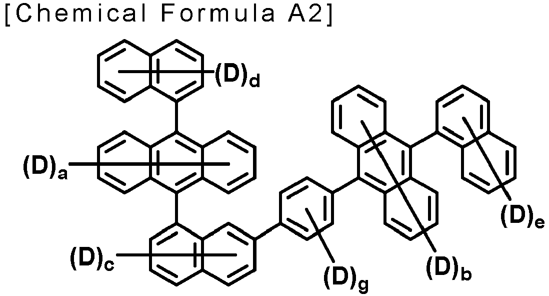
wherein, in Chemical Formula A2,
a+b+c+d+e+g is 1 to 40, 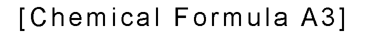 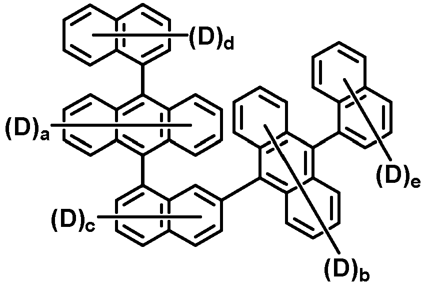
wherein, in Chemical Formula A3,
a+b+c+d+e is 1 to 36, 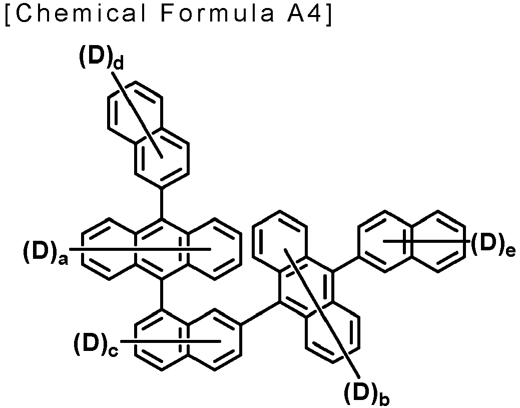
wherein, in Chemical Formula A4,
a+b+c+d+e is 1 to 36, 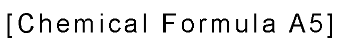 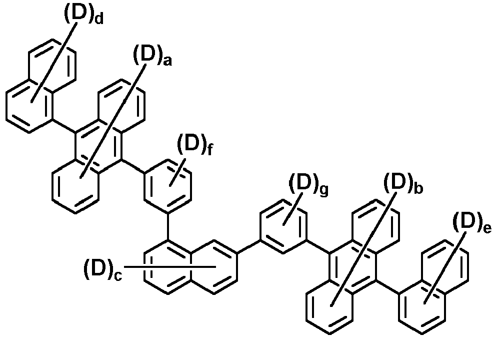
wherein, in Chemical Formula A5,
a+b+c+d+e+f+g is 1 to 44, 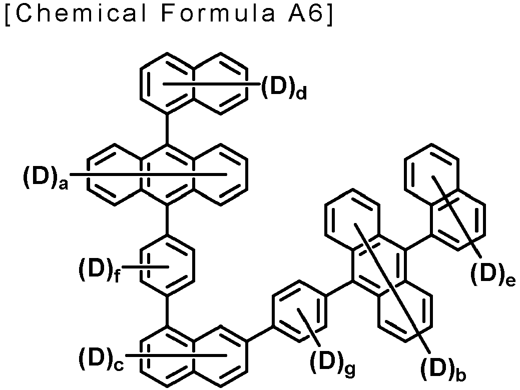
wherein, in Chemical Formula A6,
a+b+c+d+e+f+g is 1 to 44, 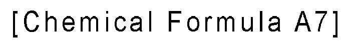 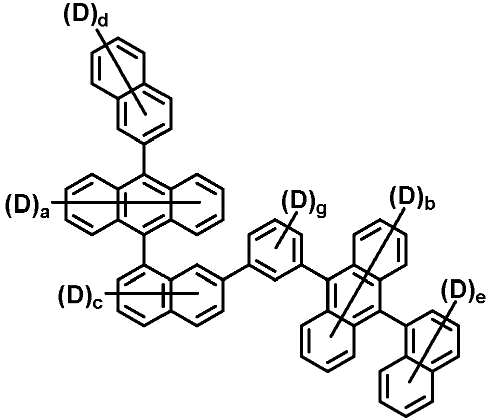
wherein, in Chemical Formula A7,
a+b+c+d+e+g is 1 to 40, 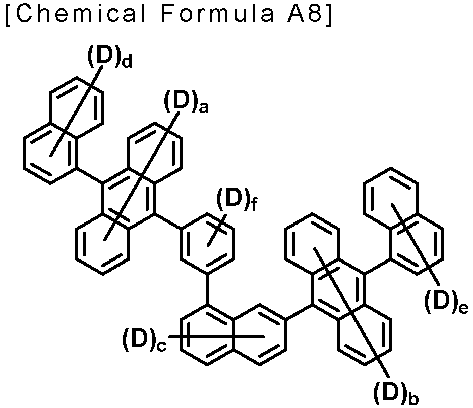
wherein, in Chemical Formula A8,
a+b+c+d+e+f is 1 to 40, 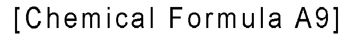 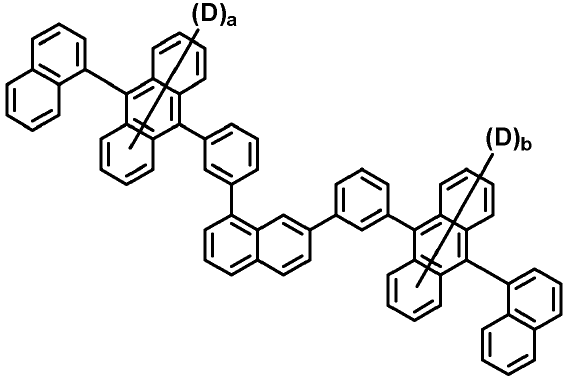
wherein, in Chemical Formula A9,
a+b is 1 to 16, 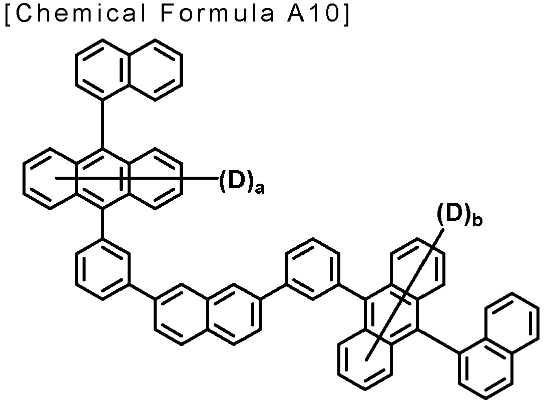
wherein, in Chemical Formula A10,
a+b is 1 to 16, 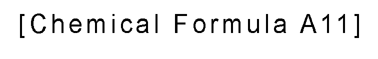 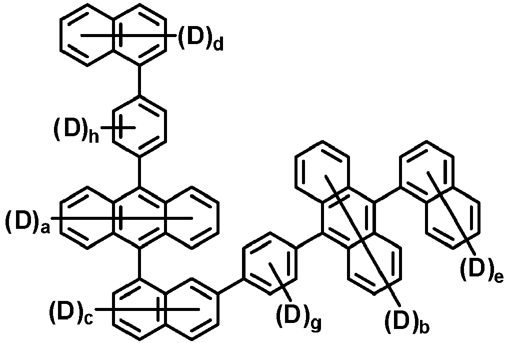
wherein, in Chemical Formula A11,
a+b+c+d+e+g+h is 1 to 44, 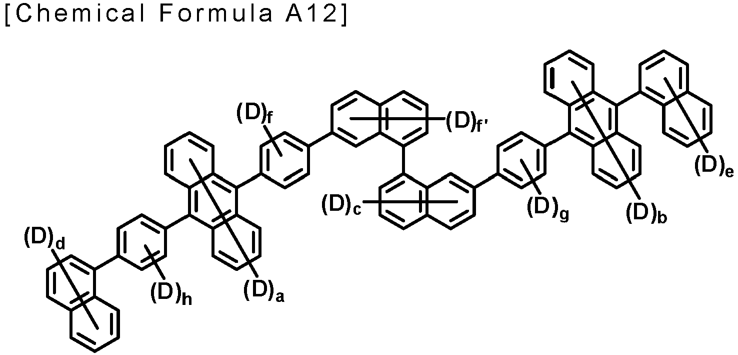
wherein, in Chemical Formula A12,
a+b+c+d+e+f+f'+g+h is 1 to 54, 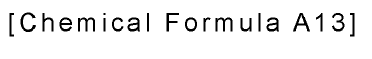 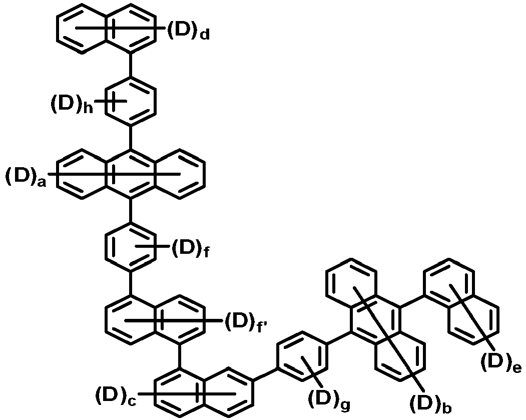
wherein, in Chemical Formula A13,
a+b+c+d+e+f+f'+g+h is 1 to 54, 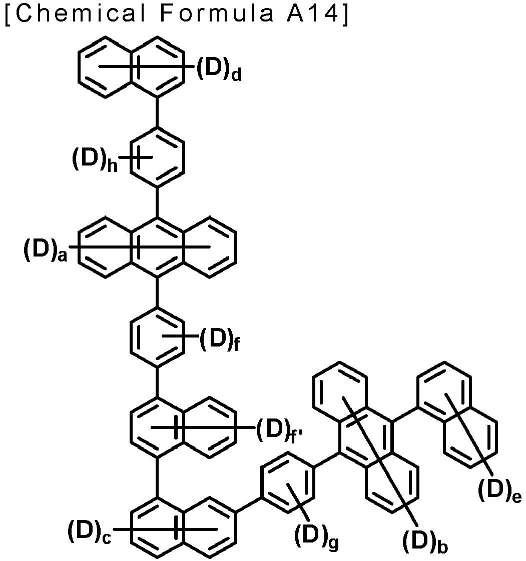
wherein, in Chemical Formula A14,
a+b+c+d+e+f+f'+g+h is 1 to 54, 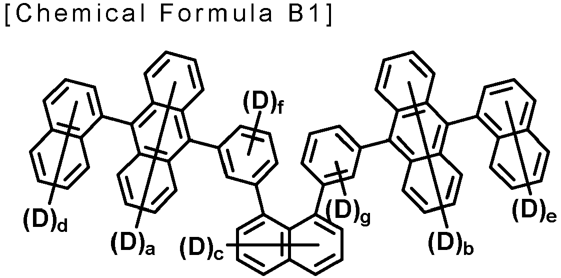
wherein, in Chemical Formula B1,
a+b+c+d+e+f+g is 1 to 44, 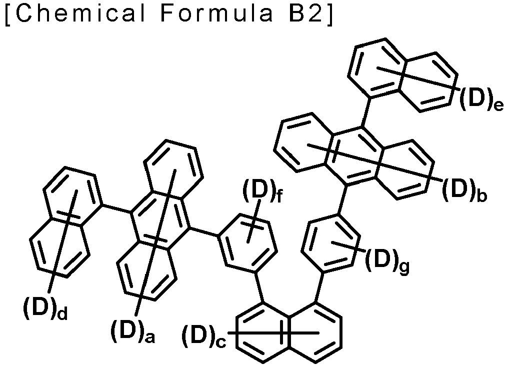
wherein, in Chemical Formula B2,
a+b+c+d+e+f+g is 1 to 44, 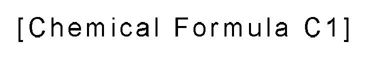 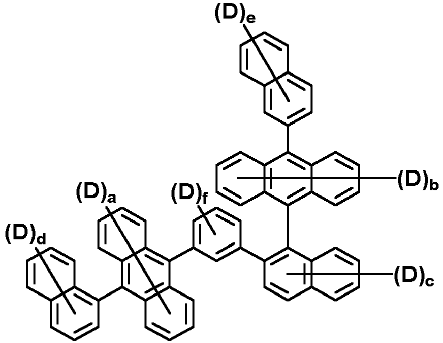
wherein, in Chemical Formula C1,
a+b+c+d+e+f is 1 to 40, 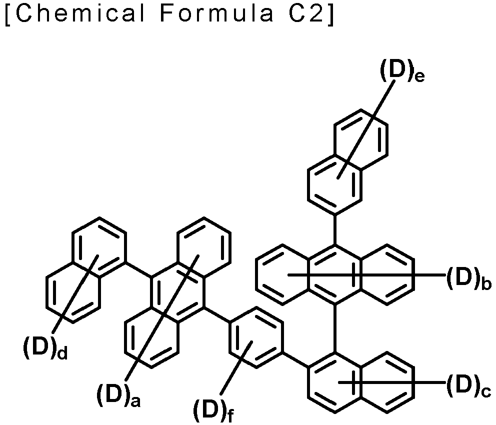
wherein, in Chemical Formula C2,
a+b+c+d+e+f is 1 to 40, 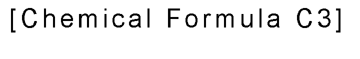 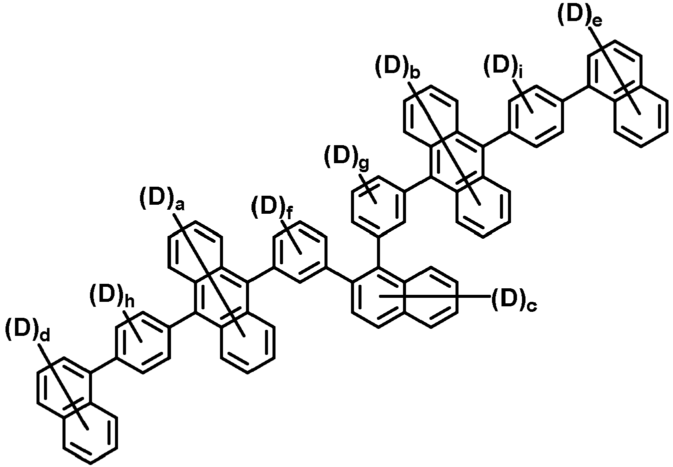
wherein, in Chemical Formula C3,
a+b+c+d+e+f+g+h+i is 1 to 52, 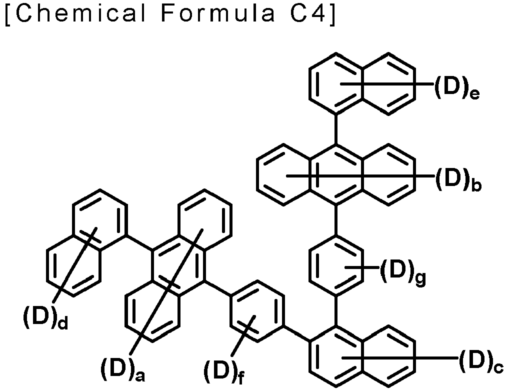
wherein, in Chemical Formula C4,
a+b+c+d+e+f+g is 1 to 44, 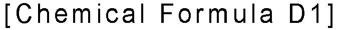 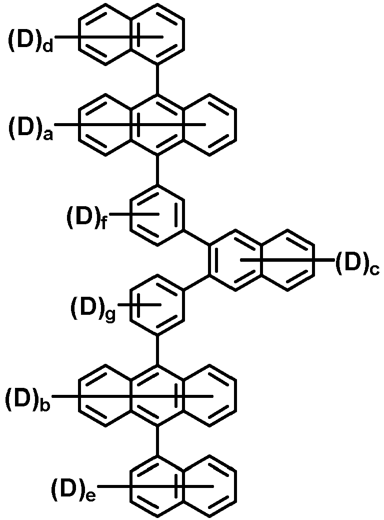
wherein, in Chemical Formula D1,
a+b+c+d+e+f+g is 1 to 44, 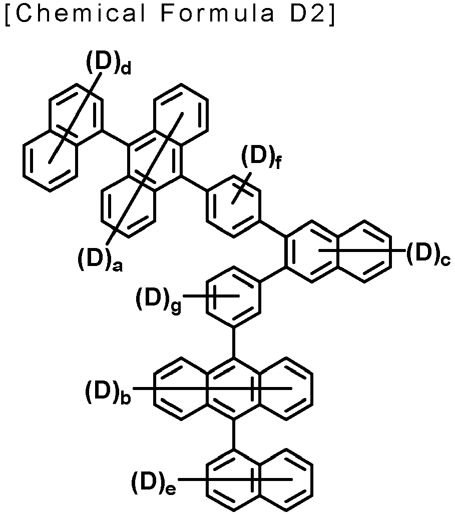
wherein, in Chemical Formula D2,
a+b+c+d+e+f+g is 1 to 44, 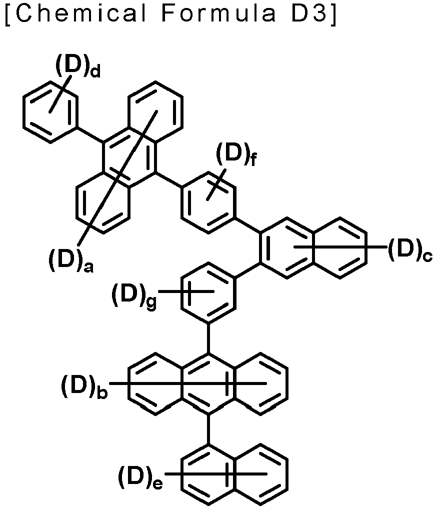
wherein, in Chemical Formula D3,
a+b+c+d+e+f+g is 1 to 42, 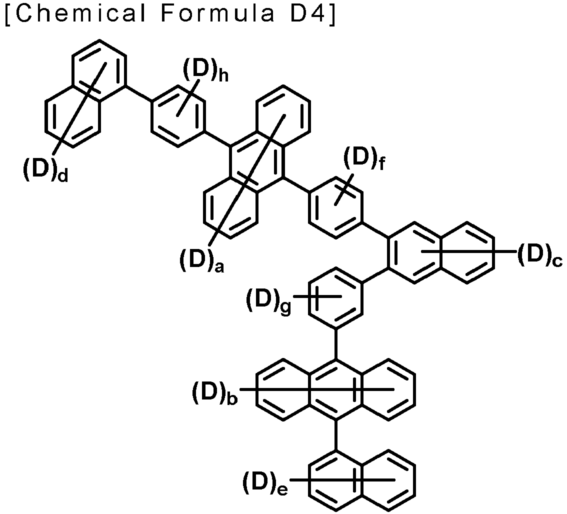
wherein, in Chemical Formula D4,
a+b+c+d+e+f+g+h is 1 to 48, 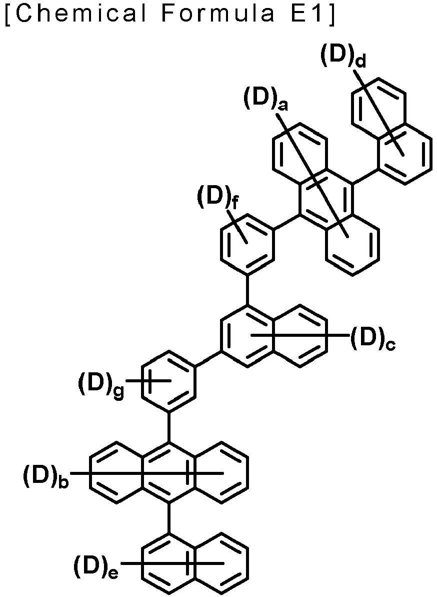
wherein, in Chemical Formula E1,
a+b+c+d+e+f+g is 1 to 44, 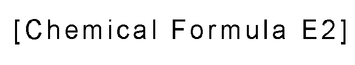 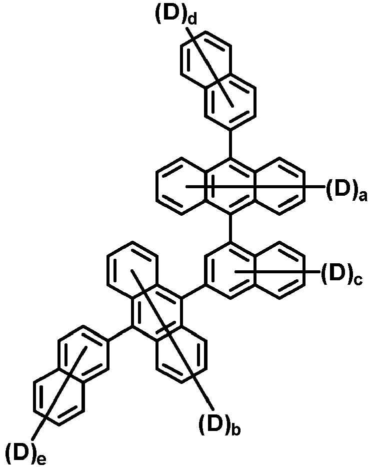
wherein, in Chemical Formula E2,
a+b+c+d+e is 1 to 36, 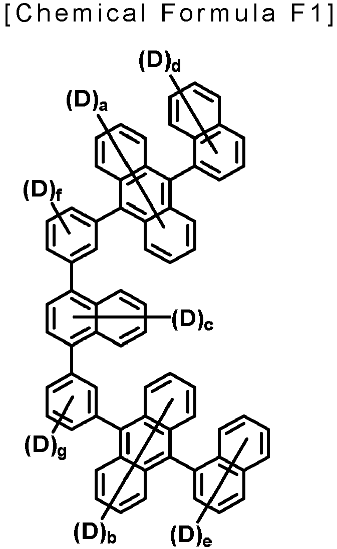
wherein, in Chemical Formula F1,
a+b+c+d+e+f+g is 1 to 44, 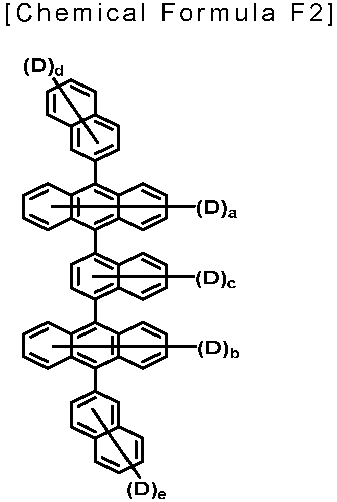
wherein, in Chemical Formula F2,
a+b+c+d+e is 1 to 36, 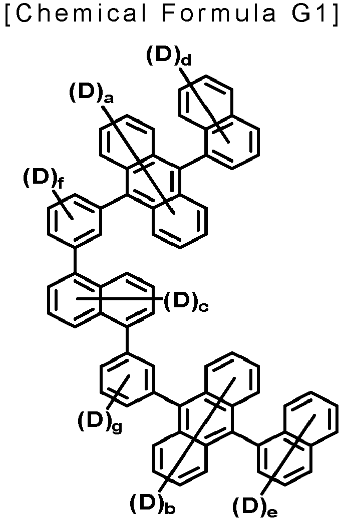
wherein, in Chemical Formula G1,
a+b+c+d+e+f+g is 1 to 44, 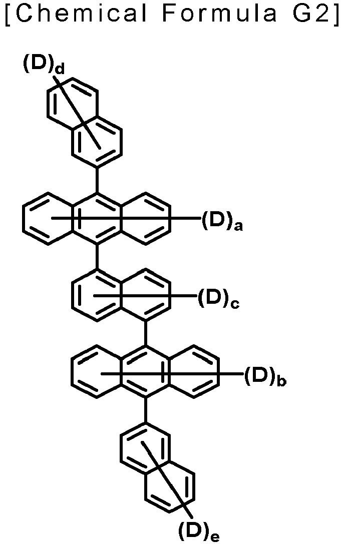
wherein, in Chemical Formula G2,
a+b+c+d+e is 1 to 36, 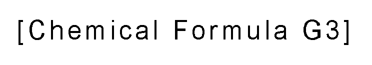 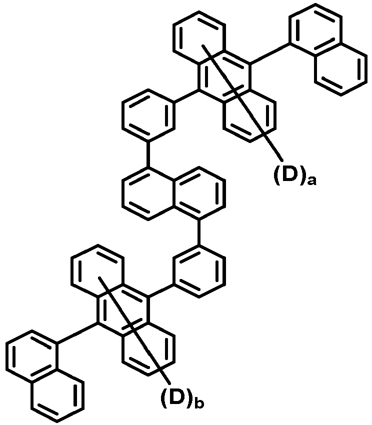
wherein, in Chemical Formula G3,
a+b is 1 to 16, 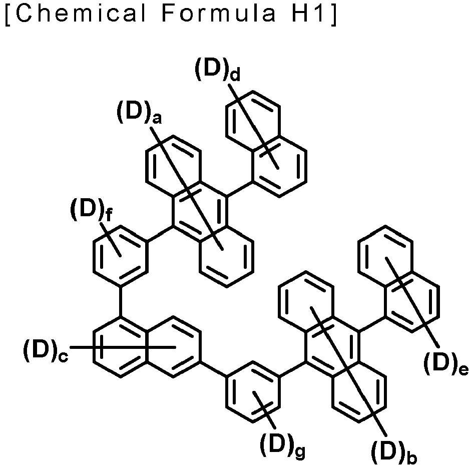
wherein, in Chemical Formula H1,
a+b+c+d+e+f+g is 1 to 44, 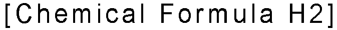 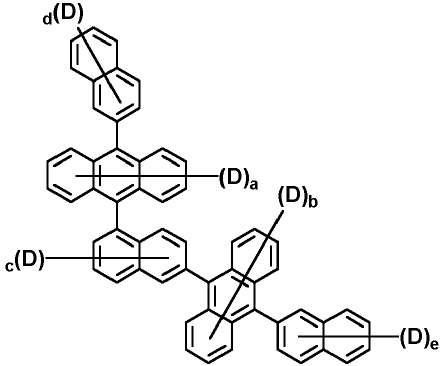
wherein, in Chemical Formula H2,
a+b+c+d+e is 1 to 36, 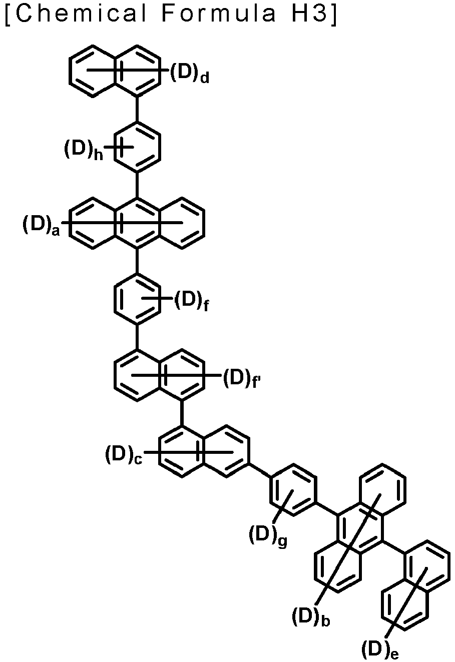
wherein, in Chemical Formula H3,
a+b+c+d+e+f+f'+g+h is 1 to 54, 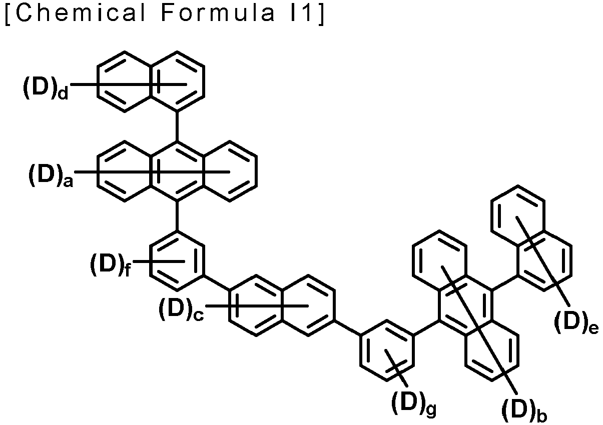
wherein, in Chemical Formula 11,
a+b+c+d+e+f+g is 1 to 44, 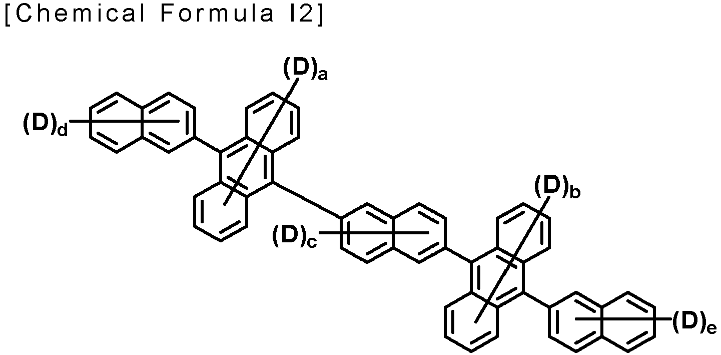
wherein, in Chemical Formula 12,
a+b+c+d+e is 1 to 36, 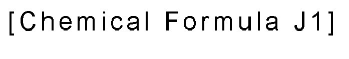 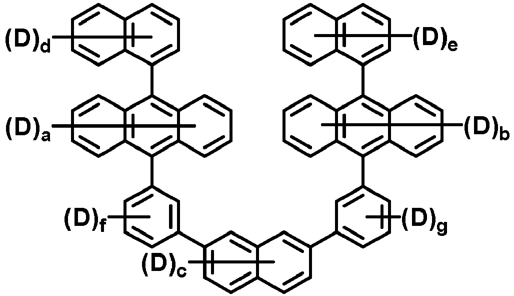
wherein, in Chemical Formula J1,
a+b+c+d+e+f+g is 1 to 44, 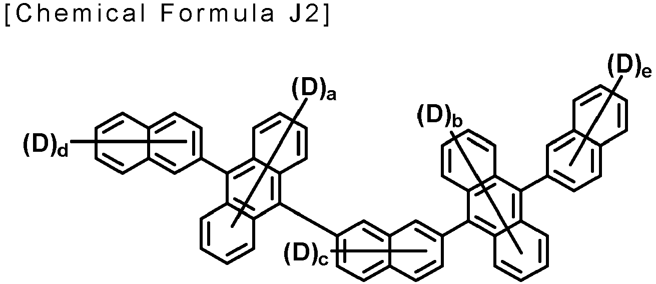
wherein, in Chemical Formula J2,
a+b+c+d+e is 1 to 36.

15. An organic light emitting device comprising: a first electrode; a second electrode that is provided opposite to the first electrode; and a light emitting layer that is provided between the first electrode and the second electrode, wherein the light emitting layer comprises the compound as set forth in any one of claims 1 to 14.

## Patentansprüche

1. Verbindung, dargestellt durch die folgende chemische Formel 1: 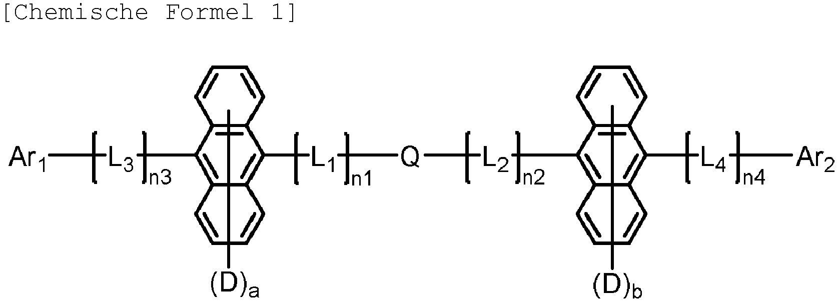 worin in der chemischen Formel 1
D Deuterium darstellt,
Q Naphthylen ist, das unsubstituiert ist oder mit Deuterium substituiert ist,
Ar₁ und Ar₂ jeweils unabhängig ein substituiertes oder unsubstituiertes C₆₋₆₀-Aryl darstellen,
L₁ bis L₄ jeweils unabhängig ein substituiertes oder unsubstituiertes C₆₋₆₀-Aryl darstellen,
n1 bis n4 jeweils unabhängig eine ganze Zahl von 0 bis 2 darstellen und
a und b jeweils unabhängig eine ganze Zahl von 0 bis 8 sind,
vorausgesetzt, dass a+b 1 oder größer ist.

2. Verbindung gemäß Anspruch 1, worin:
Q irgendeiner der folgenden divalenten Substituenten ist, dargestellt durch die folgenden chemischen Formeln 2a bis 2j: 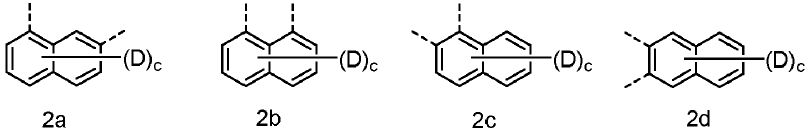 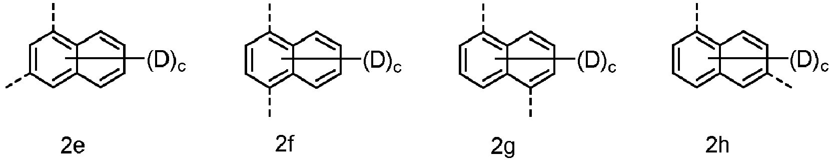 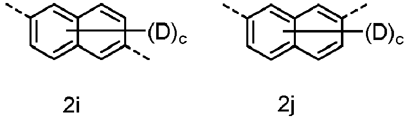 worin in den chemischen Formeln 2a bis 2j
c eine ganze Zahl von 0 bis 6 ist.

3. Verbindung gemäß Anspruch 1, worin:
Ar₁ und Ar₂ jeweils unabhängig Phenyl, das unsubstituiert ist oder mit 1 bis 5 Deuterium substituiert ist; oder Naphthyl, das unsubstituiert ist oder mit 1 bis 7 Deuterium substituiert ist, sind.

4. Verbindung gemäß Anspruch 1, worin:
zumindest eines von Ar₁ und Ar₂ 1-Naphthyl ist, das unsubstituiert ist oder substituiert ist mit 1 bis 7 Deuterium;
oder worin zumindest eines von Ar₁ und Ar₂ 2-Naphthyl ist, das unsubstituiert ist oder mit 1 bis 7 Deuterium substituiert ist.

5. Verbindung gemäß Anspruch 1, worin:
L₁ und L₂ jeweils unabhängig Phenylen, das unsubstituiert ist oder mit 1 bis 4 Deuterium substituiert ist; oder Naphthylen, das unsubstituiert oder mit 1 bis 6 Deuterium substituiert ist, sind.

6. Verbindung gemäß Anspruch 1, worin:
L₃ und L₄ jeweils unabhängig Phenylen, das unsubstituiert ist oder mit 1 bis 4 Deuterium substituiert ist, sind.

7. Verbindung gemäß Anspruch 1, worin:
n1+n2 0, 1, 2 oder 3 ist und
n3+n4 0, 1 oder 2 ist.

8. Verbindung gemäß Anspruch 1, worin:
a+b 16 ist.

9. Verbindung gemäß Anspruch 1, worin:
der Deuterium-Substitutionsgrad der Verbindung 80 % bis 100 % beträgt.

10. Verbindung gemäß Anspruch 1, worin:
die Verbindung durch die folgende chemische Formel 1-1 dargestellt ist: 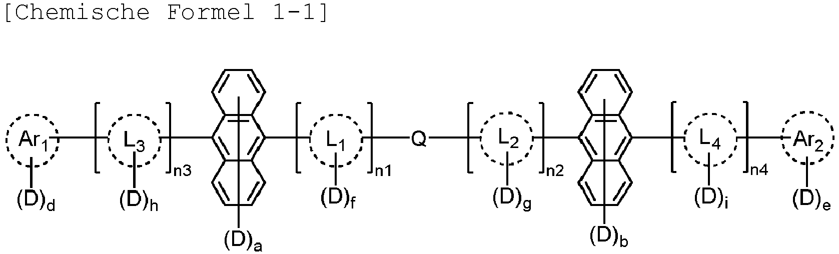 worin in der chemischen Formel 1-1,
Q irgendeiner der divalenten Substituenten ist, die durch die folgenden chemischen Formeln 2a bis 2j dargestellt sind: 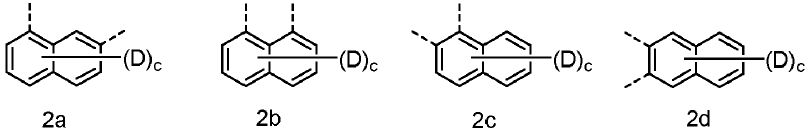 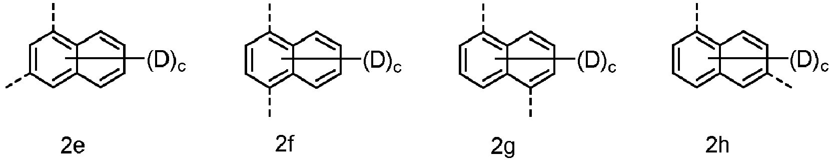 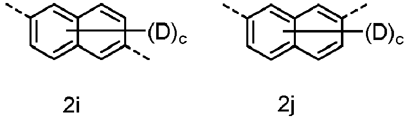 worin in den chemischen Formeln 2a bis 2j c eine ganze Zahl von 0 bis 6 ist,
Ar₁ und Ar₂ jeweils unabhängig Phenyl oder Naphthyl sind,
L₁ und L₂ jeweils unabhängig Phenylen oder Naphthylen sind,
L₃ und L₄ jeweils unabhängig Phenylen sind,
d und e jeweils unabhängig eine ganze Zahl von 0 bis 7 sind,
f und g jeweils unabhängig eine ganze Zahl von 0 bis 6 sind,
h und i jeweils unabhängig eine ganze zahl von 0 bis 4 sind und
a, b und n1 bis n4 wie in Anspruch 1 definiert sind.

11. Verbindung gemäß Anspruch 10, worin:
eines von Ar₁ und Ar₂ 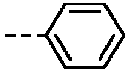 ist und der Rest 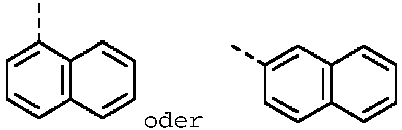 ist; oder
eines von Ar₁ und Ar₂ 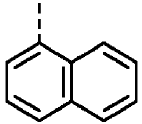 ist und der Rest 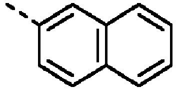 ist; oder
beide von Ar₁ und Ar₂ 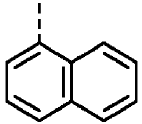 sind; oder
beide von Ar₁ und Ar₂ 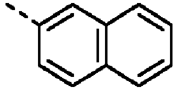 sind.

12. Verbindung gemäß Anspruch 10, worin:
L₁ und L₂ jeweils unabhängig irgendeines sind, das ausgewählt ist aus der Gruppe bestehend aus

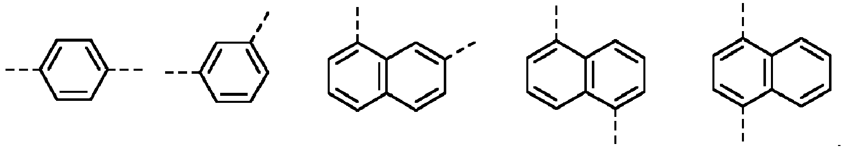

13. Verbindung gemäß Anspruch 10, worin:
L₃ und L₄ jeweils unabhängig 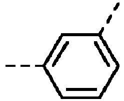 oder 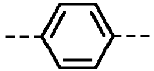 sind.

14. Verbindung gemäß Anspruch 1, worin:
die Verbindung irgendeine ist, die ausgewählt ist aus der Gruppe, bestehend aus durch die folgenden chemischen Formeln dargestellten Verbindungen: 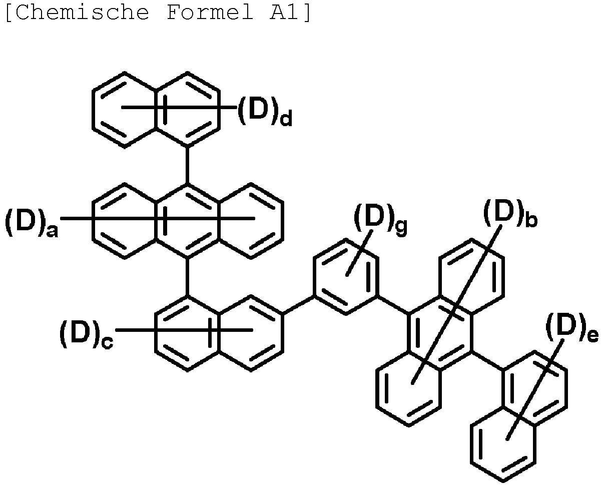
worin in der chemischen Formel A1
a+b+c+d+e+g 1 bis 40 ist, 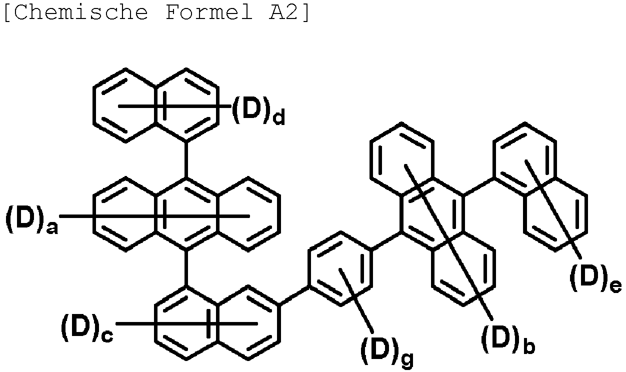
worin in der chemischen Formel A2
a+b+c+d+e+g 1 bis 40 ist, 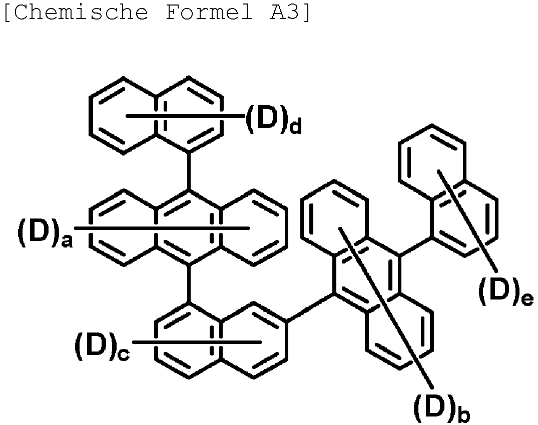
worin in der chemischen Formel A3
a+b+c+d+e 1 bis 36 ist, 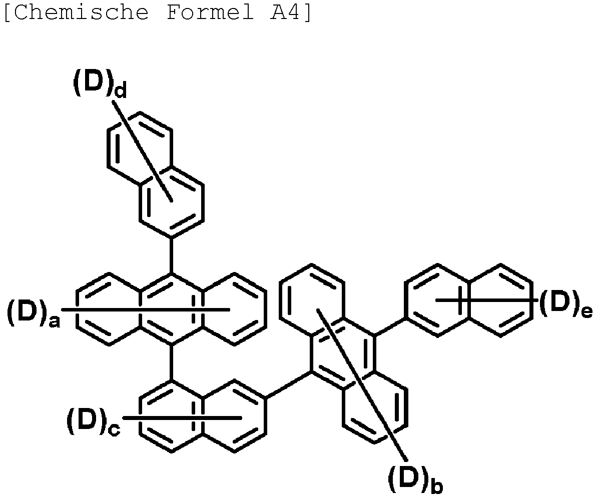
worin in der chemischen Formel A4
a+b+c+d+e 1 bis 36 ist, 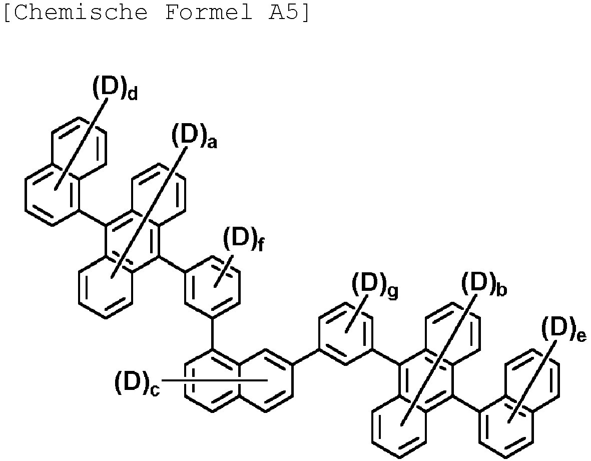
worin in der chemischen Formel A5
a+b+c+d+e+f+g 1 bis 44 ist, 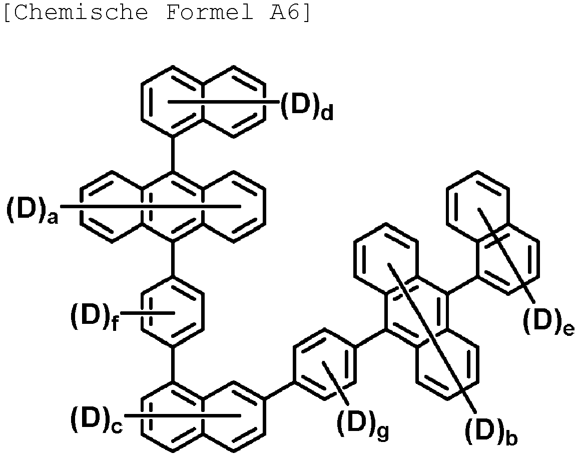
worin in der chemischen Formel A6
a+b+c+d+e+f+g 1 bis 44 ist, 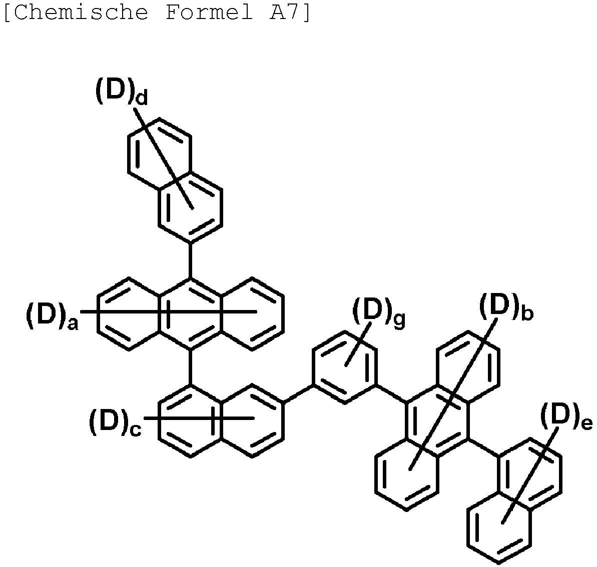
worin in der chemischen Formel A7
a+b+c+d+e+g 1 bis 40 ist, 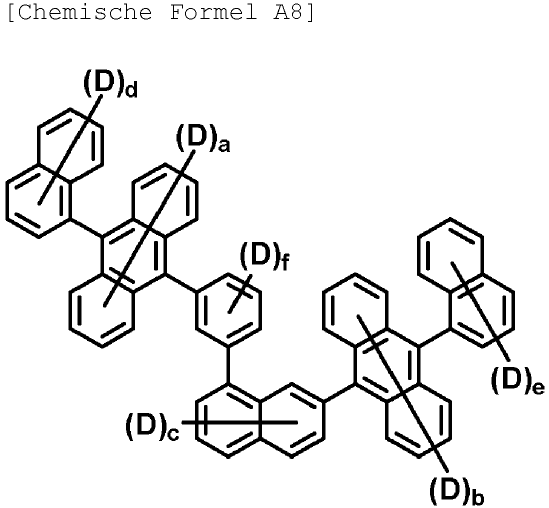
worin in der chemischen Formel A8
a+b+c+d+e+f 1 bis 40 ist, 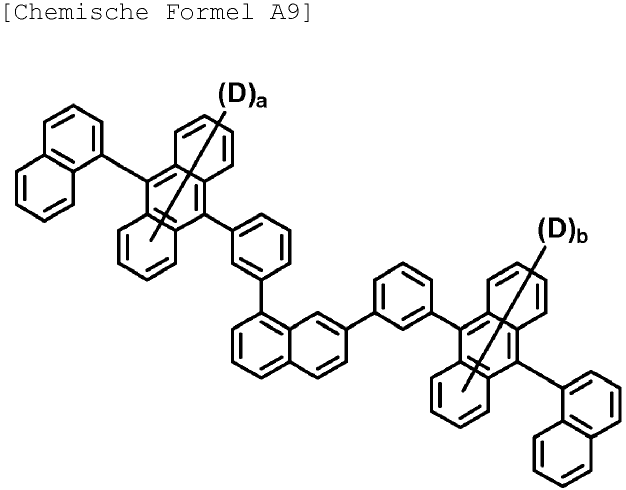
worin in der chemischen Formel A9
a+b 1 bis 16 ist, 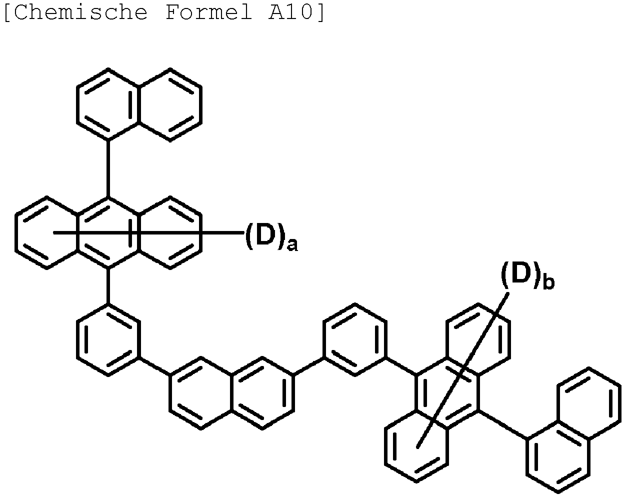
worin in der chemischen Formel A10
a+b 1 bis 16 ist, 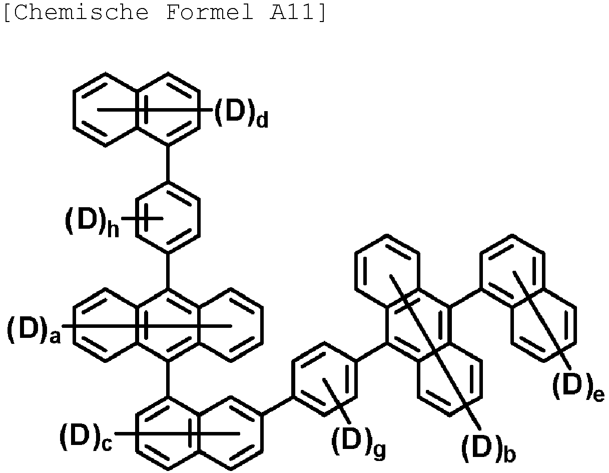
worin in der chemischen Formel A11
a+b+c+d+e+g+h 1 bis 44 ist, 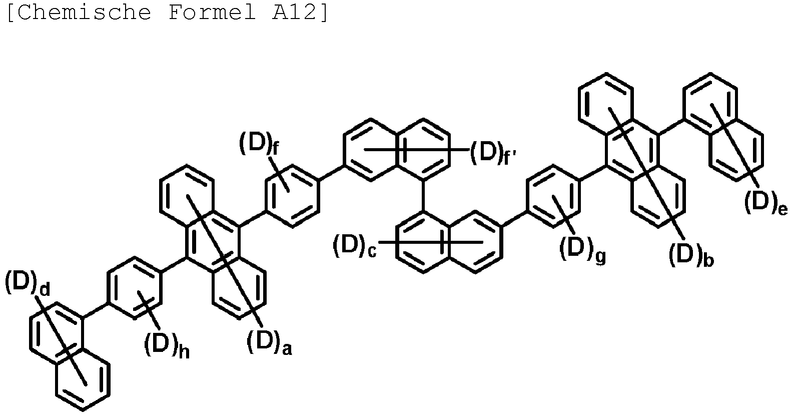
worin in der chemischen Formel A12
a+b+c+d+e+f+f'+g+h 1 bis 54 ist, 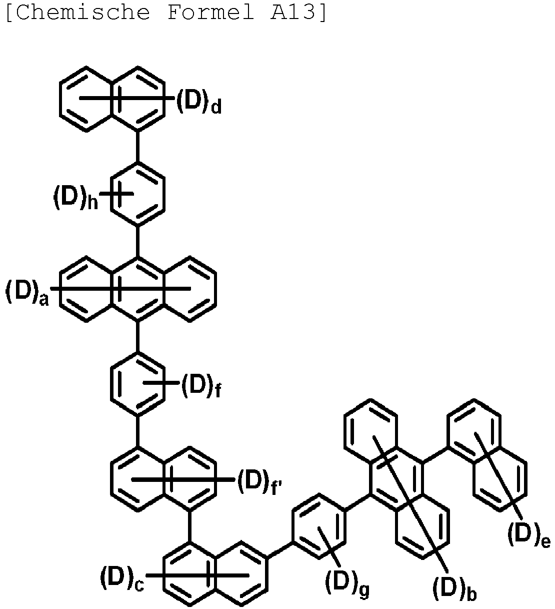
worin in der chemischen Formel A13
a+b+c+d+e+f+f'+g+h 1 bis 54 ist, 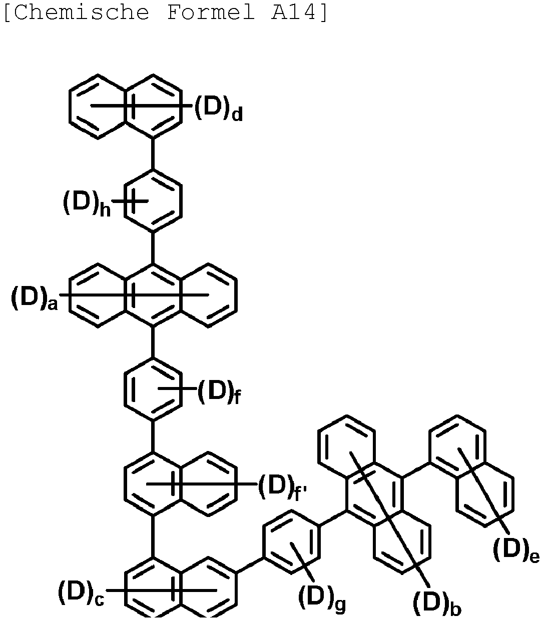
worin in der chemischen Formel A14
a+b+c+d+e+f+f'+g+h 1 bis 54 ist, 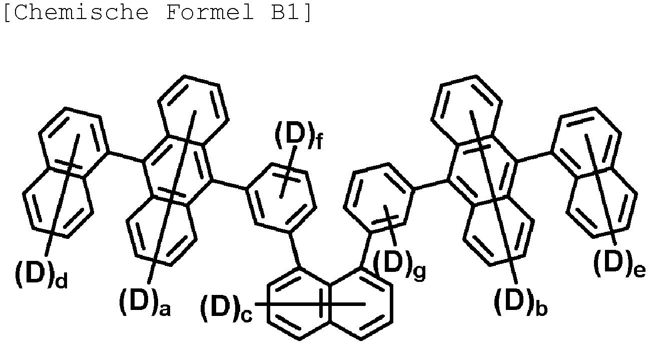
worin in der chemischen Formel B1
a+b+c+d+e+f+g 1 bis 44 ist, 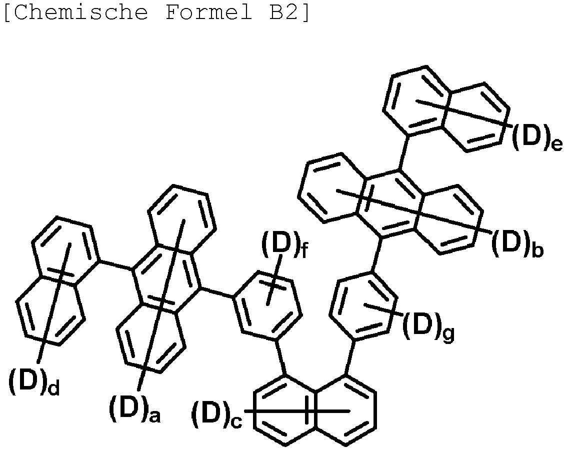
worin in der chemischen Formel B2
a+b+c+d+e+f+g 1 bis 44 ist, 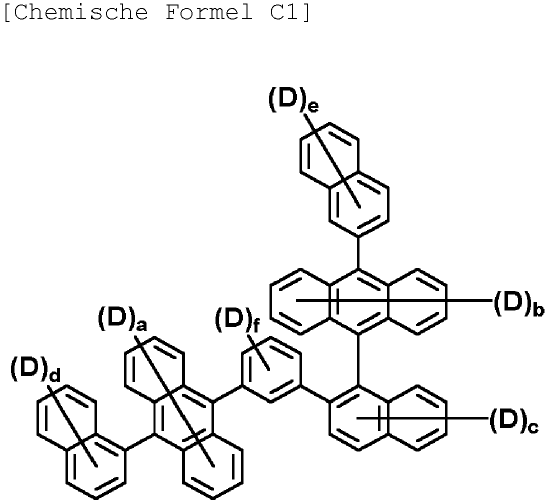
worin in der chemischen Formel C1
a+b+c+d+e+f 1 bis 40 ist, 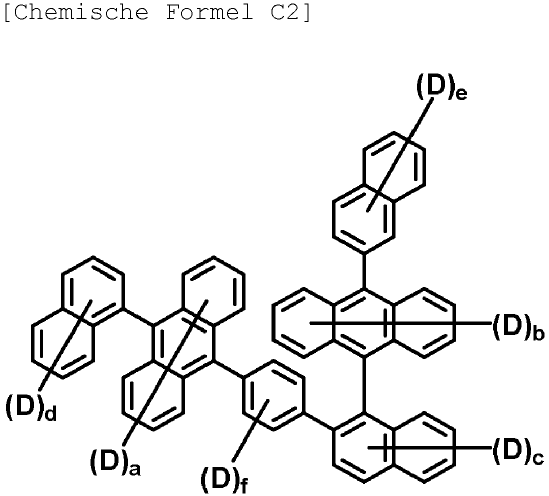
worin in der chemischen Formel C2
a+b+c+d+e+f 1 bis 40 ist, 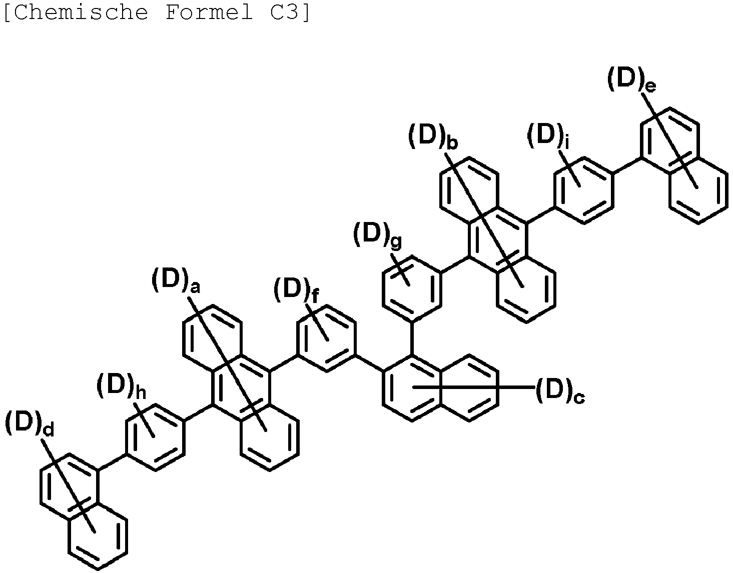
worin in der chemischen Formel C3
a+b+c+d+e+f+g+h+i 1 bis 52 ist, 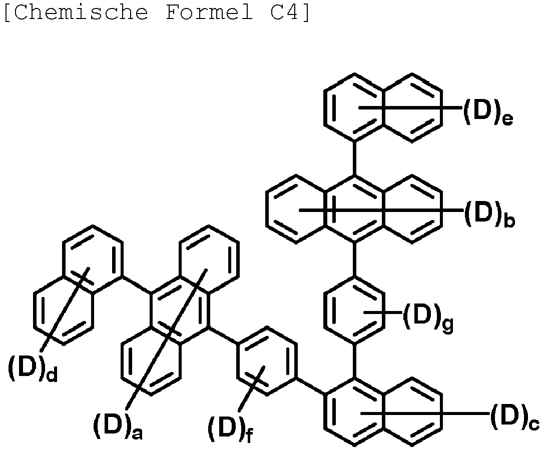
worin in der chemischen Formel C4
a+b+c+d+e+f+g 1 bis 44 ist, 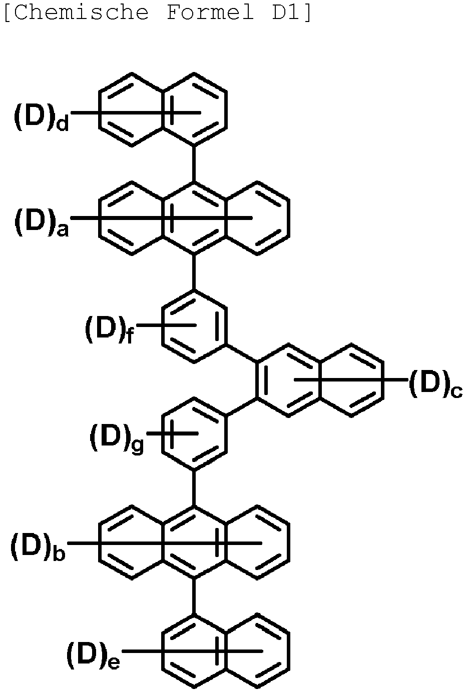
worin in der chemischen Formel D1
a+b+c+d+e+f+g 1 bis 44 ist, 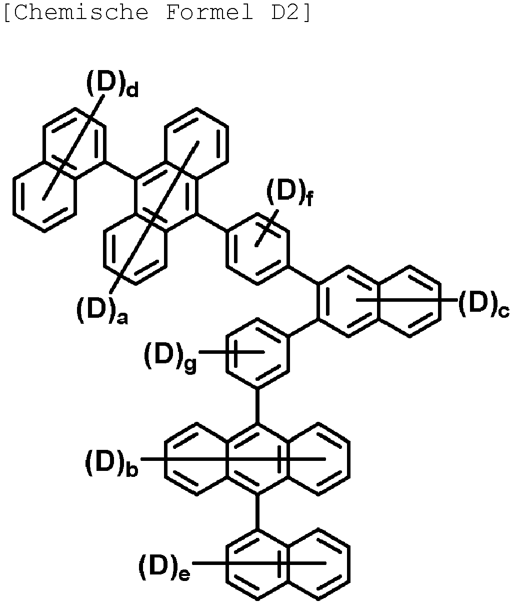
worin in der chemischen Formel D2
a+b+c+d+e+f+g 1 bis 44 ist, 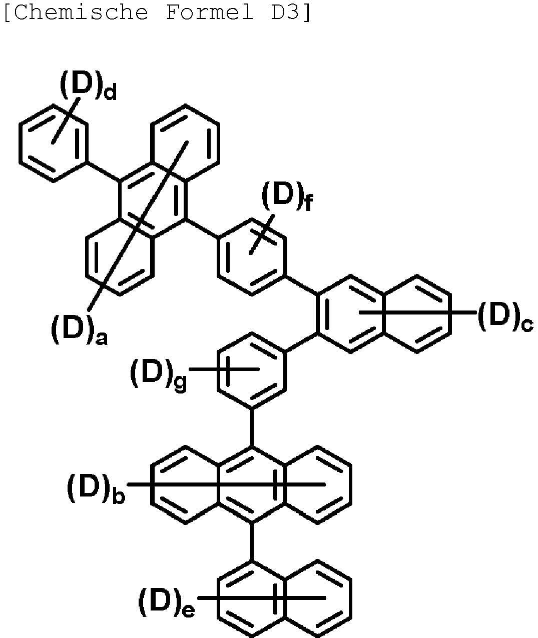
worin in der chemischen Formel D3
a+b+c+d+e+f+g 1 bis 42 ist, 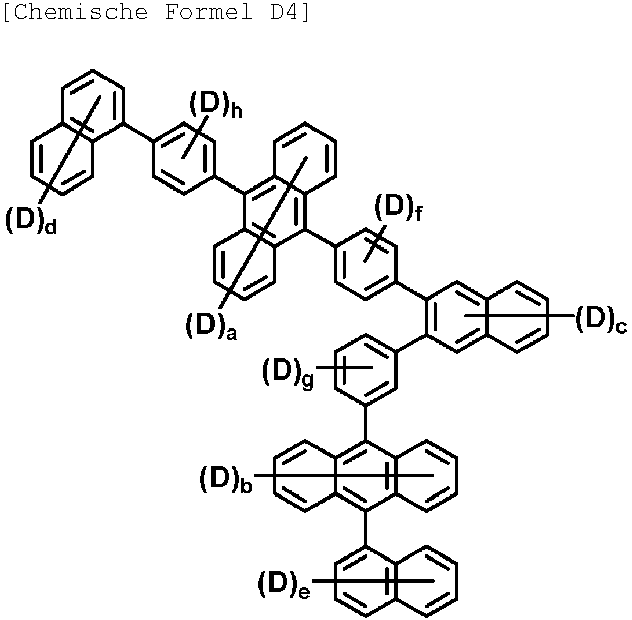
worin in der chemischen Formel D4
a+b+c+d+e+f+g+h 1 bis 48 ist, 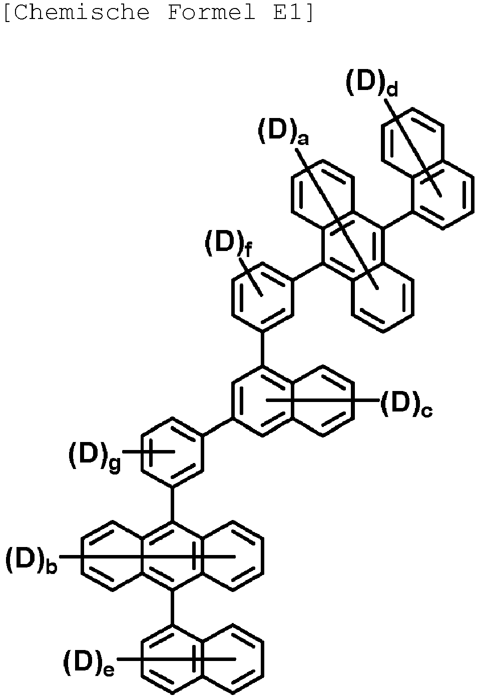
worin in der chemischen Formel E1
a+b+c+d+e+f+g 1 bis 44 ist, 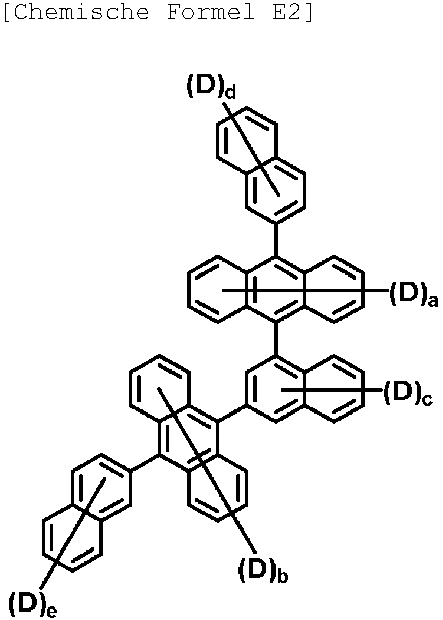
worin in der chemischen Formel E2
a+b+c+d+e 1 bis 36 ist, 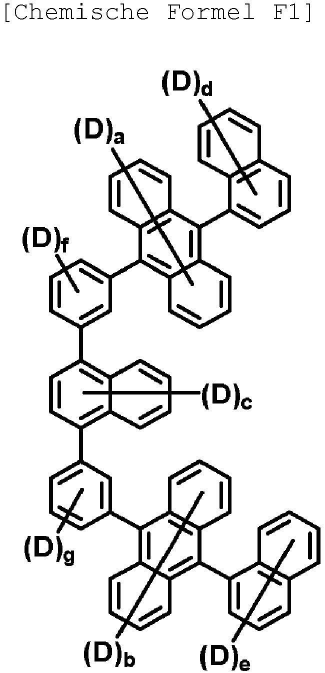
worin in der chemischen Formel F1
a+b+c+d+e+f+g 1 bis 44 ist, 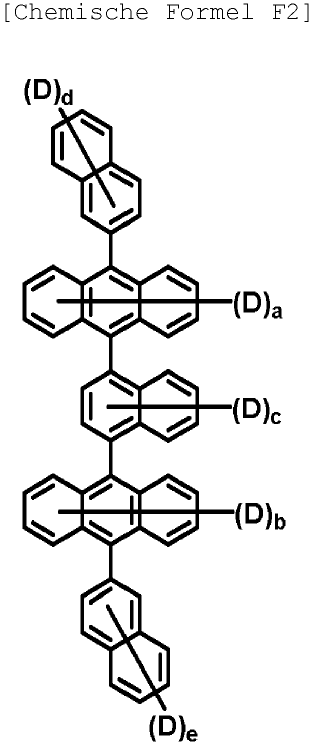
worin in der chemischen Formel F2
a+b+c+d+e 1 bis 36 ist, 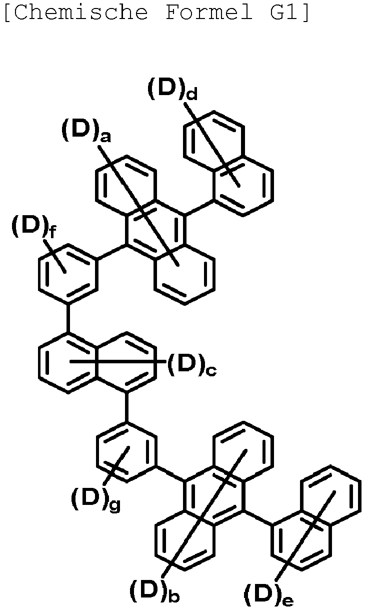
worin in der chemischen Formel G1
a+b+c+d+e+f+g 1 bis 44 ist, 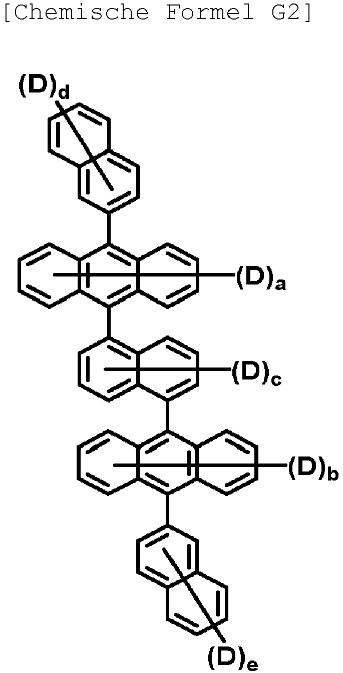
worin in der chemischen Formel G2
a+b+c+d+e 1 bis 36 ist, 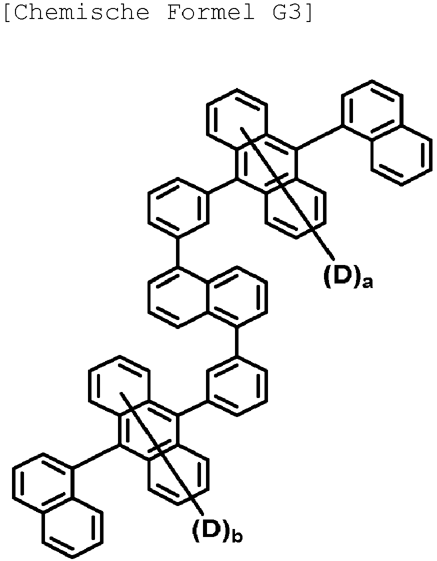
worin in der chemischen Formel G3
a+b 1 bis 16 ist, 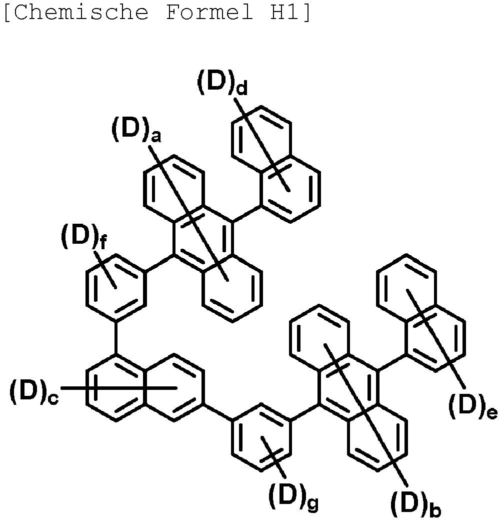
worin in der chemischen Formel H1
a+b+c+d+e+f+g 1 bis 44 ist, 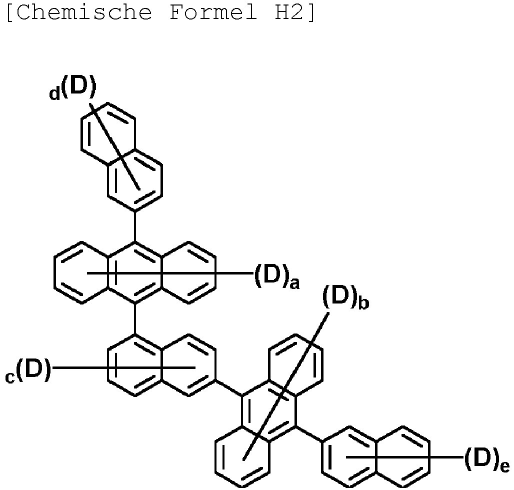
worin in der chemischen Formel H2
a+b+c+d+e 1 bis 36 ist, 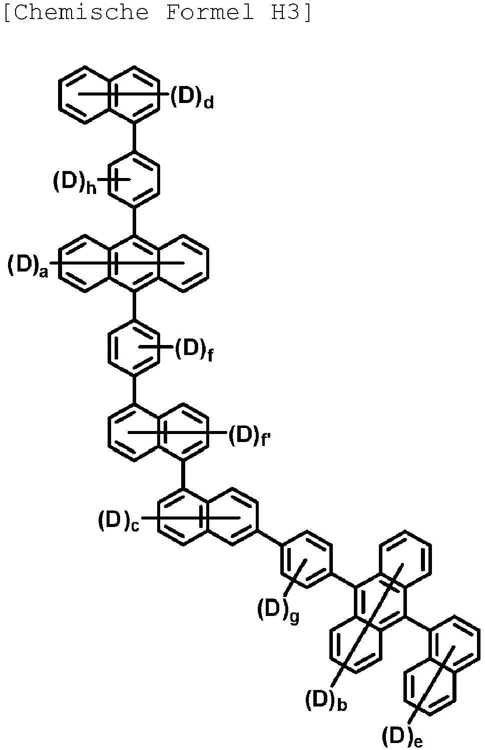
worin in der chemischen Formel H3
a+b+c+d+e+f+f'+g+h 1 bis 54 ist, 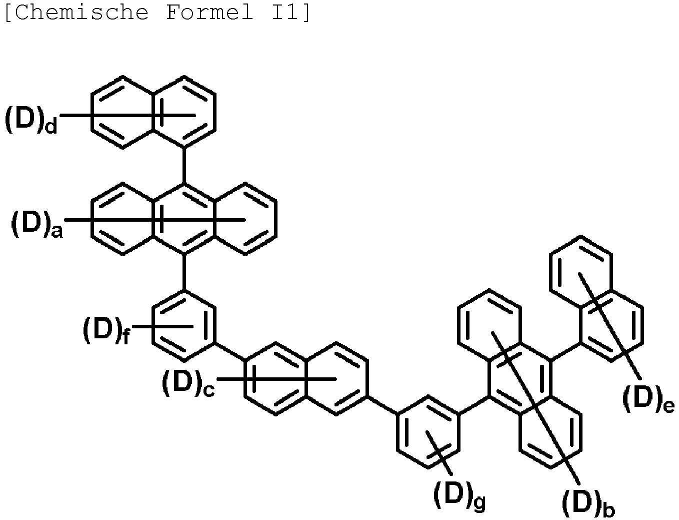
worin in der chemischen Formel I1
a+b+c+d+e+f+g 1 bis 44 ist, 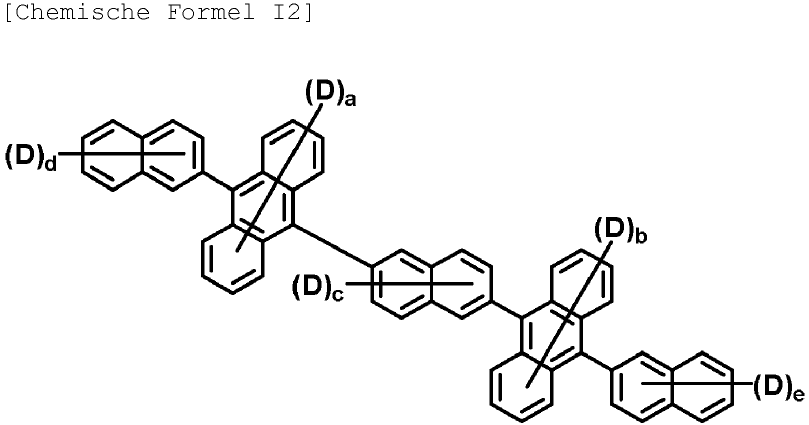
worin in der chemischen Formel I2
a+b+c+d+e 1 bis 36 ist, 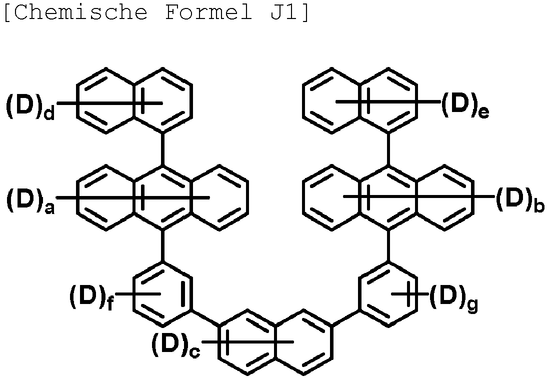
worin in der chemischen Formel J1
a+b+c+d+e+f+g 1 bis 44 ist, 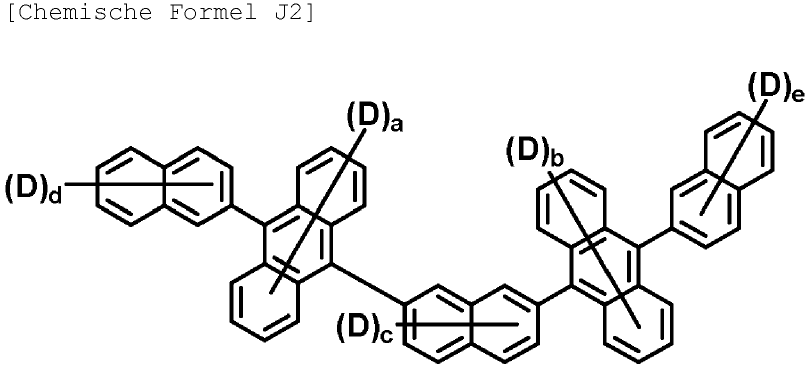
worin in der chemischen Formel J2
a+b+c+d+e 1 bis 36 ist.

15. Organische lichtemittierende Vorrichtung, umfassend:
eine erste Elektrode; eine zweite Elektrode, die der ersten Elektrode gegenüberliegend vorgesehen ist; und
eine lichtemittierende Schicht, die zwischen der ersten Elektrode und der zweiten Elektrode vorgesehen ist,
worin die lichtemittierende Schicht die Verbindung umfasst, wie sie in irgendeinem der Ansprüche 1 bis 14 definiert ist.

## Revendications

1. Composé représenté par la Formule chimique 1 suivante : 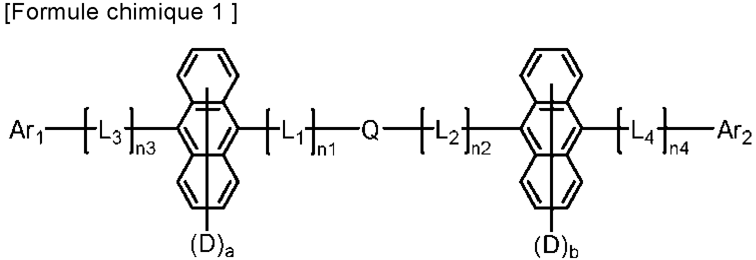 dans lequel, dans la Formule chimique 1,
D représente le deutérium,
Q est le naphthylène non substitué ou substitué par le deutérium,
Ar₁ et Ar₂ sont chacun indépendamment un aryle en C₆₋₆₀ substitué ou non substitué,
L₁ à L₄ sont chacun indépendamment un arylène en C₆₋₆₀ substitué ou non substitué,
n1 à n4 sont chacun indépendamment un nombre entier de 0 à 2, et
a et b sont chacun indépendamment un nombre entier de 0 à 8,
à la condition que a + b vaut 1 ou plus.

2. Composé selon la revendication 1, dans lequel :
Q est l'un quelconque des substituants divalents représentés par les Formules chimiques 2a à 2j : 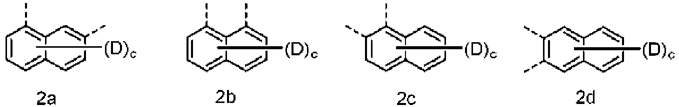 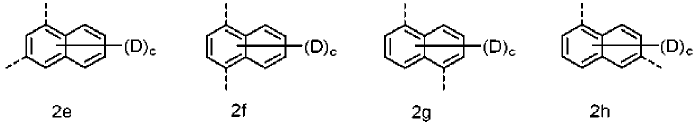 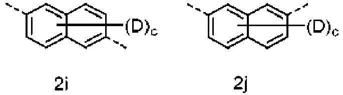 dans lequel, dans les Formules chimiques 2a à 2j,
c est un nombre entier de 0 à 6.

3. Composé selon la revendication 1, dans lequel :
Ar₁ et Ar₂ sont chacun indépendamment un phényle non substitué ou substitué par 1 à 5 deutériums ; ou un naphthyle non substitué ou substitué par 1 à 7 deutériums.

4. Composé selon la revendication 1, dans lequel :
au moins l'un de Ar₁ et Ar₂ est le 1-naphthyle non substitué ou substitué par 1 à 7 deutériums ; ou
au moins l'un de Ar₁ et Ar₂ est le 2-naphthyle non substitué ou substitué par 1 à 7 deutériums.

5. Composé selon la revendication 1, dans lequel :
L₁ et L₂ sont chacun indépendamment un phénylène non substitué ou substitué par 1 à 4 deutériums ; ou un naphthylène non substitué ou substitué par 1 à 6 deutériums.

6. Composé selon la revendication 1, dans lequel :
L₃ et L₄ sont chacun indépendamment un phénylène non substitué ou substitué par 1 à 4 deutériums.

7. Composé selon la revendication 1, dans lequel :
n1 + n2 vaut 0, 1, 2, ou 3, et
n3 + n4 vaut 0, 1, ou 2.

8. Composé selon la revendication 1, dans lequel :
a + b vaut 16.

9. Composé selon la revendication 1, dans lequel :
un taux de substitution du deutérium du composé est de 80 % à 100 %.

10. Composé selon la revendication 1, dans lequel :
le composé est représenté par la Formule chimique 1-1 suivante : 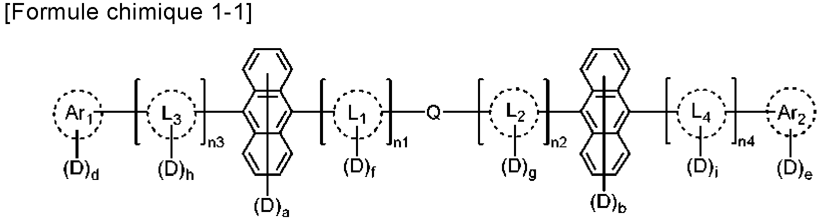 dans lequel, dans la Formule chimique 1-1,
Q est l'un quelconque des substituants divalents représentés par les Formules chimiques 2a à 2j suivantes, 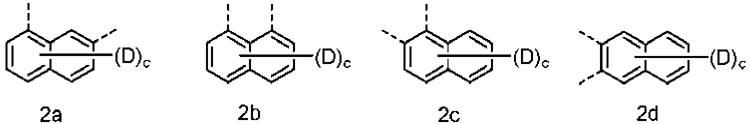 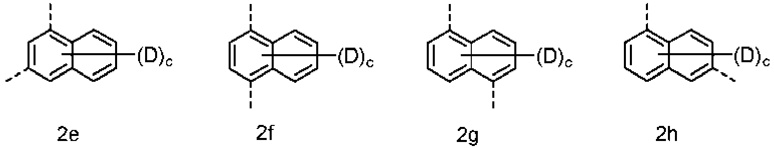 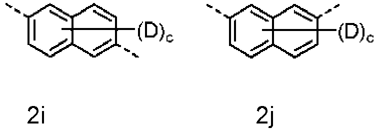 dans lequel, dans les Formules chimiques 2a à 2j,
c est un nombre entier de 0 à 6,
Ar₁ et Ar₂ sont chacun indépendamment un phényle ou un naphthyle,
L₁ et L₂ sont chacun indépendamment un phénylène ou un naphthylène,
L₃ et L₄ sont chacun indépendamment un phénylène,
d et e sont chacun indépendamment un nombre entier de 0 à 7,
f et g sont chacun indépendamment un nombre entier de 0 à 6,
h et i sont chacun indépendamment un nombre entier de 0 à 4, et
a, b, et n1 à n4 sont tels que définis dans la revendication 1.

11. Composé selon la revendication 10, dans lequel :
l'un de AR₁ et AR₂ est 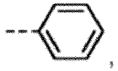 et le reste est 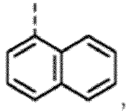 ou 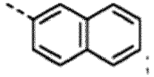 ou
l'un de AR₁ et AR₂ est 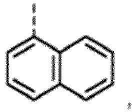 et le reste est 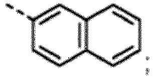 ou
à la fois AR₁ et AR₂ sont 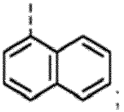 ou
à la fois AR₁ et AR₂ sont

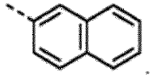

12. Composé selon la revendication 10, dans lequel :
L₁ et L₂ son chacun indépendamment l'un quelconque choisi dans le groupe constitué de :

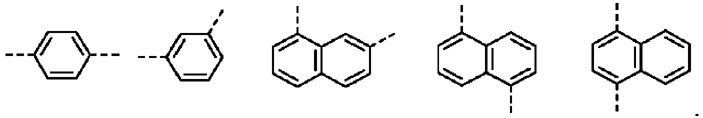

13. Composé selon la revendication 10, dans lequel :
L₃ et L₄ sont chacun indépendamment

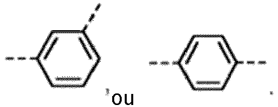

14. Composé selon la revendication 1, dans lequel :
le composé est l'un quelconque choisi dans le groupe constitué des composés représentés par les Formules chimiques suivantes : 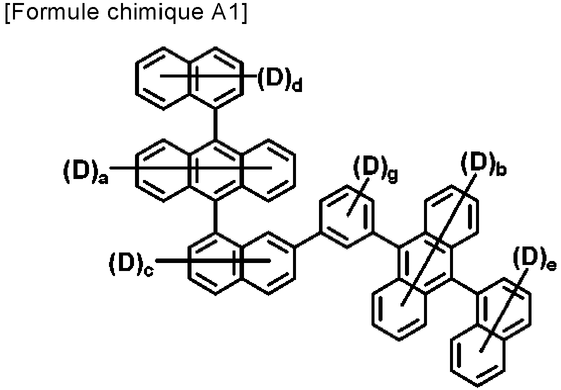
dans lequel, dans la Formule chimique A1,
a + b + c + d + e + g vaut 1 à 40, 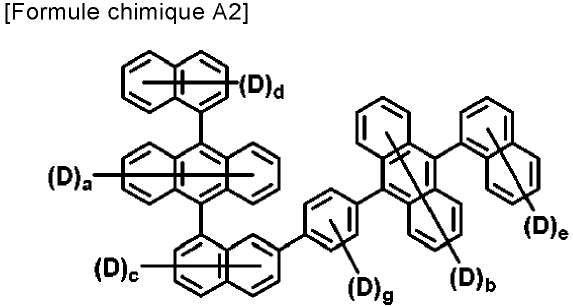
dans lequel, dans la Formule chimique A2,
a + b + c + d + e + g vaut 1 à 40, 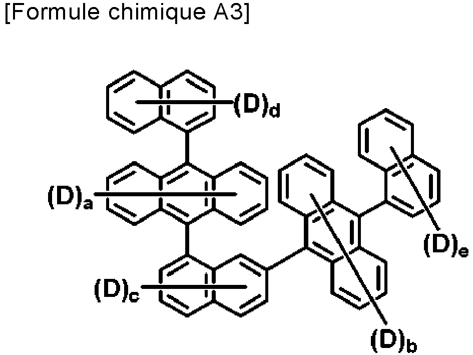
dans lequel, dans la Formule chimique A3,
a + b + c + d + e vaut 1 à 36, 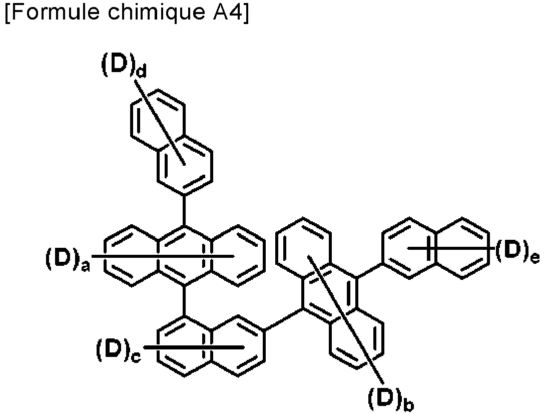
dans lequel, dans la Formule chimique A4,
a + b + c + d + e vaut 1 à 36, 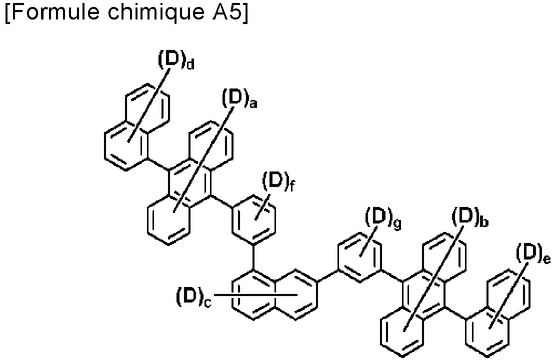
dans lequel, dans la Formule chimique A5,
a + b + c + d + e + f + g vaut 1 à 44, 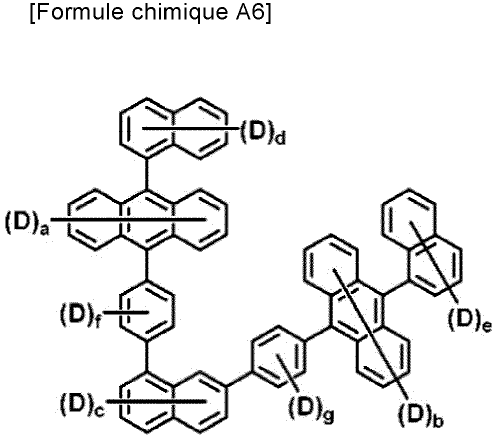
dans lequel, dans la Formule chimique A6,
a + b + c + d + e + f + g vaut 1 à 44, 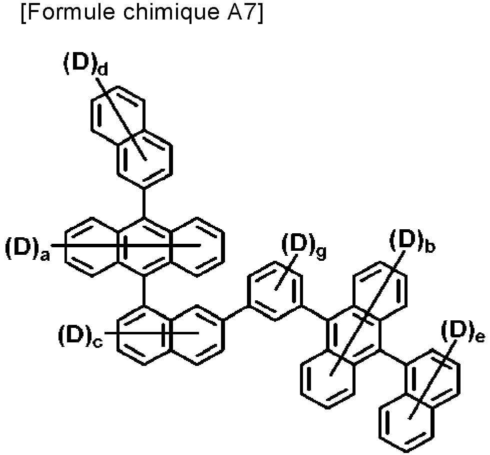
dans lequel, dans la Formule chimique A7,
a + b + c + d + e + g vaut 1 à 40, 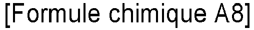 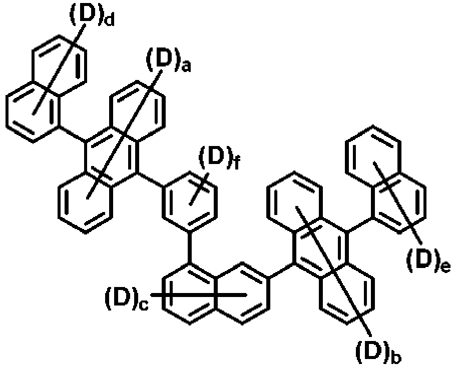
dans lequel, dans la Formule chimique A8,
a + b + c + d + e + f vaut 1 à 40, 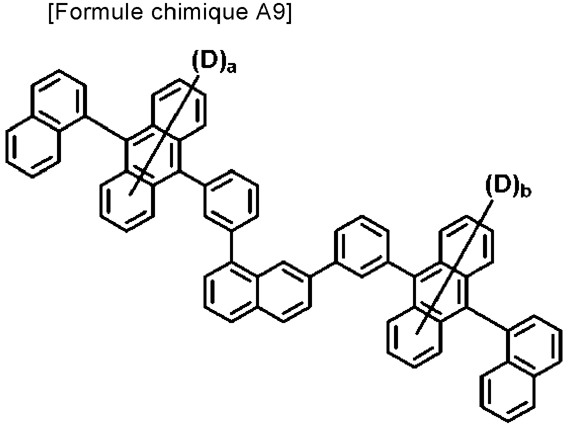
dans lequel, dans la Formule chimique A9,
a + b vaut 1 à 16, 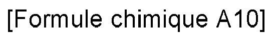 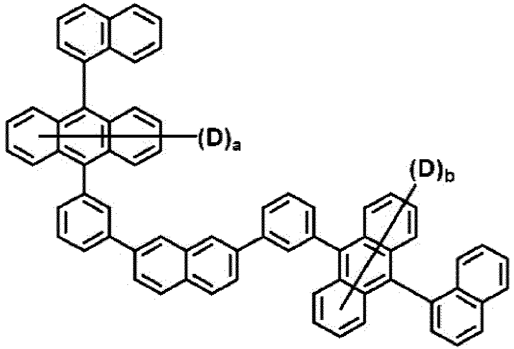
dans lequel, dans la Formule chimique A10,
a + b vaut 1 à 16, 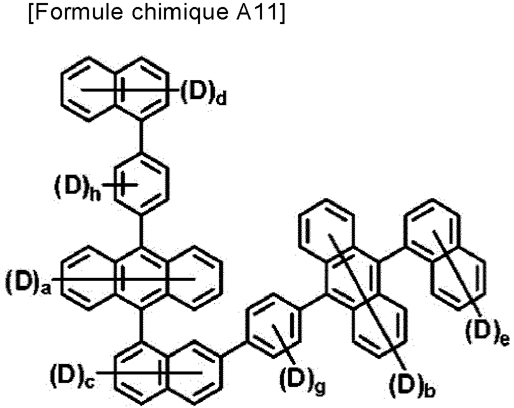
dans lequel, dans la Formule chimique A11,
a + b + c + d + e + g + h vaut 1 à 44, 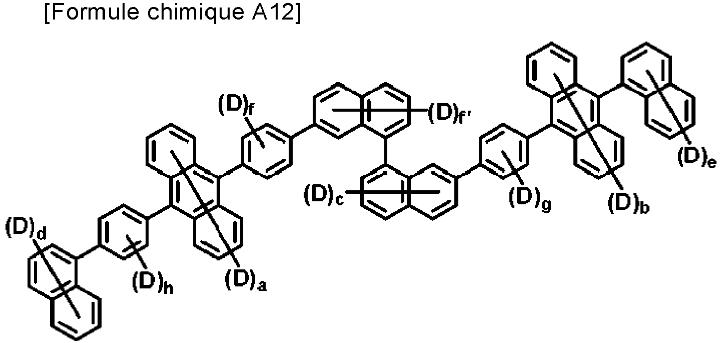
dans lequel, dans la Formule chimique A12,
a + b + c + d + e + f + f' + g + h vaut 1 à 54, 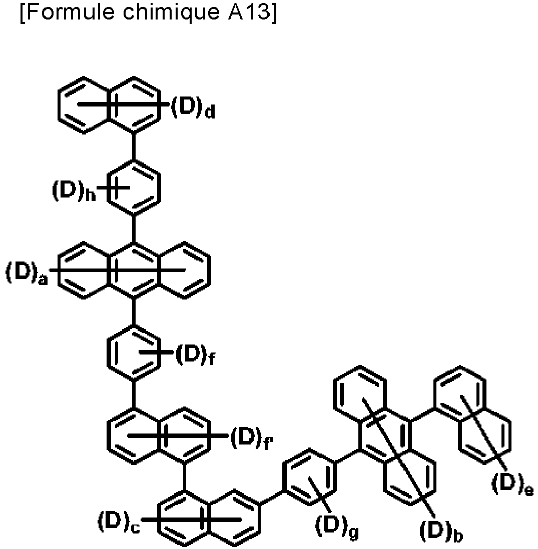
dans lequel, dans la Formule chimique A13,
a + b + c + d + e + f + f' + g + h vaut 1 à 54, 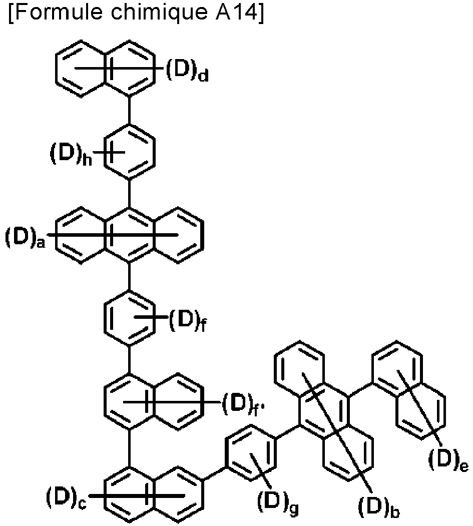
dans lequel, dans la Formule chimique A14,
a + b + c + d + e + f + f' + g + h vaut 1 à 54, 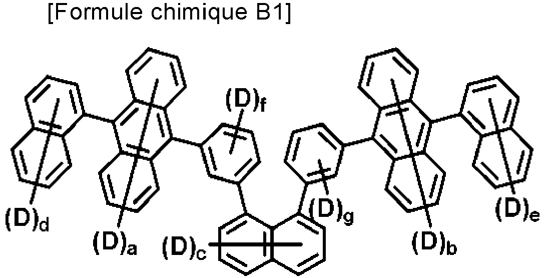
dans lequel, dans la Formule chimique B1,
a + b + c + d + e + f + g vaut 1 à 44, 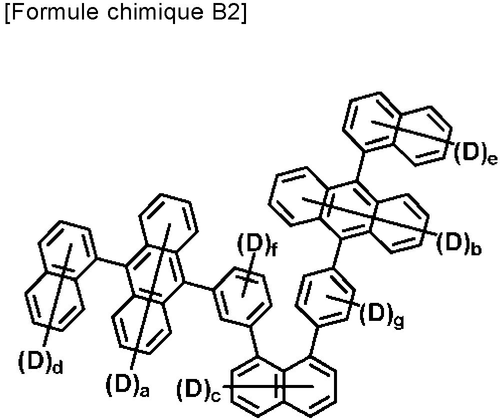
dans lequel, dans la Formule chimique B2,
a + b + c + d + e + f + g vaut 1 à 44, 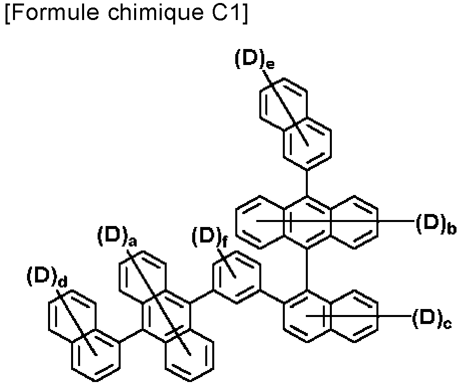
dans lequel, dans la Formule chimique C1,
a + b + c + d + e + f vaut 1 à 40, 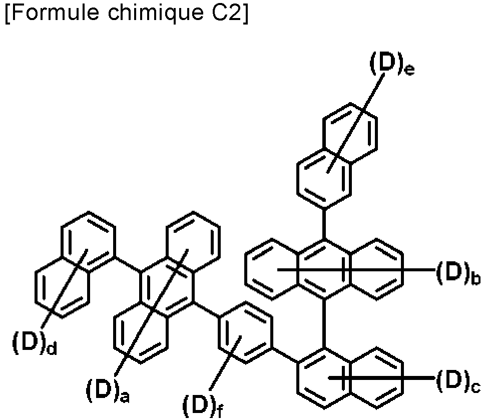
a + b + c + d + e + f vaut 1 à 40, 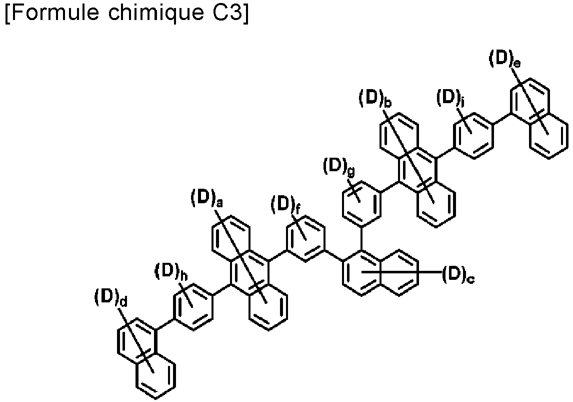
dans lequel, dans la Formule chimique C3,
a + b + c + d + e + f + g + h + i vaut 1 à 52, 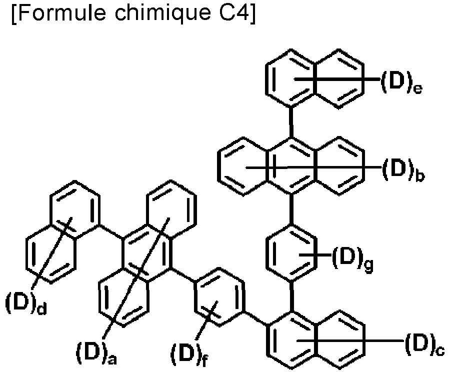
dans lequel, dans la Formule chimique C4,
a + b + c + d + e + f + g vaut 1 à 44, 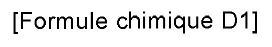 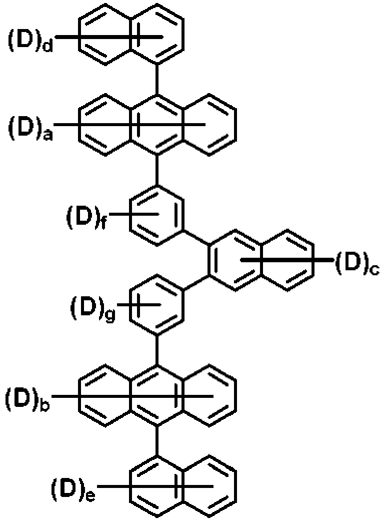
dans lequel, dans la Formule chimique D1,
a + b + c + d + e + f + g vaut 1 à 44, 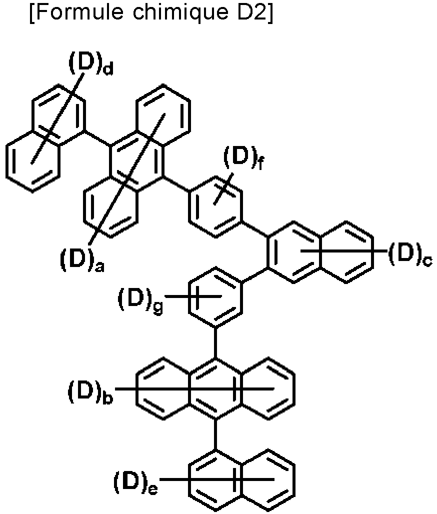
dans lequel, dans la Formule chimique D2,
a + b + c + d + e + f + g vaut 1 à 44, 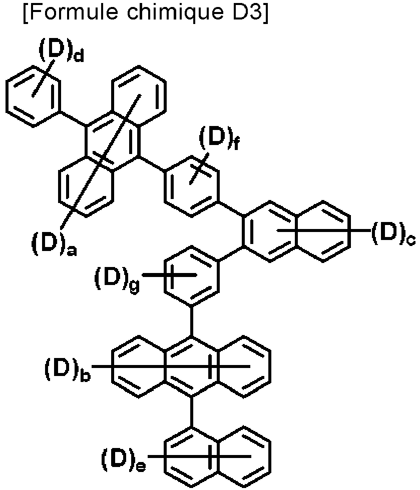
dans lequel, dans la Formule chimique D3,
a + b + c + d + e + f + g vaut 1 à 42, 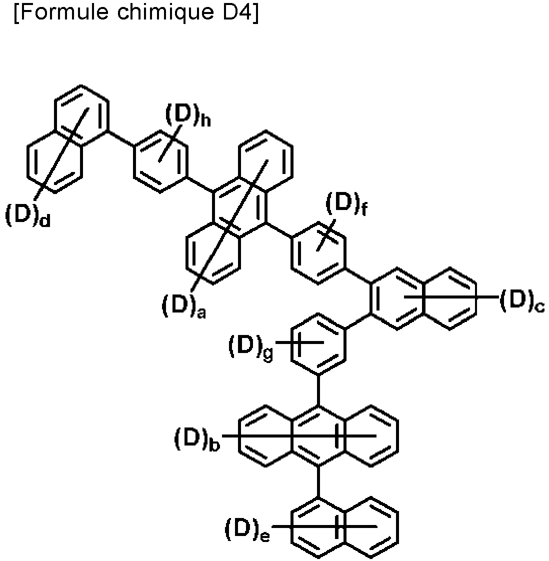
dans lequel, dans la Formule chimique D4,
a + b + c + d + e + f + g + h vaut 1 à 48, 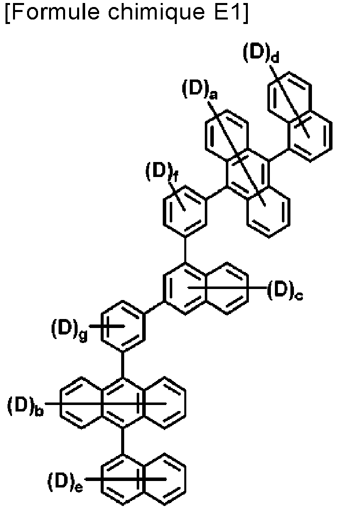
dans lequel, dans la Formule chimique E1,
a + b + c + d + e + f + g vaut 1 à 44, 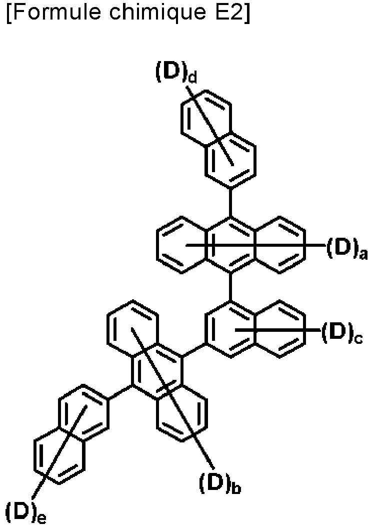
dans lequel, dans la Formule chimique E2,
a + b + c + d + e vaut 1 à 36, 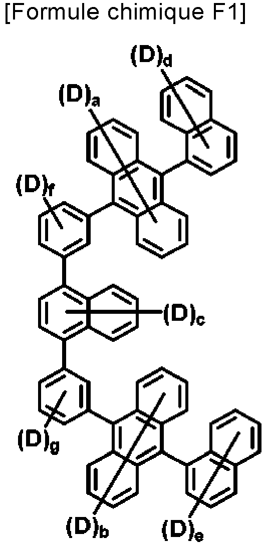
dans lequel, dans la Formule chimique F1,
a + b + c + d + e + f + g vaut 1 à 44, 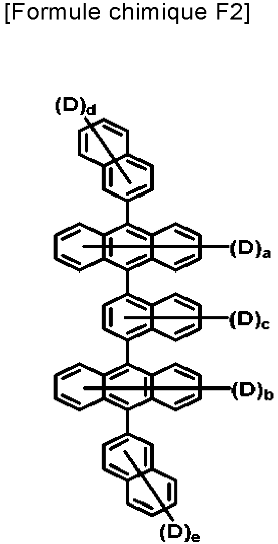
dans lequel, dans la Formule chimique F2,
a + b + c + d + e vaut 1 à 36, 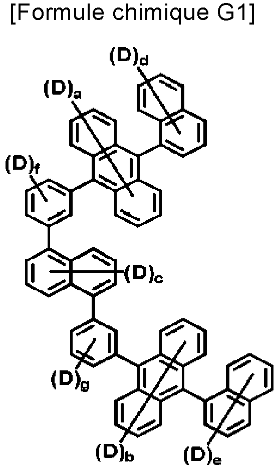
dans lequel, dans la Formule chimique G1,
a + b + c + d + e + f + g vaut 1 à 44, 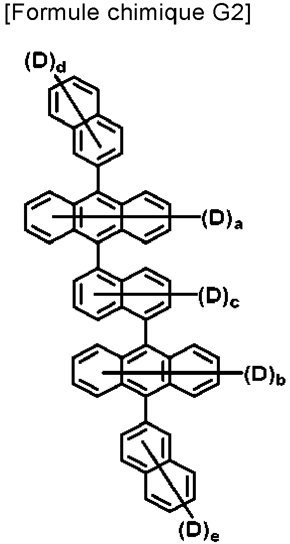
dans lequel, dans la Formule chimique G2,
a + b + c + d + e vaut 1 à 36, 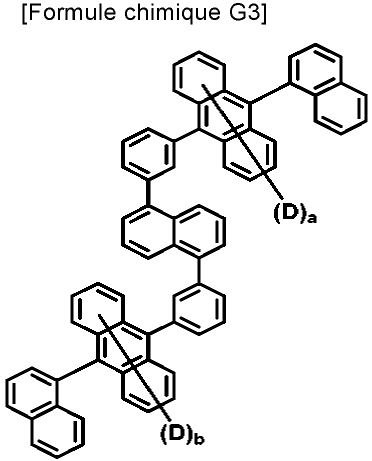
dans lequel, dans la Formule chimique G3,
a + b vaut 1 à 16, 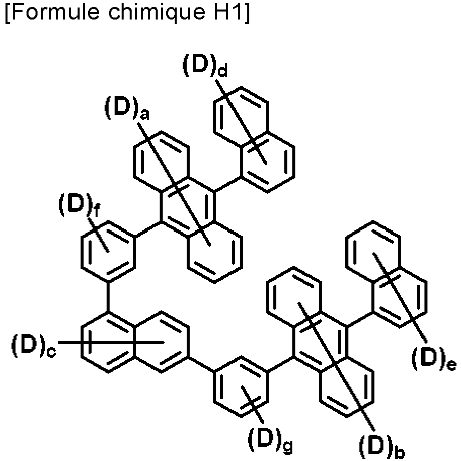
dans lequel, dans la Formule chimique H1,
a + b + c + d + e + f + g vaut 1 à 44, 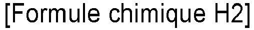 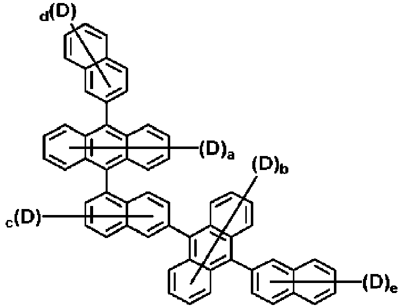
dans lequel, dans la Formule chimique H2,
a + b + c + d + e vaut 1 à 36, 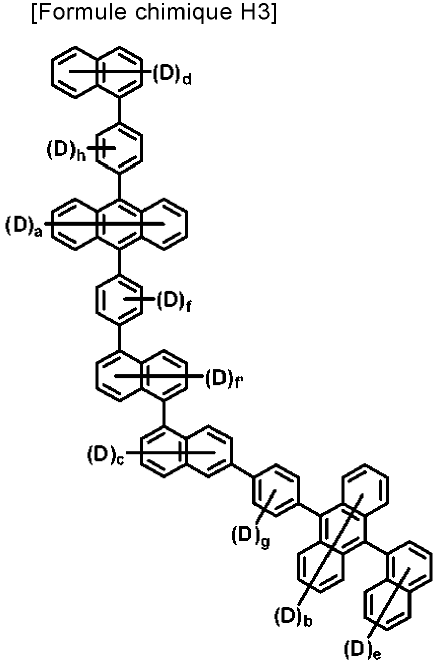
dans lequel, dans la Formule chimique H3,
a + b + c + d + e + f + f' + g + h vaut 1 à 54, 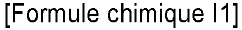 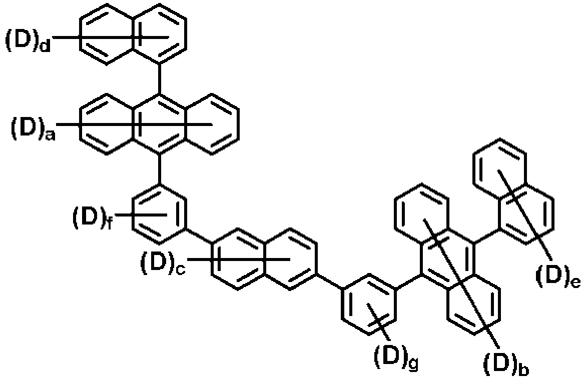
dans lequel, dans la Formule chimique 11,
a + b + c + d + e + f + g vaut 1 à 44, 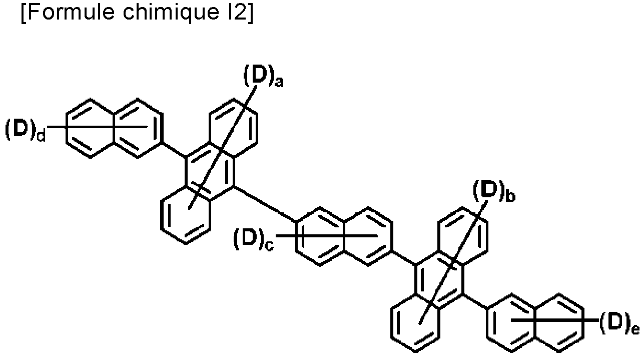
dans lequel, dans la Formule chimique I2,
a + b + c + d + e vaut 1 à 36, 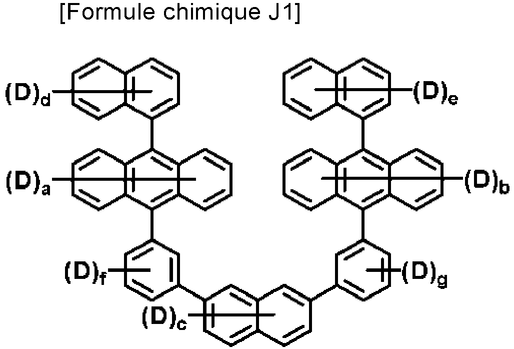
dans lequel, dans la Formule chimique J1,
a + b + c + d + e + f + g vaut 1 à 44, 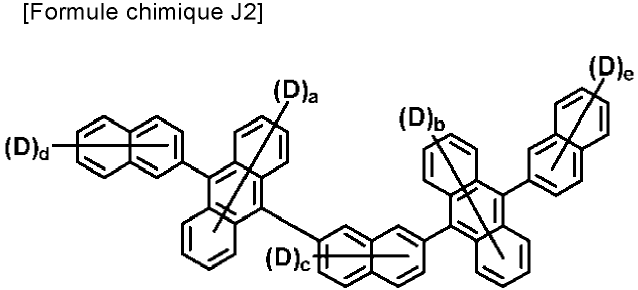
dans lequel, dans la Formule chimique J2,
a + b + c + d + e vaut 1 à 36.

15. Dispositif électroluminescent organique comprenant : une première électrode ; une deuxième électrode qui est disposée à l'opposé de la première électrode ; et une couche émettrice de lumière qui est disposée entre la première électrode et la deuxième électrode, dans lequel la couche émettrice de lumière comprend le composé tel que défini dans l'une quelconque des revendications 1 à 14.
